# EUROPEAN PATENT SPECIFICATION

(11) **EP 4 209 202 B1**
(45) Date of publication and mention of the grant of the patent: **15.04.2026**
(21) Application number: 23157858.4
(22) Date of filing: 30.09.2021
(51) Int. Cl.: A61F 13/58, A61F 13/51, A61F 13/42, A61F 13/49, A61F 13/551, A61F 13/514

(54) **ABSORBENT ARTICLE**
ABSORBIERENDER ARTIKEL
ARTICLE ABSORBANT

(30) Priority: 30.09.2020 CN 202011062112; 30.09.2020 CN 202011057915; 30.09.2020 CN 202011057367
(43) Date of publication of application: 12.07.2023
(62) Divisional of application: 21875814.2
(73) Proprietor: Unicharm Corporation, Shikokuchuo-shi, Ehime 799-0111 (JP)
(72) Inventor: KAWAKAMI, Yusuke, Kagawa 769-1602 (JP); WANG, Yinhua, Shanghai 201700 (CN); ZHENG, Lingshuang, Shanghai 201700 (CN)
(74) Representative: Dolleymores

(56) References cited:
- WO-A1-2013/108734
- JP-A- 2004 073 297
- JP-A- 2013 146 288
- JP-A- 2016 022 021
- JP-A- 2020 074 828
- US-A- 5 807 371
- US-B2- 8 822 752

## Description

### FIELD

The present invention relates to an absorbent article.

### BACKGROUND

Conventionally, an absorbent article such as a disposable diaper including a liquid-absorbent absorbent core has been generally used. As an example of such an absorbent article, a technique is known in which a breathable film is provided on the non-skin side of the absorbent core, moisture such as urine absorbed by the absorbent core is made to permeate the breathable film as water vapor and to be evaporated to the outside (non-skin side) of the diaper without leakage as liquid. For example, Patent Literature 1 discloses an underpants-shaped disposable diaper including: an absorbent core 56 (absorbent element 50); a liquid-impermeable sheet 70 that is arranged on the non-skin side of the absorbent core 56 and has liquid-impermeable and moisture-permeable properties; and a crotch portion exterior sheet 12 that is arranged on the non-skin side of the liquid-impermeable sheet 70 and is exposed on the outer surface of the product.

Patent Literature 3 discloses further pants-type disposable wearing articles.

Further, there has been conventionally known an underpants-shaped diaper using a hydrophilic nonwoven fabric whose hydrophilicity is enhanced by including a hydrophilic oil agent. For example, Patent Literature 2 discloses a technique related to a disposable diaper in which a hydrophilic nonwoven fabric is used as an exterior sheet 4 that constitutes an exterior body 3 of a diaper 1, the hydrophilic nonwoven fabric having hydrophilized fibers which are obtained by means such as making hydrophobic synthetic fibers undergo treatment with a hydrophilizing agent.

### [CITATION LIST]

### [PATENT LITERATURE]

Patent Literature 1: Japanese Patent Application Publication No.
Patent Literature 2: Japanese Patent Application Publication No. 2017-113186
Patent Literature 3: Japanese Patent Publication 2016-22021A(2)

### SUMMARY

### [TECHNICAL PROBLEM]

A problem to be solved by the present disclosure is at least one of first to third problems below.

A first problem is as follow. In a conventional absorbent article, as disclosed in Patent Literature 1, normally, an exterior sheet member (crotch portion exterior sheet 12) that constitutes the exterior of the product is provided on the non-skin side of the breathable film (liquid-impermeable sheet 70). The exterior sheet member is generally formed of hydrophobic nonwoven fabric such as meltblown nonwoven fabric, point-bonded nonwoven fabric, or air-through nonwoven fabric.

However, if the hydrophobic exterior sheet member is provided on the non-skin side of the breathable film, there is a case where moisture (e.g., urine) absorbed by the absorbent core is less likely to be evaporated to the outside of the absorbent article. For example, there is a case where water vapor that permeates the breathable film from the absorbent core and moves from the skin side to the non-skin side is inhibited by the hydrophobic exterior sheet member from further moving toward the non-skin side, and the moisture is likely to remain between the exterior sheet member and the breathable film. In this case, moisture is not discharged to the outside of the absorbent article and remains in the inside of the absorbent article. This causes stuffiness or skin problems, generating a risk of causing discomfort for the wearer.

The present disclosure was achieved in light of conventional problems such as that described above and an aspect of the present disclosure is to provide an absorbent article capable of easily discharging moisture that has been absorbed by an absorbent core, to the outside.

A second problem is as follow. In a diaper using a hydrophilic nonwoven fabric such as that of Patent Literature 2, in order to detect the presence or absence of excrement and to notify the detection to a user (caregiver or parent), there has been discussed providing an indicator that exhibits some reaction (e.g., a color reaction) by coming into contact with moisture contained in excrement. However, in the case where such an indicator is provided, the indicator may react with moisture such as sweat absorbed by the hydrophilic nonwoven fabric. That is, there is a risk that the indicator erroneously detects an object excluding excrement, making difficult accurate detection of the presence or absence of excrement.

The present disclosure was achieved in light of conventional problems such as that described above, and an aspect of the present disclosure is to suppress erroneous detection of an indicator in an absorbent article in which hydrophilic nonwoven fabric is used.

A third problem is that there is a case where an attachment such as a so-called post handling tape or a front-back display tape is attached to the surface of the exterior body of the disposable diaper using the adhesive. However, in the diaper such as that of Patent Literature 2, there is a risk that the influence of an oil agent (hydrophilizing agent) for hydrophilizing the hydrophilic nonwoven fabric make the attachment more likely to be peeled off from the surface of the exterior body. Further, there is a risk that, when the post handling tape (attachment) is pulled in order to unfold it after use of the diaper, the bonding between the fibers that constitute the hydrophilic nonwoven fabric are broken, thereby a part of the nonwoven fabric is torn, peeling off the attachment from the exterior body.

The present invention was achieved in light of conventional problems such as that described above and an aspect of the present invention is to make it less likely to peel off an attachment attached to an exterior body in an absorbent article in which hydrophilic nonwoven fabric is used.

### [SOLUTION TO PROBLEM]

A main aspect for solving the first problem is an absorbent article not in accordance with the present invention, the absorbent article including an absorbent main body including a liquid-absorbent absorbent core and a breathable film, the breathable film being provided on a non-skin side with respect to the absorbent core, a hydrophilic nonwoven fabric being provided on the non-skin side with respect to the breathable film, the absorbent core, the breathable film, and the hydrophilic nonwoven fabric at least partially overlapping each other when viewed in a thickness direction of the absorbent main body.

A main aspect for solving the second problem is an absorbent article not in accordance with the present invention, the absorbent article including: an absorbent main body including a liquid-absorbent absorbent core and a liquid-impermeable film, the liquid-impermeable film being provided on a non-skin side with respect to the absorbent core; an exterior member that is provided on a non-skin side with respect to the absorbent main body; and an indicator that exhibits a predetermined reaction upon contact with an excreted fluid, at least one of sheet members that constitute the exterior member being a hydrophilic nonwoven fabric, the hydrophilic nonwoven fabric and the liquid-impermeable film at least partially overlap each other when viewed in a thickness direction, the hydrophilic nonwoven fabric being arranged on a non-skin side with respect to the liquid-impermeable film in the thickness direction, the indicator being arranged on a skin side with respect to the liquid-impermeable film in the thickness direction.

A main aspect of the present invention for solving the third problem is an absorbent article as claimed in claim 1, the absorbent article including: a liquid-absorbent absorbent main body; an exterior member that is provided on a non-skin side with respect to the absorbent main body, the exterior member having a hydrophilic nonwoven fabric on a non-skin side, the hydrophilic nonwoven fabric containing a hydrophilic oil agent, the absorbent article has an attachment that is attached with an adhesive to a non-skin-side surface of the hydrophilic nonwoven fabric, wherein the attachment (2080) is a post handling attachment, the hydrophilic nonwoven fabric having a fiber with a fiber length longer than a dimension of the attachment, the dimension of the attachment being a smaller one of a dimension of the attachment in a longitudinal direction and a dimension of the attachment in a transverse direction that is orthogonal to the longitudinal direction.

Features of the present invention other than the above will become clear by reading the description of the present specification with reference to the accompanying drawings.

### [ADVANTAGEOUS EFFECT OF THE INVENTION]

An effect of the present disclosure on the first problem is that it is possible to provide an absorbent article capable of easily discharging moisture that has been absorbed by an absorbent core, to the outside.

An effect of the present disclosure on the second problem is that it is possible to suppress erroneous detection of an indicator in an absorbent article in which hydrophilic nonwoven fabric is used.

An effect of the present invention on the third problem is that it is possible to make it less likely to peel off an attachment attached to an exterior body in an absorbent article in which hydrophilic nonwoven fabric is used.

### BRIEF DESCRIPTION OF DRAWINGS

FIG. 1 is a schematic perspective view of a diaper 1.
FIG. 2 is a plan view of the diaper 1 in an unfolded and stretched state.
FIG. 3 is a schematic cross-sectional view taken along a line A-A in FIG. 2.
FIG. 4A is a plan view of an absorbent main body 10, and FIG. 4B is a schematic cross-sectional view of the absorbent main body 10.
FIGS. 5A to 5C are explanatory diagrams illustrating a mechanism by which moisture is discharged from the absorbent main body 10 in a region B in FIG. 3.
FIG. 6A is a schematic plan view showing a state of the diaper 1 having an underpants shape when viewed from the front side in the front-back direction in a stretched state.
FIG. 6B is a schematic plan view showing a state of the diaper 1 having an underpants shape when viewed from the back side in the front-back direction in a stretched state.
FIG. 7 is a schematic cross-sectional view taken along a line C-C in FIG. 6A.
FIGS. 8A and 8B are explanatory diagrams illustrating a method for attaching a waist elastic member 35 to a front waist portion 30 using welding portions 60.
FIG. 9A is a plan view of a diaper 2 in an unfolded and stretched state.
FIG. 9B is a schematic cross-sectional view taken along a line D-D in FIG. 9A.
FIG. 10A is a plan view of a diaper 3 in an unfolded and stretched state.
FIG. 10B is a schematic cross-sectional view taken along a line E-E in FIG. 10A.
FIG. 11A is a plan view of a diaper 4 in an unfolded and stretched state.
FIG. 11B is a schematic cross-sectional view taken along a line F-F in FIG. 11A.
FIG. 12 is a plan view of a diaper 1001 in an unfolded and stretched state.
FIG. 13 is a schematic cross-sectional view taken along a line A-A in FIG. 12.
FIG. 14A is a plan view of an absorbent main body 1010, and FIG. 14B is a schematic cross-sectional view of the absorbent main body 1010.
FIG. 15 is an explanatory diagram illustrating the basic principle when moisture is absorbed and evaporated in the diaper 1001.
FIGS. 16A and 16B are explanatory diagrams illustrating the relationship between the arrangement of an indicator 1070 and the movement of moisture in the diaper 1001.
FIG. 17 is a schematic cross-sectional view illustrating a modified example of the arrangement of the indicator 1070.
FIG. 18A is a plan view of a diaper 1002 in an unfolded and stretched state.
FIG. 18B is a schematic cross-sectional view taken along a line D-D in FIG. 18A.
FIG. 19A is a plan view of a diaper 1003 in an unfolded and stretched state.
FIG. 19B is a schematic cross-sectional view taken along a line E-E in FIG. 19A.
FIG. 20A is a plan view of a diaper 1004 in an unfolded and stretched state.
FIG. 20B is a schematic cross-sectional view taken along a line F-F in FIG. 20A.
FIG. 21 is a plan view of a diaper 2001 in an unfolded and stretched state.
FIG. 22 is a schematic cross-sectional view taken along a line A-A in FIG. 21.
FIG. 23A is a plan view of an absorbent main body 2010, and FIG. 23B is a schematic cross-sectional view of the absorbent main body 2010.
FIG. 24 is a schematic cross-sectional view illustrating a post handling tape 2080 provided on the non-skin-side surface of a back waist portion 2040.
FIG. 25A is a schematic perspective view of the diaper 2001 in a state where the post handling tape 2080 is unfolded, when viewed from the back side.
FIG. 25B is a schematic perspective view of a state in which the diaper 2001 in the state in FIG. 25A has been rolled up for disposal.
FIGS. 26A and 26B are explanatory diagrams illustrating a factor that causes the post handling tape 2080 to be separated in the post handling operation of the diaper 2001.
FIG. 27 is an explanatory diagram illustrating the relationship between fibers that constitute a non-skin-side sheet 2042 (hydrophilic nonwoven fabric) of the diaper 2001 and the post handling tape 2080.
FIG. 28 is a schematic cross-sectional view showing a modified example of the post handling tape 2080.
FIGS. 29A and 29B are explanatory diagrams illustrating forces that act on the post handling tape 2080 of the modified example when performing the post handling operation.
FIG. 30 is an explanatory diagram illustrating a state of a surface of the non-skin-side sheet 2042 in the portion to which the post handling tape 2080 is attached.

### DESCRIPTION OF FIGURES

At least following matters will become clear with description of this specification and attached drawings.

An absorbent article including an absorbent main body including a liquid-absorbent absorbent core and a breathable film, the breathable film being provided on a non-skin side with respect to the absorbent core, a hydrophilic nonwoven fabric being provided on the non-skin side with respect to the breathable film, the absorbent core, the breathable film, and the hydrophilic nonwoven fabric at least partially overlapping each other when viewed in a thickness direction of the absorbent main body.

According to the above-described absorbent article, in a region in which the absorbent core, the breathable film, and the hydrophilic nonwoven fabric overlap in the thickness direction, the hydrophilic nonwoven fabric having high hydrophilicity makes it easier to transfer moisture to the non-skin side in the thickness direction. That is, the transfer of moisture to the non-skin side is promoted, making it possible for moisture to be more likely to be discharged from the inside to the outside of the diaper.

In such an absorbent article, it is desirable that the absorbent article comprises: an absorbent main body including the absorbent core; and an exterior member including a front waist portion that is joined to a one side of the absorbent main body in a lengthwise direction and a back waist portion that is joined to another side of the absorbent main body in the lengthwise direction, that the absorbent article has a pair of side joining portions that connect two lateral side portions of the front waist portion and two lateral side portions of the back waist portion to each other in a state where the absorbent main body is folded one time at a predetermined position in the lengthwise direction, and that a hydrophilic nonwoven fabric is provided in at least a part of the exterior member.

According to the above-described absorbent article, in a so-called three-piece type underpants-shaped absorbent article composed of the absorbent main body, and the front and back waist portions (exterior member) that are arranged on the non-skin side of the absorbent main body, the transfer of moisture from the skin side to the non-skin side is promoted at a portion in which the absorbent core, the breathable film, and the hydrophilic nonwoven fabric overlap in the thickness direction. This enables to make moisture more likely to be discharged from the inside to the outside of the absorbent article.

In such an absorbent article, it is desirable that the absorbent article comprises: an absorbent main body including the absorbent core; and an exterior member that is provided on the non-skin side of the absorbent main body and that is integrally constituted so as to be continuous from a one side to another side of the absorbent main body in a lengthwise direction, that the absorbent article has a pair of side joining portions that connect two lateral side portions of the exterior member on a one side in the lengthwise direction and two lateral side portions of the exterior member on another side in the lengthwise direction to each other in a state where the absorbent main body is folded one time at a predetermined position in the lengthwise direction, and that a hydrophilic nonwoven fabric is provided in at least a part of the exterior member.

According to the above-described absorbent article, in a so-called two-piece type underpants-shaped absorbent article including an absorbent main body and an integral exterior member arranged on the non-skin side of the absorbent main body, the transfer of moisture from the skin side to the non-skin side is promoted at a portion in which the absorbent core, the breathable film, and the hydrophilic nonwoven fabric overlap in the thickness direction. This enables to make moisture more likely to be discharged from the inside to the outside of the absorbent article.

In such an absorbent article, it is desirable that concerning an area of a portion in which the absorbent core, the breathable film, and the hydrophilic nonwoven fabric overlap each other when viewed in the thickness direction, the area is larger in a region located below a lower end of the side joining portion than in a region located above the lower end of the side joining portion.

According to the above-described absorbent article, in a region (crotch region) positioned at the wearer's crotch, it is possible to increase the area of a region where the absorbent core and the hydrophilic nonwoven fabric overlap each other. Therefore, in a wide range of the crotch region, it enables moisture such as urine absorbed by the absorbent core (skin side) more likely to transfer to the hydrophilic nonwoven fabric side (non-skin side). Accordingly, this makes it possible for the wearer to be less likely to feel discomfort at the crotch portion.

In such an absorbent article, it is desirable that the breathable film as a whole overlaps the hydrophilic nonwoven fabric when viewed in the thickness direction.

According to the above-described absorbent article, of urine or the like absorbed by the absorbent core, moisture that permeates the breathable film as water vapor from the skin side to the non-skin side is more likely to transfer to the hydrophilic nonwoven fabric located on the non-skin side in the entire region of the breathable film. Accordingly, the movement of moisture from the skin side to the non-skin side in the thickness direction is more easily promoted, making it possible to make moisture less likely to remain in the inside of the absorbent article.

In such an absorbent article, it is desirable that the exterior member has an elastic member that stretches and contracts in a lateral direction, and that a stress of the elastic member in a lateral central portion of the absorbent core is smaller than a stress of the elastic member in two lateral side portions of the absorbent core.

According to the above-described absorbent article, a contractive force the elastic member is less likely to act in the lateral central portion of the absorbent core, making the absorbent main body and the exterior member more likely to maintain their planar shapes. Therefore, in the central portion, the laminate structure of the sheet members in the thickness direction is more likely to be maintained, and the movement of moisture in the thickness direction is less likely to be inhibited. Accordingly, moisture such as urine absorbed by the absorbent core on the skin side can be easily discharged to the outside of the absorbent core by transferring the moisture to the hydrophilic nonwoven fabric on the non-skin side.

In such an absorbent article, it is desirable that the absorbent article comprises: an absorbent main body including the absorbent core; and an exterior member that is provided on the non-skin side of the absorbent main body and that is integrally constituted so as to be continuous from a one side to another side of the absorbent main body in a lengthwise direction, that the absorbent article has a pair of fastening tapes in end portions of the exterior member on the one side in the lengthwise direction, and that a hydrophilic nonwoven fabric is provided in at least a part of the exterior member.

According to the above-described absorbent article, in a so-called tape-type absorbent article which has an absorbent main body and an exterior member arranged on the non-skin side of the absorbent main body and which is put on the wearer's body by using a pair of fastening tapes provided on the exterior member, the transfer of moisture from the skin side to the non-skin side is promoted at a portion in which the absorbent core, the breathable film, and the hydrophilic nonwoven fabric overlap in the thickness direction. This enables to make moisture more likely to be discharged from the inside to the outside of the absorbent article.

In such an absorbent article, it is desirable that the exterior member has a hydrophobic nonwoven fabric on a skin side with respect to the hydrophilic nonwoven fabric, the hydrophobic nonwoven fabric having lower hydrophilicity than the hydrophilic nonwoven fabric.

According to the above-described absorbent article, in the exterior member, a hydrophilic gradient generated between the hydrophobic nonwoven fabric and the hydrophilic nonwoven fabric makes it easier for moisture to be transferred from the hydrophobic nonwoven fabric side to the hydrophilic nonwoven fabric side. That is, moisture is easily smoothly transferred from the skin side to the non-skin side. Therefore, moisture such as urine can be easily discharged to the outside of the absorbent article.

In such an absorbent article, it is desirable that the hydrophilic nonwoven fabric is provided farthest on a non-skin side of the exterior member.

According to the above-described absorbent article, the hydrophilic nonwoven fabric arranged farthest on the non-skin side of the exterior member serves as an interface with the atmosphere, and this makes it easier to evaporate into the atmosphere the moisture that is transferred from the absorbent core and is contained in the hydrophilic nonwoven fabric. Accordingly, moisture is more easily discharged to the outside of the absorbent article, and this makes it possible for the wearer to be less likely to feel discomfort.

In such an absorbent article, it is desirable that the absorbent article has a lateral direction, that a lateral width of the hydrophilic nonwoven fabric is larger than a lateral width of the breathable film, and that the lateral width of the breathable film is equal to or larger than a lateral width of the absorbent core.

According to the above-described absorbent article, moisture (water vapor) can permeate the breathable film from the absorbent core throughout the lateral direction, transferring to the non-skin side. Further, moisture that has permeated the breathable film is more likely to be absorbed by the hydrophilic nonwoven fabric on the non-skin side. Therefore, moisture can be more efficiently discharged from the inside (skin side) to the outside (non-skin side) of the absorbent article.

In such an absorbent article, it is desirable that the absorbent article has a portion in which the absorbent core and the hydrophilic nonwoven fabric overlap each other when viewed in the thickness direction, and a portion in which the absorbent core and the hydrophilic nonwoven fabric do not overlap each other when viewed in the thickness direction.

According to the above-described absorbent article, in the portion in which the absorbent core and the hydrophilic nonwoven fabric overlap each other, moisture such as urine can be efficiently discharged to the outside of the absorbent article. On the other hand, in the portion in which the absorbent core and the hydrophilic nonwoven fabric do not overlap each other, moisture such as sweat of a wearer can be transferred to the hydrophilic nonwoven fabric side and discharged to the outside of the absorbent article. This makes it possible for problems such as stuffiness while the absorbent article is put on much less likely to occur.

In such an absorbent article, it is desirable that the absorbent article further comprises a slit portion that penetrates the absorbent core in the thickness direction, and that the absorbent article has a portion in which the slit portion, the breathable film, and the hydrophilic nonwoven fabric overlap one another when viewed in the thickness direction.

According to the above-described absorbent article, the breathable film and the hydrophilic nonwoven fabric are provided on the non-skin side of the slit portion of the absorbent core where moisture such as urine is likely to be accumulated, and this allows the moisture accumulated in the slit portion to easily transfer from the skin side to the non-skin side and to be evaporated to the outside. Accordingly, providing the slit portion makes the absorbent core more likely to deform along the body shape of the wearer, and suppresses stuffiness, making it possible for the wearer to be less likely to feel discomfort.

In such an absorbent article, it is desirable that a moisture permeability of the breathable film is equal to or greater than 1000 g/m²·24 h and equal to or smaller than 3500 g/m²·24 h.

According to the above-described absorbent article, in the case where the moisture permeability is equal to or greater than 1000 g/m²·24 h, water vapor easily permeates the breathable film, and moisture can be easily evaporated from the inside (skin side) to the outside (non-skin side) of the absorbent article. On the other hand, in the case where the moisture permeability is equal to or smaller than 3500 g/m²·24 h, it is possible to suppress the excessive permeation of water vapor by the breathable film and to suppress the excessive absorption of moisture by the hydrophilic nonwoven fabric provided on the outside (non-skin side). Therefore, during usage of the absorbent article, the hydrophilic nonwoven fabric on the outside is prevented from becoming wet while discharging an appropriate amount of moisture, making it possible for the wearer to be less likely to feel discomfort.

An absorbent article including: an absorbent main body including a liquid-absorbent absorbent core and a liquid-impermeable film, the liquid-impermeable film being provided on a non-skin side with respect to the absorbent core; an exterior member that is provided on a non-skin side with respect to the absorbent main body; and an indicator that exhibits a predetermined reaction upon contact with an excreted fluid, at least one of sheet members that constitute the exterior member being a hydrophilic nonwoven fabric, the hydrophilic nonwoven fabric and the liquid-impermeable film at least partially overlap each other when viewed in a thickness direction, the hydrophilic nonwoven fabric being arranged on a non-skin side with respect to the liquid-impermeable film in the thickness direction, the indicator being arranged on a skin side with respect to the liquid-impermeable film in the thickness direction.

According to the above-described absorbent article, in the case where moisture such as sweat is absorbed by the hydrophilic nonwoven fabric that constitutes the exterior member, the liquid-impermeable film provided between the hydrophilic nonwoven fabric and the indicator blocks the movement of moisture from the hydrophilic nonwoven fabric (non-skin side) to the indicator (skin side). That is, moisture from sweat or the like absorbed by the hydrophilic nonwoven fabric is less likely to come into contact with the indicator. Accordingly, this makes it possible to prevent the indicator from erroneously detecting a liquid excluding excreted fluid.

In such an absorbent article, it is desirable that the exterior member includes a front waist portion and a back waist portion, that the front waist portion is joined to a one side of the absorbent main body in a lengthwise direction, that the back waist portion is joined to another side of the absorbent main body in the lengthwise direction, that two lateral side portions of the front waist portion and two lateral side portions of the back waist portion are connected to each other in a state where the absorbent main body is folded one time at a predetermined position in the lengthwise direction, and that the hydrophilic nonwoven fabric is arranged in at least one of the front waist portion and the back waist portion on the non-skin side.

According to the above-described absorbent article, in a so-called three-piece type underpants-shaped absorbent article composed of an absorbent main body, and the front and back waist portions (exterior member) that are arranged on the non-skin side of the absorbent main body, it is possible to prevent moisture such as sweat absorbed by the hydrophilic nonwoven fabric (non-skin side) from moving toward the indicator side (skin side) and coming into contact with the indicator. Therefore, in the three-piece type underpants-shaped diaper, the erroneous detection of the indicator can be suppressed.

In such an absorbent article, it is desirable that in at least one of the front waist portion and the back waist portion, the absorbent article has a portion in which the hydrophilic nonwoven fabric and the indicator overlap each other when viewed in the thickness direction.

According to the above-described absorbent article, in a three-piece type underpants-shaped diaper, while arranging the indicator over a wide range to the extent to have a portion that overlaps the exterior member, it is possible to suppress the erroneous detection of moisture such as sweat absorbed by the exterior member by the indicator.

In such an absorbent article, it is desirable that in the back waist portion, the absorbent article has a portion in which the hydrophilic nonwoven fabric and the indicator overlap each other when viewed in the thickness direction.

According to the above-described absorbent article, the moisture such as sweat absorbed by the hydrophilic nonwoven fabric in the back waist portion can be prevented from coming into contact with the indicator. Therefore, in the back-side in which the wearer is likely to sweat, the erroneous detection of the indicator can be effectively suppressed.

In such an absorbent article, it is desirable that concerning a number of overlaid sheet members located on a non-skin side with respect to the indicator in a region where the indicator and the hydrophilic nonwoven fabric overlap each other when viewed in the thickness direction, concerning a number of overlaid sheet members located on the non-skin side with respect to the indicator in a region where the indicator and the hydrophilic nonwoven fabric do not overlap each other when viewed in the thickness direction, the former is larger than the latter.

According to the above-described absorbent article, in the region where the indicator overlaps the hydrophilic nonwoven fabric, the larger the number of overlaid sheet members located on the non-skin side, the less likely moisture contained in the non-skin-side hydrophilic nonwoven fabric to move toward the skin side. This can make it more likely to suppress erroneous detection by the indicator. Further, in the region where the indicator and the hydrophilic nonwoven fabric do not overlap each other, the smaller the number of overlaid sheet members, the more flexible the absorbent article becomes, the better fit can be obtained during usage of the absorbent article.

In such an absorbent article, it is desirable that the exterior member is integrally constituted so as to be continuous from a one side to another side of the absorbent main body in a lengthwise direction, that two lateral side portions of the exterior member on a one side in the lengthwise direction and two lateral side portions of the exterior member on another side in the lengthwise direction are connected to each other in a state where the absorbent main body is folded one time at a predetermined position in the lengthwise direction, and that the hydrophilic nonwoven fabric is arranged in at least a part of the exterior member on the non-skin side.

According to the above-described absorbent article, in a so-called two-piece type or simple three-piece type underpants-shaped absorbent article including an absorbent main body and an integral exterior member arranged on the non-skin side of the absorbent main body, it is possible to prevent moisture such as sweat absorbed by the hydrophilic nonwoven fabric (non-skin side) from moving toward the indicator side (skin side) and coming into contact with the indicator. Therefore, in the two-piece type underpants-shaped diaper or the like, the erroneous detection of the indicator can be suppressed.

In such an absorbent article, it is desirable that the exterior member is integrally constituted so as to be continuous from a one side to another side of the absorbent main body in a lengthwise direction, that the absorbent article has a pair of fastening tapes in end portions of the exterior member on the one side in the lengthwise direction, the pair of fastening tapes being for engaging with end portions of the exterior member on the other side in the lengthwise direction, and that the hydrophilic nonwoven fabric is arranged in at least a part of the exterior member on the non-skin side.

According to the above-described absorbent article, in a so-called tape-type absorbent article which has an absorbent main body and an exterior member arranged on the non-skin side of the absorbent main body and which is put on the wearer's body by using a pair of fastening tapes provided on the exterior member, it is possible to prevent moisture such as sweat absorbed by the hydrophilic nonwoven fabric (non-skin side) from moving toward the indicator side (skin side) and coming into contact with the indicator. Therefore, in the tape-type underpants-shaped diaper, the erroneous detection of the indicator can be suppressed.

In such an absorbent article, it is desirable that in at least a part of the exterior member, the absorbent article has a portion in which the hydrophilic nonwoven fabric and the indicator overlap each other when viewed in the thickness direction.

According to the above-described absorbent article, while arranging the indicator over a wide range, it is possible to suppress the erroneous detection of moisture such as sweat absorbed by the exterior member by the indicator. That is, while securing a wide excrement detection range, it is possible to make it more likely to suppress erroneous detection caused by moisture such as sweat.

In such an absorbent article, it is desirable that on a back side with respect to a lengthwise central position of the exterior member, the absorbent article has a portion in which the hydrophilic nonwoven fabric and the indicator overlap each other when viewed in the thickness direction.

According to the above-described absorbent article, moisture such as sweat absorbed by the hydrophilic nonwoven fabric of the exterior member on the back side (back waist portion) can be prevented from coming into contact with the indicator. Therefore, in the back-side in which the wearer is likely to sweat, the erroneous detection of the indicator can be effectively suppressed.

In such an absorbent article, it is desirable that the absorbent article has a portion in which the indicator as a whole overlaps the hydrophilic nonwoven fabric when viewed in the thickness direction.

According to the above-described absorbent article, the entire region of the indicator can be made less likely to come into contact with moisture contained in the hydrophilic nonwoven fabric. Therefore, by arranging the indicators in a wide range, erroneous detection can be less likely to occur while increasing the excrement detection range.

In such an absorbent article, it is desirable that a hydrophobic nonwoven fabric is provided in at least a part of a space between the liquid-impermeable film and the hydrophilic nonwoven fabric in the thickness direction, the hydrophobic nonwoven fabric having a lower hydrophilicity than the hydrophilic nonwoven fabric.

According to the above-described absorbent article, the hydrophobic nonwoven fabric is interposed between the liquid-impermeable film and the hydrophilic nonwoven fabric in the thickness direction, and this makes it more likely to suppress the movement of moisture contained in the hydrophilic nonwoven fabric toward the liquid-impermeable film side (skin side). Therefore, this can make more likely to suppress erroneous detection by the indicator.

In such an absorbent article, it is desirable that the exterior member has the hydrophobic nonwoven fabric on a skin side with respect to the hydrophilic nonwoven fabric.

According to the above-described absorbent article, a capillary phenomenon occurs due to fibers that are densely provided and constitute the hydrophobic nonwoven fabric. This makes moisture such as sweat likely to be absorbed from the wearer's skin. Further, the moisture absorbed by the hydrophobic nonwoven fabric is likely to transfer to the hydrophilic nonwoven fabric on the skin side due to a difference in magnitude of hydrophilicity. Thus, making moisture such as sweat likely to transfer to the non-skin side makes it possible to suppress the occurrence of erroneous detection in the indicator on the skin side.

In such an absorbent article, it is desirable that the hydrophilic nonwoven fabric is arranged farthest on a non-skin side of the exterior member.

According to the above-described absorbent article, the hydrophilic nonwoven fabric serves as an interface with the atmosphere, and therefore moisture such as sweat contained in the hydrophilic nonwoven fabric can be efficiently evaporated to the outside of the absorbent article. Therefore, this makes it possible to suppress the occurrence of erroneous detection by the indicator.

In such an absorbent article, it is desirable that the hydrophobic nonwoven fabric contains fibers having an average fiber length of 100 mm or more.

According to the above-described absorbent article, by using the hydrophobic nonwoven fabric containing long fibers having an average fiber length of 100 mm or more, moisture is more likely to be diffused in the planar direction of the hydrophobic fibers, and this makes the moisture less likely to move in the thickness direction of the hydrophobic fibers. Accordingly, moisture is prevented from permeating the hydrophobic fibers in the thickness direction and moving to the liquid-impermeable film, and this can make it more likely to suppress erroneous detection by the indicator.

In such an absorbent article, it is desirable that the exterior member includes an elastic member that stretches and contracts in a lateral direction, and that a stress of the elastic member in a portion that overlaps the indicator when viewed in the thickness direction is smaller than a stress of the elastic member in a portion that does not overlap the indicator when viewed in the thickness direction.

According to the above-described absorbent article, the stress of the elastic member in the portion that overlaps the indicator is reduced so that the contractive force is less likely to act on the portion. This can prevent the hydrophilic nonwoven fabric containing moisture from being pressed against the indicator side (skin side). Accordingly, this can make erroneous detection by the indicator less likely to occur.

In such an absorbent article, it is desirable that in a region where the indicator and the exterior member overlap each other when viewed in the thickness direction, an adhesive layer is provided between the hydrophilic nonwoven fabric and the liquid-impermeable film in the thickness direction.

According to the above-described absorbent article, the adhesive layer is provided between the hydrophilic nonwoven fabric and the indicator in the thickness direction, and this makes the adhesive layer likely to hinder the movement of moisture in the thickness direction. That is, moisture such as sweat contained in the hydrophilic nonwoven fabric is prevented from moving toward the skin side in the thickness direction and coming into contact with the indicator. This can make it more likely to suppress erroneous detection by the indicator.

In such an absorbent article, it is desirable that the adhesive layer includes at least a first adhesive layer that joins the exterior member and the absorbent main body, and a second adhesive layer that joins the liquid-impermeable film and a sheet member adjacent to a non-skin side of the liquid-impermeable film, and that the adhesive layer has a portion in which the first adhesive layer and the second adhesive layer overlap each other when viewed in the thickness direction.

According to the above-described absorbent article, the amount of the adhesive per unit volume is increased in the portion in which the first adhesive layer and the second adhesive layer overlap each other, and thus the movement of moisture in the thickness direction is more likely to be suppressed in the overlapping portion. Therefore, moisture such as sweat contained in the hydrophilic nonwoven fabric can be prevented from moving toward the skin side in the thickness direction and coming into contact with the indicator. This can make it further more likely to suppress erroneous detection by the indicator.

An absorbent article including: a liquid-absorbent absorbent main body; an exterior member that is provided on a non-skin side with respect to the absorbent main body, the exterior member having a hydrophilic nonwoven fabric on a non-skin side, the hydrophilic nonwoven fabric containing a hydrophilic oil agent, the absorbent article has an attachment that is attached with an adhesive to a non-skin-side surface of the hydrophilic nonwoven fabric, the hydrophilic nonwoven fabric having a fiber with a fiber length longer than a dimension of the attachment, the dimension of the attachment being a smaller one of a dimension of the attachment in a longitudinal direction and a dimension of the attachment in a transverse direction that is orthogonal to the longitudinal direction.

According to the above-described absorbent article, when the fiber length of the fibers that constitute the hydrophilic nonwoven fabric is larger than the minimum width of the attachment, tangling (entanglement point) between the fibers is likely to be formed in a range wider than the minimum width of the attachment, making stronger bonding between the fibers. Therefore, even in the case of exerting a force that peels off the attachment from the hydrophilic nonwoven fabric, the hydrophilic nonwoven fabric is less likely to be torn in a portion to which the attachment is attached. This enables to make the attachment less likely to peel off.

In such an absorbent article, it is desirable that the longitudinal direction is a direction in which the attachment is pulled from the hydrophilic nonwoven fabric, and that the fiber is arranged so as to extend across the attachment in the transverse direction.

According to the above-described absorbent article, the fibers are likely to be entangled with each other in the regions located outside the two transverse ends of the attachment and the bonding force becomes stronger. Therefore, even in the case where a force that pulls the attachment acts along the longitudinal direction of the attachment, the hydrophilic nonwoven fabric is further less likely to be torn. This enables to make it more likely to suppress the separation of the attachment.

In such an absorbent article, it is desirable that the hydrophilic nonwoven fabric has a plurality of the fibers, and that a number of fibers arranged so as to extend across the attachment in the transverse direction is larger than a number of fibers arranged so as to extend across the attachment in the longitudinal direction.

According to the above-described absorbent article, among the plurality of fibers that constitute the hydrophilic nonwoven fabric, as the number of fibers extending across the attachment in a direction (transverse direction) orthogonal to a direction in which the attachment is pulled increases, the hydrophilic nonwoven fabric is much less likely to be torn. That is, the bonding force of the fibers that constitute the hydrophilic nonwoven fabric becomes likely to resist the force that pulls the attachment. This enables to make the attachment further less likely to peel off.

In such an absorbent article, it is desirable that the hydrophilic nonwoven fabric has a plurality of compressed portions where the hydrophilic nonwoven fabric is compressed in a thickness direction.

According to the above-described absorbent article, providing the plurality of compressed portions makes it possible to enhance the strength of the hydrophilic nonwoven fabric. Accordingly, even in the case where the force that pulls the attachment acts, the hydrophilic nonwoven fabric is less likely to be torn, enabling to make it more likely to suppress the peeling of the attachment.

In such an absorbent article, it is desirable that the fiber length is larger than a minimum value of a space between two adjacent compressed portions.

According to the above-described absorbent article, in the case where the space between the compressed portions is shorter than the fiber length, the compressed portions are more likely to be formed overlapping with the fibers. That is, there is a high possibility that the fibers themselves are compressed. By pressing the fibers and other fibers against each other in the portion where the fibers are compressed, it increases the bonding force between the fibers, making the hydrophilic nonwoven fabric further less likely to be torn. This enables to make the attachment further less likely to peel off.

In such an absorbent article, it is desirable that at least one compressed portion is arranged in a region where the attachment and the hydrophilic nonwoven fabric overlap each other when viewed in a thickness direction of the exterior member.

According to the above-described absorbent article, in the portion of the hydrophilic nonwoven fabric where the compressed portions are arranged, the strength is increased, and the hydrophilic nonwoven fabric is less likely to be torn. Therefore, the attachment is attached to the region of the hydrophilic nonwoven fabric where the strength is enhanced by arranging the compressed portions, enabling to make it more likely to suppress the peeling of the attachment.

In such an absorbent article, it is desirable that, of the dimension of the attachment in the longitudinal direction and the dimension of the attachment in the transverse direction, the smaller dimension is larger than a minimum value of a space between two adjacent compressed portions.

According to the above-described absorbent article, in the case where the minimum width of the attachment is larger than the space between the compressed portions, there is a high possibility that two or more compressed portions are arranged in the width direction (lateral direction) of the attachment. Therefore, the bonding between the fibers in the hydrophilic nonwoven fabric becomes much stronger, and the hydrophilic nonwoven fabric is less likely to be torn in the portion to which the attachment is attached. This enables to make the attachment further less likely to peel off.

In such an absorbent article, it is desirable that the exterior member has a hydrophobic nonwoven fabric on a skin side with respect to the hydrophilic nonwoven fabric, the hydrophobic nonwoven fabric having lower hydrophilicity than the hydrophilic nonwoven fabric.

According to the above-described absorbent article, a capillary phenomenon occurs due to high density fibers that constitute the hydrophobic nonwoven fabric provided on the skin side, making moisture such as sweat more likely to be absorbed from the wearer's skin. Further, the moisture absorbed by the hydrophobic nonwoven fabric is likely to transfer to the hydrophilic nonwoven fabric located on the non-skin side, due to a difference in magnitude of hydrophilicity. This makes moisture such as sweat more likely to be absorbed in the exterior member, to transfer to the non-skin side, and to be evaporated to the outside of the absorbent article. Accordingly, this makes it possible to suppress the occurrence of stuffiness, rashes, or the like while the absorbent article is put on.

In such an absorbent article, it is desirable that the hydrophilic nonwoven fabric has a hole portion that penetrates the hydrophilic nonwoven fabric in a thickness direction, and at least one hole portion is arranged in a region where the attachment and the hydrophilic nonwoven fabric overlap each other when viewed in a thickness direction of the exterior member.

According to the above-described absorbent article, in the portion where the hole portion is provided, the adhesive used to attach the attachment to the hydrophilic nonwoven fabric is likely to adhere to the hydrophobic nonwoven fabric on the skin side. That is, a portion is formed in which the hydrophobic nonwoven fabric, which is less likely to be affected by an oil agent, and the attachment are directly glued with the adhesive, and the adhesive strength of the attachment is increased. This enables to make it more likely to suppress the peeling of the attachment from the hydrophilic nonwoven fabric.

In such an absorbent article, it is desirable that the attachment is a post handling tape for maintaining the absorbent article in a compact form when disposing of the absorbent article after use.

According to the above-described absorbent article, during a post handling operation for disposing of the absorbent article after use, it is possible to suppress the separation of the post handling tape from the exterior member. Therefore, it is possible to reduce the stress of the user when performing the post handling operation of the absorbent article.

In such an absorbent article, it is desirable that the post handling tape includes a first member that is attached to the hydrophilic nonwoven fabric, and a second member that is joined to a non-skin side of the first member by a joining portion, the joining portion being provided between a one-side end and a other-side end of the first member in the lengthwise direction.

According to the above-described absorbent article, during the post handling operation, when the second member of the post handling tape is pulled, a tensile force is likely to be dispersed to two lengthwise sides (one side and the other side) of the first member with the joining portion being centered. Therefore, compared with a case where the tensile force to the attachment (first member) (a force that attempts to pull apart the attachment) acts in a concentrated manner at a single position, the attachment can be made less likely to peel off.

In such an absorbent article, it is desirable that in the first member, a portion located on a lengthwise one side with respect to the joining portion and a portion located on a lengthwise other side with respect to the joining portion are each attached to the hydrophilic nonwoven fabric.

According to the above-described absorbent article, in the case where a force that pulls the first member acts through the joining portion adhering to the second member during the post handling operation, the attaching portions provided on two lengthwise side of the joining portion of the first member allows the first member to resist the force that pulls the first member. Accordingly, the post handling tape is less likely to be separated from the hydrophilic nonwoven fabric, and the user can more easily perform the post handling operation of the absorbent article.

### First Example

The following describes an absorbent article by way of an example of a disposable diaper (hereinafter also referred to as a "diaper 1"). However, the absorbent article includes napkins, panty liners, and other types of absorbent article.

### Configuration of Diaper 1

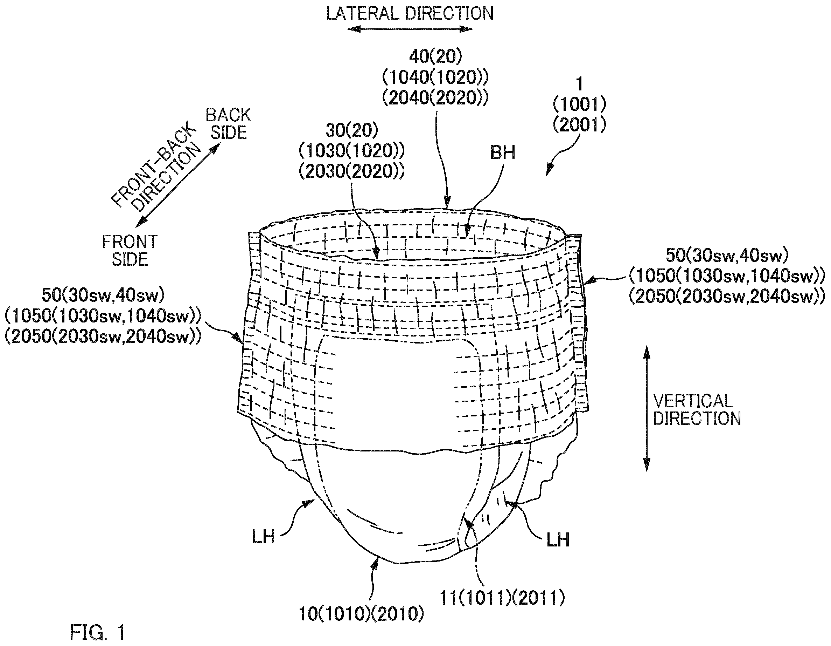

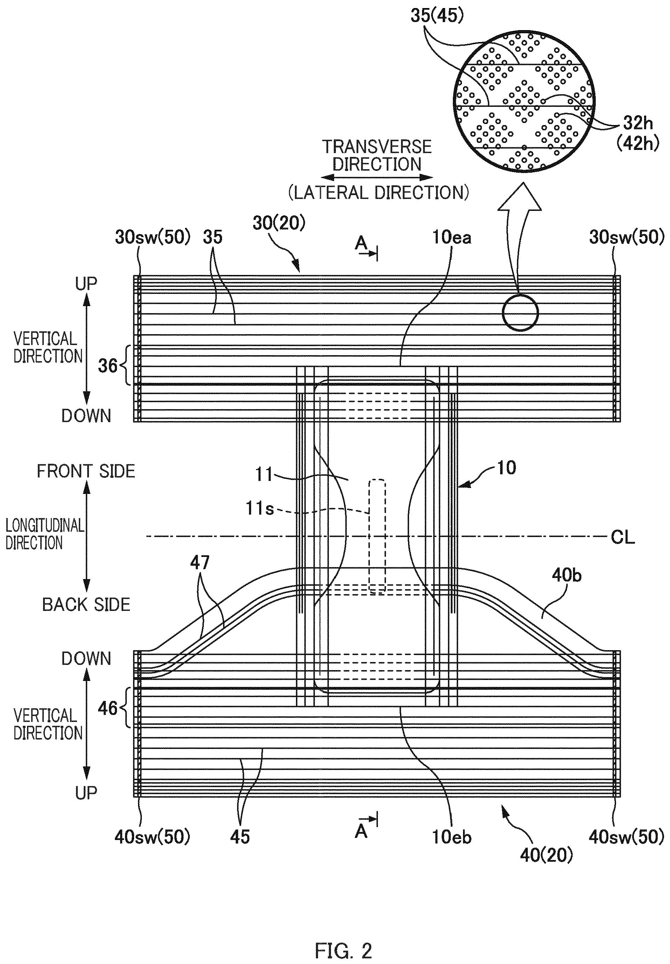

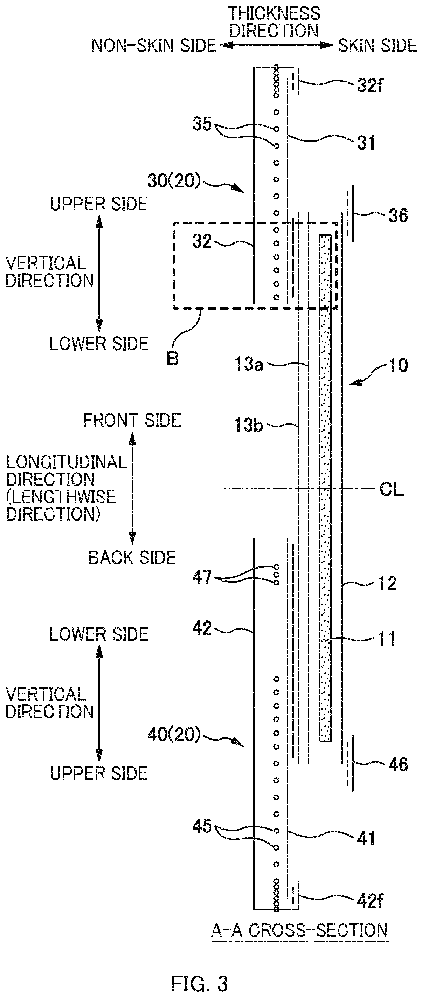

FIG. 1 is a schematic perspective view of the diaper 1. FIG. 2 is a plan view of the diaper 1 in an unfolded and stretched state. FIG. 3 is a schematic cross-sectional view taken along a line A-A in FIG. 2. It should be noted that the "stretched state" of the diaper 1 refers to a state where the entire diaper 1 (the entire product) is stretched without being wrinkled, specifically, a state where the diaper is stretched such that the dimensions of constituent members of the diaper 1 (e.g., an absorbent main body 10, a waist member 20, or the like to be described later) match or are close to the dimensions of the members on their own. Further, FIG. 1 also commonly shows a diaper 1001 (fifth example) and a diaper 1002 (ninth example), which will be described later.

The diaper 1 is a disposable diaper having an underpants shape in a natural state, and in the underpants-shaped state in FIG. 1, the diaper 1 has a vertical direction, a lateral direction, and a front-back direction that intersect with each other and has a waist opening BH and a pair of leg openings LH and LH. The upper side in the vertical direction corresponds to the waist opening BH side, and the lower side corresponds to the crotch side. In addition, the front side in the front-back direction corresponds to the wearer's front side, and the back side corresponds to the wearer's back side. Further, in an unfolded state in FIG. 2, the diaper 1 has a longitudinal direction and a transverse direction that intersect with each other. The longitudinal direction is a direction extending along the vertical direction in FIG. 1 and corresponds to the lengthwise direction of the absorbent main body 10. The transverse direction is a direction extending along the lateral direction in FIG. 1. In addition, as shown in FIG. 3, a direction in which constituent members of the diaper 1 are overlaid is referred to as a thickness direction. In the thickness direction, the side that comes into contact with the wearer's skin is defined as the skin side, and the side opposite to the skin side is defined as the non-skin side.

The diaper 1 has a liquid-absorbent absorbent main body 10 that absorbs excrement, and a waist member 20 that is arranged on the non-skin side of the absorbent main body 10. The waist member 20 is an exterior member that constitutes the exterior of the diaper 1, and has a front waist portion 30 corresponding to the front panel of the diaper 1 and a back waist portion 40 corresponding to the back panel of the diaper 1. That is, the diaper 1 of the first example is a so-called three-piece type underpants-shaped diaper including: as a first component, an absorbent main body 10 that is applied to the wearer's crotch portion and absorbs excrement such as urine or the like; as a second component, a front waist portion 30 that covers the wearer's stomach side portion; and as a third component, a back waist portion 40 that covers the wearer's back side portion.

In an unfolded state in FIG. 2, in a state where the front waist portion 30 and the back waist portion 40 are arranged side by side in the longitudinal direction with a space with respect to each other, lengthwise (longitudinal) end portions 10ea and 10eb of the absorbent main body 10 are respectively joined and fixed to the skin side of the nearest waist portions 30 and 40 while the absorbent main body 10 is spanned between the front waist portion 30 and the back waist portion 40. The external shape thereof forms a substantially H shape in a plan view. Then, from this state, the absorbent main body 10 is folded one time at a fold position, which is the longitudinal central position CL. In this folded state, the front waist portion 30 and the back waist portion 40 that face each other are joined and connected to each other at two lateral side portions 30sw and 40sw to form a pair of side joining portions 50 and 50. That is, the front waist portion 30 and the back waist portion 40 are shaped into an annular shape by the pair of side joining portions 50 and 50. It should be noted that the side joining portion 50 is formed by commonly-known joining means such as welding or adhesive. Accordingly, the diaper 1 is in an underpants-shaped state in which the waist opening BH and the pair of leg openings LH and LH are formed as shown in FIG. 1.

### Absorbent Main body 10

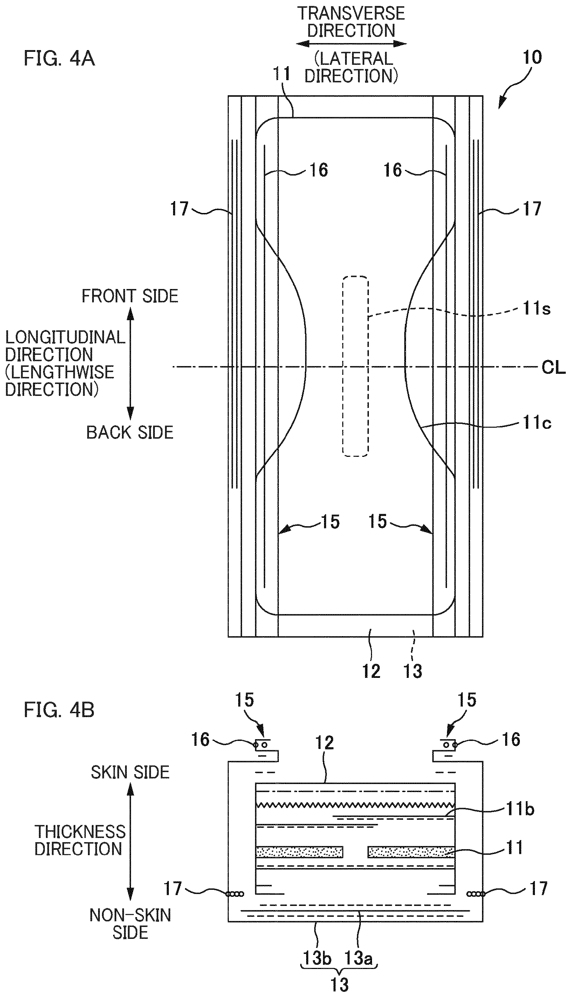

FIG. 4A is a plan view of the absorbent main body 10, and FIG. 4B is a schematic cross-sectional view of the absorbent main body 10. The absorbent main body 10 has an absorbent core 11 that absorbs excreted fluids, a top sheet 12 that is arranged on the skin side in the thickness direction with respect to the absorbent core 11, and a back sheet 13 that is arranged on the non-skin side with respect to the absorbent core 11. However, the absorbent main body 10 may include other sheet members. For example, a second sheet (not shown) may be provided between the top sheet 12 and the absorbent core 11 in the thickness direction.

The absorbent core 11 is a member that absorbs and holds excreted fluid such as urine, and is formed of, for example, a liquid absorbent fiber (e.g., a pulp fiber) containing a superabsorbent polymer (SAP). It should be noted that the outer circumferential surface of the absorbent core 11 may be covered with a liquid-permeable sheet member (core-wrapping sheet 11b) such as tissue paper or nonwoven fabric. The absorbent core 11 of the present example has a narrow portion 11c, which has a narrow width in the lateral direction, between a front end and a back end in the lengthwise direction, and has a substantially hourglass shape in a plan view as shown in FIG. 4A. This narrow portion 11c is a portion sandwiched between the wearer's two legs while the diaper 1 is put on, and the lateral length thereof is small (the width is narrow), making the absorbent core 11 easier to fit to the wearer's crotch.

Further, in the absorbent core 11, a slit portion 11s that penetrates the absorbent core 11 in the thickness direction is provided. The slit portion 11s is formed in a substantially rectangular shape extending along the longitudinal direction (lengthwise direction), and is provided at one location in the lateral (transverse) central portion of the absorbent core 11. However, the shape and the number of the slit portion 11s are not limited to those shown in FIG. 4. Further, the slit portion 11s need not be provided in the absorbent core 11.

The top sheet 12 is a liquid-permeable sheet, and, for example, a hydrophilic air-through nonwoven fabric, spunbond nonwoven fabric, or the like may be used. In the present example, as shown in FIG. 4B, two lateral side portions are folded back toward the non-skin side so as to enclose the absorbent core 11.

The back sheet 13 has a two-layer structure including a liquid-impermeable sheet 13a and an exterior sheet 13b arranged on the non-skin side of the liquid-impermeable sheet 13a. As the liquid-impermeable sheet 13a, a liquid-impermeable and moisture-permeable sheet member may be used. For example, it is possible to use a microporous breathable resin film in which a plurality of fine pores are provided in a sheet mainly made of a resin such as polyethylene or polypropylene. In the present specification, the liquid-impermeable sheet 13a will also be referred to as a "breathable film". That is, the breathable film is a sheet member that is not permeable to liquid, but is permeable to water vapor or air. On the other hand, as the exterior sheet 13b, a hydrophobic nonwoven fabric having flexibility may be used. For example, it is possible to use an air-through nonwoven fabric, a spunbond nonwoven fabric, or the like.

On two lateral side portions of the absorbent main body 10, a pair of the leak-proof wall portions 15 are respectively provided along the longitudinal direction (the lengthwise direction of the absorbent main body 10). In the present example, the leak-proof wall portion 15 is formed by the above-described exterior sheet 13b. Specifically, in the lateral direction (transverse direction), a part of the exterior sheet 13b is folded toward the skin side at a plurality of locations as shown in FIG. 4B, while extending outward with respect to two end portions of the absorbent core 11, to form the pair of leak-proof wall portions 15. To the skin-side end portion (leading end portion) of each leak-proof wall portion 15, leak-proof-wall elastic members 16 such as elastic strings are attached in a state of being stretched along the longitudinal direction (the lengthwise direction of the absorbent main body 10). While the diaper 1 is put on, the leak-proof wall portions 15 rise toward the wearer's skin side and fit to the wearer's crotch portion due to the stretchability developed by the leak-proof-wall elastic members 16.

Further, to two lateral side portions of the absorbent main body 10, leg elastic members 17 such as elastic strings are attached in a state of being stretched along the longitudinal direction (the lengthwise direction of the absorbent main body 10). While the diaper 1 is put on, the two side portions of the absorbent main body 10 contract and becomes more likely to fit around the wearer's legs due to the stretchability developed by the leg elastic members 17.

### Front Waist Portion 30

As shown in FIG. 3, the front waist portion 30 includes: a skin-side sheet 31 that is arranged farthest on the skin side in the thickness direction; a non-skin-side sheet 32 that is overlaid so as to be adjacent to the non-skin side of the skin-side sheet 31; and waist elastic members 35 that are provided between the skin-side sheet 31 and the non-skin-side sheet 32 in the thickness direction. The front waist portion 30 serving as an exterior member of the diaper 1 basically has a two-layer structure constituted by the skin-side sheet 31 and the non-skin-side sheet 32, but may partially have a configuration of three or more layers including a skin surface sheet 36 or the like described below.

The skin-side sheet 31 and the non-skin-side sheet 32 are sheet members having a rectangular shape in a plan view as shown in FIG. 2, and are formed of, for example, an SMS nonwoven fabric sheet or the like. In the diaper 1, the sheet member (nonwoven fabric sheet) that constitutes the non-skin-side sheet 32 has higher hydrophilicity than the sheet member (nonwoven fabric sheet) that constitutes the skin-side sheet 31. That is, the skin-side sheet 31 is formed of a nonwoven fabric sheet having a low hydrophilicity, and the non-skin-side sheet 32 is formed of a nonwoven fabric sheet having higher hydrophilicity than the skin-side sheet 31. Hereinafter, the nonwoven fabric that constitutes the skin-side sheet 31 will also be referred to as "hydrophobic nonwoven fabric", and the nonwoven fabric that constitutes the non-skin-side sheet 32 will also be referred to as "hydrophilic nonwoven fabric". The "hydrophilicity" of the nonwoven fabric will be described later.

Further, on the surface of the non-skin-side sheet 32, the plurality of hole portions 32h as shown in a partially enlarged view of FIG. 2 are provided. The hole portions 32h are each a through hole that penetrates the non-skin-side sheet 32 in the thickness direction. Providing the hole portions 32h makes it possible to enhance the breathability of the front waist portion 30. Further, arranging the hole portions 32h visibly on the non-skin surface side of the front waist portion 30 makes the user more likely to invoke that the front waist portion 30 has good breathability. Each of the hole portions 32h may have, for example, a circular shape having a diameter of approximately 1 mm, but the shape and arrangement (number and pattern) of the hole portions 32h can be appropriately changed. It should be noted that, in the diaper 1, the through hole corresponding to the hole portion 32h is not provided in the skin-side sheet 31.

The front waist portion 30 of the present example has a folded-back portion 32f in which the upper end portion (the front end portion in the longitudinal direction) of the non-skin-side sheet 32 is folded back from the non-skin side to the skin side and from the front side to the back side in the longitudinal direction. By covering a part (upper end portion) of the skin-side sheet 31 with the folded-back portion 32f, the upper end edge of the skin-side sheet 31 is prevented from biting into the wearer's skin. However, the folded-back portion 32f need not have to be necessarily provided.

Between the skin-side sheet 31 and the non-skin-side sheet 32, a plurality of waist elastic members 35 are arranged side by side in the vertical direction, and are attached in a state of being stretched in the lateral direction. The front waist portion 30 fits around the wearer's front waist due to the stretchability developed by the waist elastic member 35.

The waist elastic members 35 can be attached using an adhesive such as a hot-melt adhesive. For example, it is possible to attach the waist elastic members 35 by applying a hot-melt adhesive to each of them, stretching the waist elastic member 35 at a predetermined stretch factor, and sandwiching the waist elastic member 35 between the skin-side sheet 31 and the non-skin-side sheet 32. That is, the skin-side sheet 31 and the non-skin-side sheet 32 are joined using the adhesive with the waist elastic members 35 interposed therebetween. Further, the waist elastic members 35 may be attached by applying an adhesive to the skin-side sheet 31 side and the non-skin-side sheet 32 side, or the waist elastic member 35 may be attached by welding means in which a later-described welding portion 60 is used.

Further, the front waist portion 30 may have the skin surface sheet 36. As shown in FIG. 3, the skin surface sheet 36 is a sheet member arranged so as to cover the upper end portion (the front end portion in the longitudinal direction) of the absorbent main body 10 from the skin side, and functions as a cover sheet. Accordingly, the upper end edge of the absorbent main body 10 is prevented from biting into the wearer's skin while the diaper 1 is put on. The skin surface sheet 36 is formed of, for example, an SMS nonwoven fabric sheet or the like. It should be noted that the skin surface sheet 36 need not be necessarily provided.

In the front waist portion 30 of the present example, in the case where a sheet member is provided on the skin side with respect to the skin-side sheet 31 or on the non-skin side with respect to the non-skin-side sheet 32, these sheets are arranged so as to cover only a part of the skin-side sheet 31 and the non-skin-side sheet 32. For example, the skin surface sheet 36 in FIG. 3 is provided so as to cover only a part of the skin-side sheet 31, and at least a part of the skin-side sheet 31 is in a state of being exposed to the wearer's skin side.

### Back Waist Portion 40

The back waist portion 40 has substantially the same configuration as the front waist portion 30. That is, the back waist portion 40 includes: a skin-side sheet 41 that is arranged farthest on the skin side in the thickness direction; a non-skin-side sheet 42 that is overlaid so as to be adjacent to the non-skin side of the skin-side sheet 41; and waist elastic members 45 that is provided between the skin-side sheet 41 and the non-skin-side sheet 42 in the thickness direction. Further, similar to the front waist portion 30, the back waist portion 40 may have hole portions 42h, a folded-back portion 42f, a skin surface sheet 46, and the like (see FIGS. 2 and 3). The configuration of the members is substantially the same as that of the front waist portion 30, and therefore description thereof is omitted.

On the other hand, the external shape of the back waist portion 40 is different from the external shape of the front waist portion 30. Specifically, as shown in FIG. 2, the back waist portion 40 has a buttocks cover 40b whose lower portion in the vertical direction with respect to the side joining portion 50 (side portion 40sw) has a substantially trapezoidal shape. The buttocks cover 40b is a portion whose lateral width is narrowed from the upper side to the lower side in the vertical direction and whose outer edge is curved. By providing the buttocks cover 40b, the back waist portion 40 can widely cover the wearer's buttocks while the diaper 1 is put on.

Further, in the buttocks cover 40b, curved elastic members 47 such as elastic strings as shown in FIG. 2 are provided. The curved elastic members 47 are each attached between the skin-side sheet 41 and the non-skin-side sheet 42 in a state of being stretched along the end edge portion of the buttocks cover 40b. The stretchability developed by the curved elastic members 47 makes the buttocks cover 40b of the back waist portion 40 likely to fit to the wearer's buttocks while the diaper 1 is put on, and also makes it difficult to be turned up from the buttocks.

### Hydrophilicity of Sheet Member

Here, the hydrophilicity of the sheet member will be described. In the diaper 1, a hydrophobic nonwoven fabric is used as the skin-side sheets 31 and 41 that constitute the waist member 20 (the front waist portion 30 and the back waist portion 40) that is the exterior member, and the exterior sheet 13b of the absorbent main body 10. On the other hand, as the non-skin-side sheets 32 and 42 that constitute the waist member 20, a hydrophilic nonwoven fabric having higher hydrophilicity than a hydrophobic nonwoven fabric is used.

The hydrophilic nonwoven fabric of the present example has increased hydrophilicity by undergoing a treatment for attaching a predetermined oil agent to the hydrophobic nonwoven fabric (hydrophilic treatment). As the oil agent used in the hydrophilic treatment, it is possible to use commercially available oil agents having an effect as antistatic agents for fibers, such as anionic oil agents, nonionic oil agents, and blends thereof. These oil agents are put into an oil tank and then subjected to oiling with an oiling roller or the like. As a result, the hydrophilicity of the hydrophobic nonwoven fabric can be enhanced, obtaining the hydrophilic nonwoven fabric. However, the hydrophilic nonwoven fabric may be formed by other methods. For example, a hydrophilic nonwoven fabric may be obtained by manufacturing a nonwoven fabric using highly hydrophilic fibers.

It should be noted that, in the present example, it is assumed that the entire nonwoven fabric that constitutes the non-skin-side sheets 32 and 42 is subject to the hydrophilic treatment, and the hydrophilicity of the entirety of each constituent sheet member is enhanced. However, a configuration is possible in which the hydrophilicity is enhanced only in a partial region of each sheet member. For example, the sheet member may have a locally high hydrophilic portion and a locally low hydrophilic portion by undergoing a hydrophilic treatment only on the partial region of the non-skin-side sheet 32.

The hydrophilicity of a sheet member can be evaluated by measuring a contact angle when ion exchange water is brought into contact with the surface of the sheet member. Specifically, in the case where the contact angle between the hydrophilic nonwoven fabric and the ion exchange water is smaller than the contact angle between the hydrophobic nonwoven fabric and the ion exchange water, the hydrophilicity of the hydrophilic nonwoven fabric becomes higher than the hydrophilicity of the hydrophobic nonwoven fabric. For the hydrophilic nonwoven fabric (non-skin-side sheet 32 or the like) used in the present example, the contact angle with the ion exchange water is preferably less than 90°, and more preferably 50° or less. On the other hand, for the hydrophobic nonwoven fabric (skin-side sheet 31), the contact angle with the ion exchange water is preferably 90° or more, and more preferably 120° or more.

The contact angle can be measured by the following method using, for example, a contact angle meter MCA-J manufactured by Kyowa Interface Science Co., Ltd. First, ion exchange water is dropped (approximately 20 picoliters) onto the surface of the fibers that constitute a sheet member (sheet to be measured), and then immediately the contact angle is measured using the contact angle meter. The measurement is performed at a plurality of places (e.g., five or more places) on the surface of the sheet to be measured, and the average value of these positions is defined as the contact angle. It should be noted that the measurement environment temperature is set to 22°C.

Alternatively, the contact angle may be measured by capturing images of the sheet to be measured onto which ion exchange water is dropped from the cross-sectional direction of the sheet to be measured, analyzing the captured image, and measuring the angle between the ion exchange water droplet and the sheet to be measured.

Absorption and Discharge of Moisture by Absorbent main body 10 In the diaper 1, excreted fluid (moisture) such as urine is absorbed by the absorbent main body 10 (absorbent core 11), but by appropriately discharging the absorbed moisture to the outside of the diaper 1, the water retaining capacity (the amount of moisture that can be absorbed) of the absorbent core 11 is prevented from reaching the limit, making it easier to maintain the water absorption function and make it less likely to cause stuffiness inside of the absorbent main body 10.

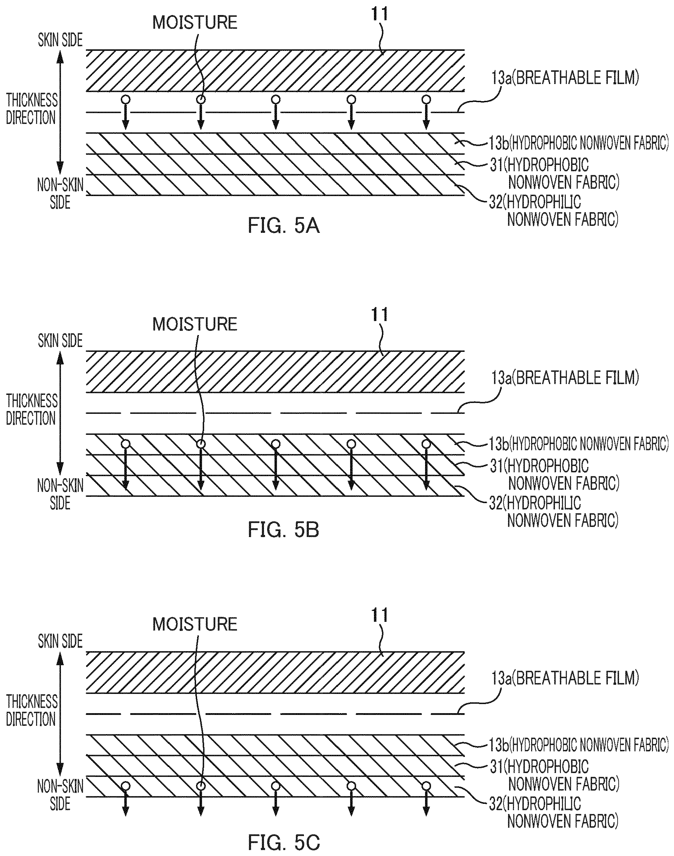

FIGS. 5A to 5C are explanatory diagrams illustrating a mechanism by which moisture is discharged from the absorbent main body 10 in a region B in FIG. 3. In FIGS. 5A to 5C, a state is shown in which the absorbent core 11, the liquid-impermeable sheet 13a (breathable film), the exterior sheet 13b (hydrophobic nonwoven fabric), the skin-side sheet 31 (hydrophobic nonwoven fabric), and the non-skin-side sheet 32 (hydrophilic nonwoven fabric) are overlaid in this order from the skin side toward the non-skin side in the thickness direction.

When urine or the like is excreted while the diaper 1 is put on, moisture of the urine or the like is absorbed by the absorbent core 11. Then, as shown in FIG. 5A, part of the absorbed moisture permeates the liquid-impermeable sheet 13a (breathable film) as water vapor from the skin side to the non-skin side in the thickness direction and moves toward the exterior sheet 13b and the skin-side sheet 31. Although the exterior sheet 13b and the skin-side sheet 31 are each hydrophobic nonwoven fabric sheet, voids are formed by multiple fibers that constitute the nonwoven fabric sheet, and thus part of moisture (water vapor) is drawn into the exterior sheet 13b and the skin-side sheet 31 due to the capillary phenomenon caused by the voids.

Subsequently, as shown in FIG. 5B, the moisture drawn into the exterior sheet 13b and the skin-side sheet 31 is transferred to the non-skin-side sheet 32 which is adjacent to the non-skin side in the thickness direction. Since the non-skin-side sheet 32 is a hydrophilic nonwoven fabric sheet having higher hydrophilicity than the exterior sheet 13b and the skin-side sheet 31, the moisture contained in the exterior sheet 13b and the skin-side sheet 31 is likely to transfer to the hydrophilic nonwoven fabric side (the non-skin-side sheet 32 side) due to the hydrophilic gradient generated between the hydrophilic nonwoven fabric and the hydrophobic nonwoven fabric.

Then, as shown in FIG. 5C, moisture (water vapor) that is transferred to the non-skin-side sheet 32 is evaporated from the non-skin-side surface of the non-skin-side sheet 32 to the outside (the atmosphere side in FIG. 5C). In the diaper 1, the non-skin-side sheet 32 is arranged on the outermost (non-skin-side) surface of the absorbent main body 10. That is, the non-skin-side surface of the non-skin-side sheet 32 serves as an interface with the atmosphere. Therefore, the moisture contained in the non-skin-side sheet 32 can be efficiently evaporated to the outside of the diaper 1 from a wide range of the non-skin-side surface. However, it is not necessary that the hydrophilic nonwoven fabric is arranged on the entire non-skin-side surface of the exterior member. For example, another member (e.g., a tape member) may be provided on a part of the farthest-non-skin-side surface of the exterior member.

In a conventional absorbent article such as a disposable diaper, it is common that a sheet member having high hydrophilicity (the non-skin-side sheet 32 in the diaper 1) is not provided on the non-skin side with respect to the absorbent core and the breathable film (the liquid-impermeable sheet 13a in the diaper 1). Therefore, water vapor that permeates the breathable film is more likely to remain in a portion of the sheet member having low hydrophilicity (hydrophobic nonwoven fabric) provided on the non-skin side. That is, the configuration is such that moisture is more likely to be accumulated between the breathable film (corresponding to the liquid-impermeable sheet 13a) and the hydrophobic nonwoven fabric (the exterior sheet 13b and the skin-side sheet 31), and this may cause stuffiness or the like in the diaper.

In contrast, in the diaper 1 of the present example, a hydrophilic nonwoven fabric having high hydrophilicity (non-skin-side sheet 32) is provided on the non-skin side with respect to the breathable film (liquid-impermeable sheet 13a) and the hydrophobic nonwoven fabric (exterior sheet 13b and skin-side sheet 31). Therefore, moisture is attracted to the hydrophilic nonwoven fabric side (non-skin side) having higher hydrophilicity, and the moisture is less likely to be accumulated between the breathable film (corresponding to the liquid-impermeable sheet 13a) and the hydrophobic nonwoven fabric (exterior sheet 13b or skin-side sheet 31). That is, the configuration is such that the moisture absorbed by the absorbent core 11 is easily transferred from the skin side to the non-skin side in the thickness direction.

It should be noted that in the diaper 1, a hydrophobic nonwoven fabric (exterior sheet 13b or skin-side sheet 31) is provided between the liquid-impermeable sheet 13a (breathable film) and the non-skin-side sheet 32 (hydrophilic nonwoven fabric) in the thickness direction. However, even in the case where the hydrophobic nonwoven fabric is not provided, moisture can be discharged as described above. At least, it is sufficient that the breathable film is arranged on the non-skin side with respect to the absorbent core, and the sheet member having high hydrophilicity (hydrophilic nonwoven fabric) is arranged on the non-skin side with respect to the breathable film. According to the above-described configuration, the movement of moisture in the thickness direction is promoted, and moisture such as urine absorbed by the absorbent core can be efficiently discharged to the outside.

In this manner, in the diaper 1, the absorbent core, the breathable film, and the hydrophilic nonwoven fabric are arranged to overlap each other in the thickness direction, making it easier to promote the movement of moisture in the thickness direction in a region in which the three members overlap each other. That is, moisture is easily discharged to the outside from the inside (absorbent main body) of the diaper 1. Therefore, moisture is less likely to remain inside of the diaper 1, the water absorption capacity of the absorbent core 11 is less likely to reach the limit, and the water absorption function is more likely to maintain. Further, the wearer can be made less likely to feel discomfort such as stuffiness while the diaper 1 is put on.

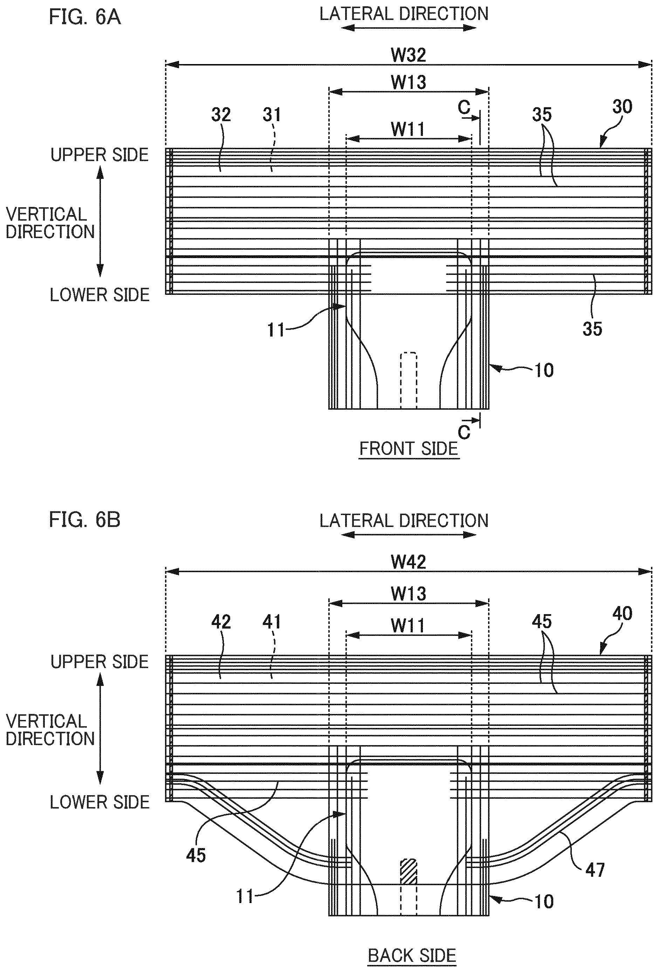

FIG. 6A is a schematic plan view showing a state of the diaper 1 having an underpants shape when viewed from the front side in the front-back direction in a stretched state. FIG. 6B is a schematic plan view showing a state of the diaper 1 having an underpants shape when viewed from the back side in the front-back direction in a stretched state. As shown in FIGS. 6A and 6B, in the diaper 1, the lateral length (width) W13 of the liquid-impermeable sheet 13a (breathable film) is larger than the lateral length (width) W11 of the absorbent core 11 (W11 < W13). Further, in FIG. 6A, the lateral length (width) W32 of the non-skin-side sheet 32 (hydrophilic nonwoven fabric) is larger than the lateral length (width) W13 of the liquid-impermeable sheet 13a (breathable film) (W13 < W32). Further, in FIG. 6B, the lateral length (width) W42 of the non-skin-side sheet 42 (hydrophilic nonwoven fabric) is larger than the lateral length (width) W13 of the liquid-impermeable sheet 13a (breathable film) (W13 < W42).

As shown in FIGS. 6A and 6B, the absorbent core 11 overlaps the liquid-impermeable sheet 13a (breathable film) throughout the lateral direction, and this allows moisture to permeate the breathable film from the absorbent core 11 throughout the lateral direction and the moisture (water vapor) to move to the non-skin side. The liquid-impermeable sheet 13a (breathable film) overlaps the non-skin-side sheets 32 and 42 (hydrophilic nonwoven fabric) throughout the lateral direction, and this makes the hydrophilic nonwoven fabric on the non-skin side more likely to absorb moisture that has permeated the breathable film. Accordingly, moisture is likely to be transferred from the entire lateral region of the absorbent core 11 to the hydrophilic nonwoven fabric, making it possible to efficiently discharge the moisture from the inside (skin side) to the outside (non-skin side) of the diaper 1.

Further, from the above-described relationship, the diaper 1 has a portion in which the non-skin-side sheets 32 and 42 (hydrophilic nonwoven fabrics) and the absorbent core 11 overlap each other when viewed in the thickness direction and a portion in which the non-skin-side sheets 32 and 42 (hydrophilic nonwoven fabrics) do not overlap each other when viewed in the thickness direction. In the portion in which the absorbent core 11 and the hydrophilic nonwoven fabric overlap each other (a region indicated by W11 in FIGS. 6A and 6B), moisture such as urine absorbed by the absorbent core 11 can be efficiently discharged to the outside of the diaper 1 as described above.

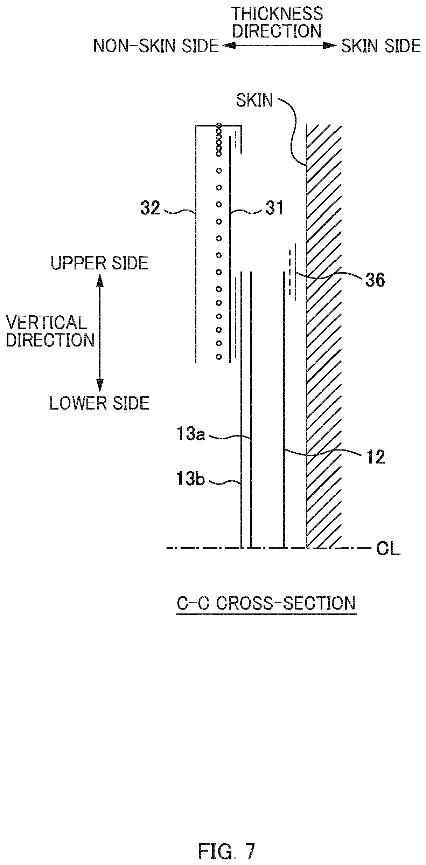

On the other hand, in the region in which the absorbent core 11 and the hydrophilic nonwoven fabric do not overlap each other, moisture such as sweat from the wearer's body can be transferred to the hydrophilic nonwoven fabric side. FIG. 7 is a schematic cross-sectional view taken along a line C-C in FIG. 6A. FIG. 7 is a diagram corresponding to FIG. 3 and shows a cross-sectional view of a portion in which the absorbent core 11 and the hydrophilic nonwoven fabric do not overlap each other. In FIG. 7, when the wearer sweats, the sweat that has permeated the top sheet 12 of the absorbent main body 10 permeates the liquid-impermeable sheet 13a (breathable film) as water vapor, is transferred to the non-skin-side sheet 32 (hydrophilic nonwoven fabric) through the exterior sheet 13b and the skin-side sheet 31, and is finally evaporated to the outside of the diaper 1. That is, moisture can be transferred from the skin side to the non-skin side of the diaper 1 and the moisture can be discharged from the surface of the hydrophilic nonwoven fabric to the non-skin side. Therefore, in the diaper 1, in addition to moisture such as urine absorbed by the absorbent core 11, moisture such as sweat attached to the wearer's skin can be discharged to the outside. Accordingly, this makes it possible to further suppress the occurrence of stuffiness while the diaper 1 is put on.

It should be noted that in at least a part of the region in which the hydrophilic nonwoven fabric and the absorbent core 11 do not overlap each other, the hydrophobic nonwoven fabric is arranged between the hydrophilic nonwoven fabric and the breathable film in the thickness direction. For example, in the case of FIG. 7, the exterior sheet 13b and the skin-side sheet 31 (both formed of a hydrophobic nonwoven fabric) are provided between the non-skin-side sheet 32 (hydrophilic nonwoven fabric) and the liquid-impermeable sheet 13a (breathable film). In this case, as described above, the hydrophilic gradient generated between the hydrophilic nonwoven fabric and the hydrophobic nonwoven fabric makes it easier for moisture to be transferred from the hydrophobic nonwoven fabric side to the hydrophilic nonwoven fabric side. That is, moisture is easily smoothly transferred from the skin side to the non-skin side. Therefore, moisture such as sweat can be easily discharged to the outside of the diaper 1.

Further, the waist member 20 of the diaper 1 is provided with the waist elastic members 35 and 45 that stretch and contract in the lateral direction, and the curved elastic members 47. But all of these elastic members is prevented from developing stretchability in a part of a region that overlaps the absorbent main body 10. For example, in FIGS. 6A and 6B, among the waist elastic members 35 and 45 and the curved elastic members 47, some elastic members are cut at the lateral central portion of the absorbent main body 10, and thereby the elastic members are in a state of being contracted from the cut portion toward two lateral sides (regions that straddle two lateral ends of the absorbent main body 10, hereinafter, also referred to as "two side portions"). Therefore, the contractive force caused by the elastic members 35, 45, and 47 that acts on the lateral central portion of the absorbent main body 10 is weaker than the contractive force caused by the elastic members 35, 45, and 47 that acts on two lateral side portions. In other words, the stress of the elastic members 35, 45, and 47 in the lateral central portion of the absorbent main body 10 is smaller than the stress of the elastic members 35, 45, and 47 in the two lateral side portions of the absorbent main body 10. It should be noted that the elastic members 35, 45, and 47 are not necessarily cut in the lateral central portion of the absorbent main body 10, and it is sufficient that the stress become weak in the lateral central portion compared with the two lateral side portions.

In such a configuration, in the lateral central portion of the absorbent main body 10 (absorbent core 11), the contractive force of the elastic members 35, 45, and 47 is less likely to act, wrinkles and the like are less likely to be formed on the surfaces of the absorbent main body 10 and the waist member 20, making the planar shape likely to be maintained. Therefore, as illustrated in FIGS. 5A to 5C, the laminate structure of the sheet members in the thickness direction is more likely to be maintained, and the movement of moisture in the thickness direction is less likely to be inhibited. Accordingly, moisture such as urine absorbed by the absorbent core 11 on the skin side can be transferred to the hydrophilic nonwoven fabric on the non-skin side and evaporated toward the non-skin side with respect to the hydrophilic nonwoven fabric.

The stress of the elastic member can be measured as follows using, for example, an autograph tensile tester (e.g., AG-1KN1) manufactured by Shimadzu Corporation. First, in a state where the waist member 20 (30 and 40) of the diaper 1 is stretched to an extent that no wrinkles are formed on the surface thereof, there are measured the lateral dimensions of to-be-measured regions in regions where the elastic members 35, 45, and 47 are arranged (the lateral central portion and two side portions of the absorbent main body 10). Then a cutter is used to cut the to-be-measured regions to the dimensions to obtain samples. Next, the one-side end of the sample is pinched between a fixed chuck, the other-side end is pinched between a movable chuck. After stretching the sample at a speed of 300 mm/min to a length of about 90% the initial dimension, the sample is inverted, and then a tensile load (N) is obtained when contracting the sample to a length of about 75% the initial dimension. Thus, the tensile load is converted into a stress (N/mm) per unit length (mm), and is used as a stress.

Further, the absorbent main body 10 of the diaper 1 has a slit portion 11s that penetrates the absorbent core 11 in the thickness direction. Then, the diaper has a portion in which the slit portion 11s and the hydrophilic nonwoven fabric overlap each other when viewed in the thickness direction. For example, in the diaper 1, in a region indicated by hatched portions in FIG. 6B, the slit portion 11s and the hydrophilic nonwoven fabric (the non-skin-side sheet 42 of the back waist portion 40) overlap each other.

The stiffness is low in the slit portion 11s in the absorbent core 11, and it enables the absorbent core 11 to easily deform along the body shape of the wearer. On the other hand, since the slit portion 11s is formed in a groove shape, there is a risk that moisture such as excrement is likely to be accumulated therein or likely to move thereto, causing discomfort for the wearer. In contrast, in the diaper 1, since the hydrophilic nonwoven fabric is provided on the non-skin side of the slit portion 11s, the diaper 1 has a structure that makes it easier to transfer moisture from the skin side to the non-skin side and to evaporate moisture to the non-skin side (the outside of the diaper 1) with respect to the hydrophilic nonwoven fabric as described above. Therefore, by making moisture more likely to be discharged to the outside while maintaining good fit of the absorbent core 11 to the body, it is possible for the wearer to be less likely to feel discomfort.

Further, in the diaper 1, it is desirable that the moisture permeability of the liquid-impermeable sheet 13a (breathable film) is equal to or greater than 1000 g/m²·24 h and equal to or smaller than 3500 g/m²·24 h. In the case where the moisture permeability is equal to or greater than 1000 g/m²·24 h, water vapor easily permeates the liquid-impermeable sheet 13a, and moisture can be easily evaporated from the inside (skin side) to the outside (non-skin side) of the diaper 1. On the other hand, in the case where the moisture permeability is equal to or smaller than 3500 g/m²·24 h, it is possible to suppress the excessive permeation of water vapor by the liquid-impermeable sheet 13a and to suppress the excessive absorption of moisture by the hydrophilic nonwoven fabric provided on the outside (non-skin side). Therefore, during usage of the diaper 1, the hydrophilic nonwoven fabric on the outside is prevented from becoming wet while discharging an appropriate amount of moisture, making it possible for the wearer to be less likely to feel discomfort.

It should be noted that the moisture permeability can be measured in accordance with JIS Z 0208 (Testing Methods for Determination of the Water Vapour Transmission Rate of Moisture-Proof Packaging Materials) as follows, for example. First, a plurality of samples (three or more circular samples having a diameter that is larger than the inner diameter of a moisture permeable cup used for measurement, by approximately 10 mm) are taken from the liquid-impermeable sheet 13a. Next, a moisture absorbent of JIS K 8123 [calcium chloride (anhydrous)] is put in a moisture permeable cup which has a moisture permeable area of 25 cm² or more, the sample is attached, sealed with a sealing wax, and left to stand in a constant temperature and humidity chamber for 16 hours or more. Then, the sample is taken out, and is allowed to equilibrate to room temperature, and the mass is measured using an electronic balance or the like. Thereafter, the sample is placed again in the constant temperature and humidity chamber, the weighing operation with the electronic balance is repeated at regular intervals (e.g., every 24 hours) to determine the mass increase, and the test is continued until the amount of the mass increase becomes constant within 5%. Then, for each sample, the moisture permeability is calculated by Equation (1) described below, and a value rounded off to two significant digits in accordance with JIS Z 8401 (Rounding of numbers) is defined as the moisture permeability of the liquid-impermeable sheet 13a.

### Moisture permeability = 240 m/(t·s) ... (1)

s: moisture permeable area (cm²)
t: total time (h) of the last two weighing intervals during test
m: total increased mass (mg) of the last two weighing intervals during test

### Modified Example of Method for Attaching Waist Elastic Members 35 and 45

In the above-described example, the waist elastic members 35 and 45 are attached to the waist member 20 by adhering means using a hot-melt adhesive or the like, but the method for attaching the waist elastic members 35 and 45 is not limited thereto. For example, the waist elastic members 35 and 45 may be attached to the waist member 20 using welding means such as ultrasonic welding. It should be noted that, since ultrasonic welding is a commonly-known technique, the description of ultrasonic welding is omitted in the present specification.

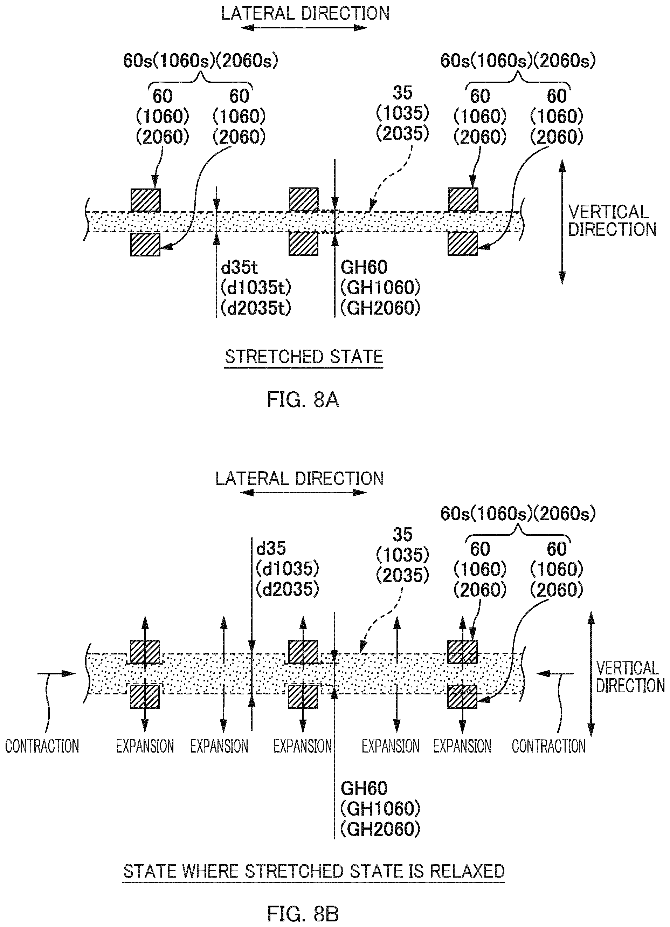

FIGS. 8A and 8B are explanatory diagrams illustrating a method for attaching the waist elastic member 35 to the front waist portion 30 using welding portions 60. In the present modified example, the waist elastic member 35 is attached to the front waist portion 30 by a plurality of welding portions 60, 60, ..., which are discretely arranged in the lateral direction and the vertical direction. Each of the welding portions 60 is formed into a substantially rectangular shape by ultrasonic welding, and joins the skin-side sheet 31 and the non-skin-side sheet 32 of the front waist portion 30 in the thickness direction. The waist elastic member 35 is attached to the front waist portion 30 by sandwiching the waist elastic member 35 from two vertical sides between welding portion pairs 60s each of which is formed of two welding portions 60 and 60 that are adjacent to each other in the vertical direction.

As shown in FIG. 8A, a pair of welding portions 60 and 60 that constitute the welding portion pair 60s are arranged side by side in the vertical direction with a space GH60. The size of the space GH60 is set to be the same size as or slightly larger than the diameter d35t of the waist elastic member 35 in a state where the waist elastic member 35 is stretched to a predetermined stretch factor (GH60 ≥ d35t). That is, the waist elastic member 35 in the stretched state is arranged between the welding portion pair 60s in the vertical direction.

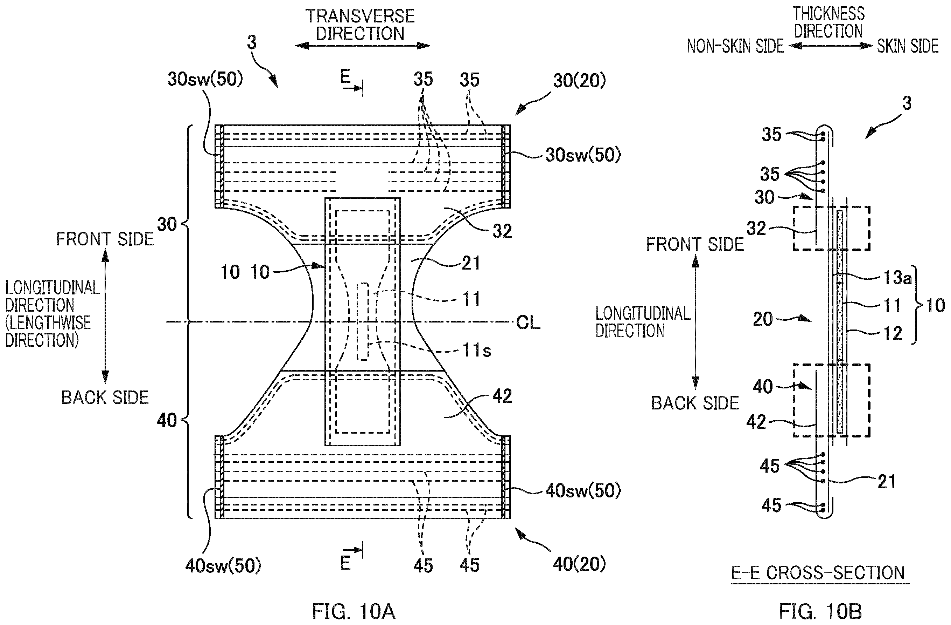

Next, when the waist elastic member 35 is relaxed from the stretched state, as shown in FIG. 10B, the waist elastic member 35 expands in the vertical direction while contracting in the lateral direction, and the diameter d35 in the natural state becomes larger than the vertical space GH60 of the welding portion pair 60s (d35 > GH60). Accordingly, the waist elastic member 35 is sandwiched between the welding portions 60 and 60 in the vertical direction. As a result, the waist elastic member 35 is attached to the front waist portion 30.

It should be noted that in the diaper 1 in the underpants-shaped state in FIG. 1, the waist elastic members 35 (45) are in a natural state where the waist elastic members 35 (45) are relaxed from the above-described stretched state. Further, in the diaper 1 in the underpants-shaped state, the waist elastic members 35 (45) are joined to the front waist portion 30 (back waist portion 40) by the side joining portions 50 and 50 on two lateral side portions. Therefore, even when the front waist portion 30 (back waist portion 40) is stretched in the lateral direction while the diaper 1 is put on, the waist elastic members 35 (45) are not detached from the waist member 20.

### Second Example

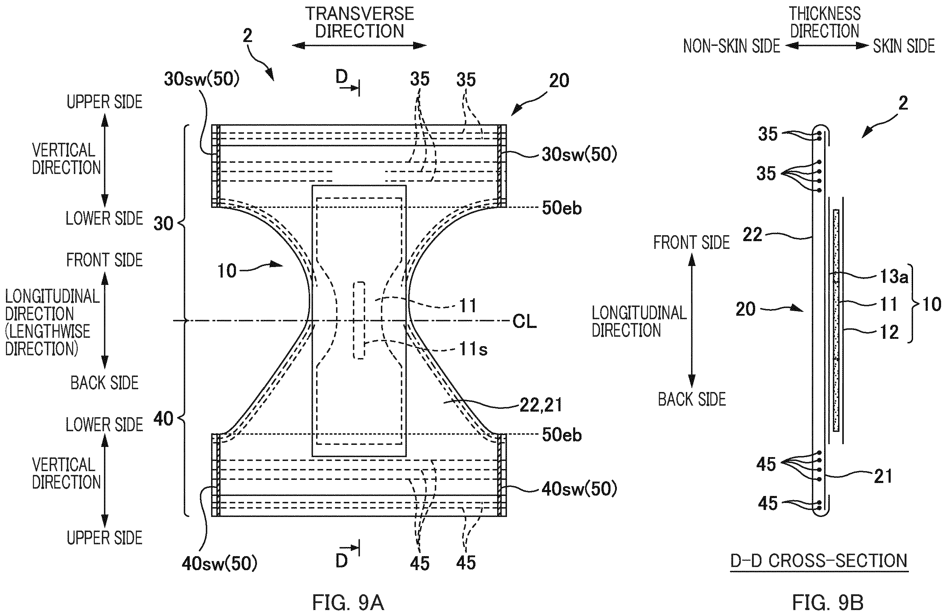

In a second example, an underpants-shaped diaper 2 (hereinafter, also referred to as a "diaper 2") having a configuration partially different from that of the first example will be described. FIG. 9A is a plan view of the diaper 2 in the unfolded and stretched state. FIG. 9B is a schematic cross-sectional view taken along a line D-D in FIG. 9A. It should be noted that the directions (e.g., the longitudinal direction, the transverse direction, or the like) in FIGS. 9A and 9B is the same as the directions defined in the first example.

The diaper 2 of the second example has the liquid-absorbent absorbent main body 10 and the waist member 20 that is joined to the non-skin side of the absorbent main body 10 as an exterior member. Further, as shown in FIGS. 9A and 9B, the waist member 20 of the diaper 2 is integrally constituted so as to be continuous in the longitudinal direction (the lengthwise direction of the absorbent main body 10). That is, the diaper 2 is a so-called two-piece type disposable diaper formed of two parts: the absorbent main body 10 and an exterior member (waist member 20). Hereinafter, in the exterior member (waist member 20) of the diaper 2, a portion located on the front side with respect to the longitudinal central position CL is defined as the front waist portion 30, and a portion located on the back side with respect to the central position CL is defined as the back waist portion 40 (see FIG. 9A).

When shaping the diaper 2 in the unfolded state in FIG. 9A into an underpants shape, the absorbent main body 10 and the waist member 20 are folded one time at a fold position, which is the longitudinal central position CL. In this folded state, the front waist portion 30 and the back waist portion 40 that face each other are joined and connected to each other at two lateral side portions 30sw and 40sw to form the pair of side joining portions 50 and 50. Accordingly, similar to the diaper 1 in FIG. 1, the diaper 2 is in an underpants-shaped state having a waist opening BH and a pair of leg openings LH and LH.

In the diaper 2, the basic configuration and function of the absorbent main body 10 are substantially the same as those of the absorbent main body 10 of the diaper 1 of the first example, and therefore description thereof is omitted. However, the back sheet 13 of the diaper 2 may include only the liquid-impermeable sheet 13a (breathable film) and need not include the exterior sheet 13b. This is because in the diaper 2, the waist member 20 is provided on the entire non-skin-side surface of the absorbent main body 10, and the waist member 20 functions as an exterior sheet of the absorbent main body 10. Therefore, as the back sheet 13 of the absorbent main body 10 of the diaper 2, a sheet member corresponding to the exterior sheet 13b of the diaper 1 need not be provided (see FIG. 9B).

The waist member 20 includes: a skin-side sheet 21; a non-skin-side sheet 22 that is overlaid to be adjacent to the non-skin side of the skin-side sheet 21; and waist elastic members 35 and 45 that are arranged between the skin-side sheet 21 and the non-skin-side sheet 22 in the thickness direction (see FIG. 9B). The skin-side sheet 21 is a hydrophobic nonwoven fabric sheet similar to the skin-side sheets 31 and 41 of the diaper 1. The non-skin-side sheet 22 is a hydrophilic nonwoven fabric sheet similar to the non-skin-side sheets 32 and 42 of the diaper 1. That is, in the diaper 2, the non-skin-side sheet 22 is a nonwoven fabric sheet having higher hydrophilicity than the skin-side sheet 21. Then, similar to the diaper 1, the waist elastic members 35 and 45 are formed of elastic strings or the like, and are attached between the skin-side sheet 21 and the non-skin-side sheet 22 in a state of being stretched in the lateral direction.

Also in the diaper 2 of the second example, the same effect as that of the diaper 1 can be obtained. For example, in the diaper 2, there is a portion in which the absorbent core 11, the breathable film (liquid-impermeable sheet 13a), and the hydrophilic nonwoven fabric (non-skin-side sheet 22) overlap each other when viewed in the thickness direction. In the portion in which the absorbent core 11, the breathable film, and the hydrophilic nonwoven fabric overlap each other, as illustrated in FIGS. 5A to 5C, the movement of moisture from the skin side to the non-skin side in the thickness direction is easily promoted, making moisture more likely to be discharged from the inside (the absorbent core 11) to the outside of the diaper 2. In addition, the diaper 2 basically has substantially the same features as those of the diaper 1 described in the first example.

Further, in the diaper 2, concerning the area of the region where the absorbent core 11 and the hydrophilic nonwoven fabric (non-skin-side sheet 22) overlap each other, the area is larger in the region located vertically above lower ends 50eb of the side joining portions 50 than in the region located vertically below the lower ends 50eb of the side joining portions 50 (see FIG. 9A). That is, in a region (crotch region) located below the lower ends 50eb of the side joining portions 50 and at the wearer's crotch while the diaper 2 is put on, the area of the region where the absorbent core 11 and the hydrophilic nonwoven fabric (non-skin-side sheet 22) overlap each other is increased.

Therefore, in the diaper 2, in a wide range of the crotch region, it enables moisture such as urine absorbed by the absorbent core 11 more likely to be discharged to the non-skin side with respect to the hydrophilic nonwoven fabric (to the outside of the diaper 2). Accordingly, stuffiness is less likely to occur in the crotch portion, and this makes it possible for the wearer to be less likely to feel discomfort.

Further, in the case of FIG. 9A, the entirety of breathable film (liquid-impermeable sheet 13a) overlaps the hydrophilic nonwoven fabric (non-skin-side sheet 22) when viewed in the thickness direction. Therefore, of urine or the like absorbed by the absorbent core 11, moisture that permeates the breathable film as water vapor from the skin side to the non-skin side becomes more likely to transfer to the hydrophilic nonwoven fabric located on the non-skin side in the entire region of the breathable film. Accordingly, the transfer of moisture from the skin side to the non-skin side in the thickness direction is more easily promoted, making it possible to make moisture less likely to remain inside of the diaper 2. However, as described above, in the case where the absorbent core 11, the breathable film (liquid-impermeable sheet 13a), and the hydrophilic nonwoven fabric (non-skin-side sheet 22) at least partially overlap each other, it is possible to obtain an effect of discharging moisture to the outside of the diaper 2, and it is not necessarily needed that the entire breathable film (liquid-impermeable sheet 13a) overlap the hydrophilic nonwoven fabric (non-skin-side sheet 22).

### Third Example

In a third example, an underpants-shaped diaper 3 (hereinafter, also referred to as a "diaper 3") having a configuration partially different from that of the above examples will be described. FIG. 10A is a plan view of the diaper 3 in the unfolded and stretched state. FIG. 10B is a schematic cross-sectional view taken along a line E-E in FIG. 10A. It should be noted that the directions (e.g., the longitudinal direction, the transverse direction, or the like) in FIGS. 10A and 10B is the same as the directions defined in the first example.

The diaper 3 of the third example has the liquid-absorbent absorbent main body 10 and the waist member 20 that is joined to the non-skin side of the absorbent main body 10 as an exterior member. As shown in FIGS. 10A and 10B, the waist member 20 of the diaper 3 is integrally constituted so as to be continuous in the longitudinal direction (the lengthwise direction of the absorbent main body 10). That is, the skin-side sheet 21 is configured as an integral sheet member that is continuous from an end portion located on the one side to an end portion located on the other side in the longitudinal direction. On the other hand, the non-skin-side sheet 32 arranged on the longitudinal front side (front non-skin-side sheet) and the non-skin-side sheet 42 arranged on the longitudinal back side (back non-skin-side sheet) are configured as different sheet members that are non-continuous in the longitudinal direction. A diaper having such a structure will also be referred to as a simple three-piece type disposable diaper. Further, hereinafter, in the exterior member (waist member 20) of the diaper 3, a portion located on the front side with respect to the longitudinal central position CL is defined as the front waist portion 30, and a portion located on the back side with respect to the central position CL is defined to as the back waist portion 40 (see FIG. 10A).

When shaping the diaper 3 in the unfolded state in FIG. 10A into an underpants shape, the absorbent main body 10 and the waist member 20 are folded one time at a fold position, which is the longitudinal central position CL. In this folded state, the front waist portion 30 and the back waist portion 40 that face each other are joined and connected to each other at two lateral side portions 30sw and 40sw to form the pair of side joining portions 50 and 50. Accordingly, similar to the diaper 1 in FIG. 1, the diaper 3 is in an underpants-shaped state having a waist opening BH and a pair of leg openings LH and LH.

In the diaper 3, the basic configuration and function of the absorbent main body 10 are substantially the same as those of the absorbent main body 10 of the diaper 2 of the second example, and therefore description thereof is omitted.

In the exterior member (waist member 20) of the diaper 3, the front waist portion 30 includes: the single skin-side sheet 21 that is located on the skin side in the thickness direction and extends from one end side (front side) to the other end side (back side) in the longitudinal direction; the non-skin-side sheet 32 that is located on the front side in the longitudinal direction and is overlaid to be adjacent to the non-skin side of the skin-side sheet 21; and the waist elastic members 35 that are arranged between the skin-side sheet 21 and the non-skin-side sheet 32 in the thickness direction. The back waist portion 40 includes: the skin-side sheet 21 that is common to the front waist portion 30; the non-skin-side sheet 42 that is located on the back side in the longitudinal direction and is overlaid to be adjacent to the non-skin side of the skin-side sheet 21; and the waist elastic members 45 that are arranged between the skin-side sheet 21 and the non-skin-side sheet 42 in the thickness direction.

The skin-side sheet 21 is a hydrophobic nonwoven fabric sheet similar to the skin-side sheets 31 and 41 of the diaper 1. The non-skin-side sheets 32 and 42 are hydrophilic nonwoven fabric sheets similar to the non-skin-side sheets 32 and 42 of the diaper 1. That is, in the diaper 3, the non-skin-side sheets 32 and 42 are nonwoven fabric sheets having higher hydrophilicity than the skin-side sheet 21. Then, similar to the diaper 1, the waist elastic members 35 and 45 are formed of elastic strings or the like, and are attached between the skin-side sheet 21 and the non-skin-side sheets 32 and 42 in a state of being stretched in the lateral direction.

Also in the diaper 3 of the third example, the same effect as that of the diaper 1 and the diaper 2 can be obtained. For example, in the diaper 3, in a region surrounded by a dashed line in FIG. 10B, the absorbent core 11, the breathable film (liquid-impermeable sheet 13a), and the hydrophilic nonwoven fabric (non-skin-side sheets 32 and 42) overlap each other when viewed in the thickness direction. In the portion in which the absorbent core 11, the breathable film, and the hydrophilic nonwoven fabric overlap each other, as illustrated in FIGS. 5A to 5C, the movement of moisture from the skin side to the non-skin side in the thickness direction is easily promoted, making moisture more likely to be discharged from the inside (the absorbent core 11) to the outside of the diaper 3. In addition, the diaper 3 basically has substantially the same features as those of the diaper 2 described in the second example.

### Fourth Example

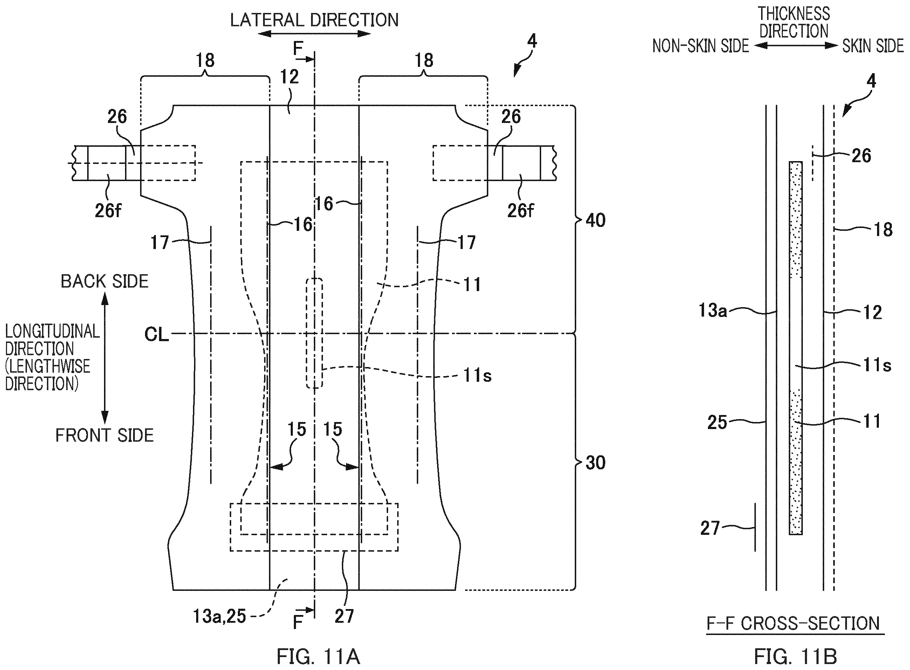

In a fourth example, a "tape-type diaper" different from the "underpants-shaped diaper" described in the first to third examples (hereinafter, also referred to as a" diaper 4") will be described as an example of the absorbent article. FIG. 11A is a plan view of the diaper 4 in the unfolded and stretched state. FIG. 11B is a schematic cross-sectional view taken along a line F-F in FIG. 11A. It should be noted that the directions (e.g., the longitudinal direction, the transverse direction, or the like) in FIGS. 11A and 11B is the same as the directions defined in the first example.

As shown in FIGS. 11A and 11B, the diaper 4 includes: the absorbent core 11 that absorbs excrement; the liquid-permeable top sheet 12 that is located on the skin side with respect to the absorbent core 11; the liquid-impermeable sheet 13a that is located on the non-skin side with respect to the absorbent core 11; an exterior sheet 25 that is located on the non-skin side with respect to the liquid-impermeable sheet 13a; and a pair of side sheets 18 that are joined to the skin side of the top sheet 12 in two lateral side portions of the top sheet 12. Further, the leg elastic members 17 (e.g., elastic strings) that stretch and contract in the longitudinal direction are arranged on two lateral side portions of the diaper 4.

The absorbent core 11, the top sheet 12, and the liquid-impermeable sheet 13a of the diaper 4 are portions corresponding to the absorbent main body 10 in the diaper 1, and respectively have the same functions as the absorbent core 11, the top sheet 12, and the liquid-impermeable sheet 13a that constitute the absorbent main body 10 of the diaper 1. For example, the liquid-impermeable sheet 13a is a breathable film that allows moisture such as urine absorbed by the absorbent core 11 to permeate as water vapor.

The exterior sheet 25 of the diaper 4 is an exterior member corresponding to the waist member 20 in the diaper 1, and is formed of hydrophilic nonwoven fabric, similar to the non-skin-side sheets 33 and 43 that constitute the waist member 20 of the diaper 1. It should be noted that the exterior member of the diaper 4 may have the same configuration as the waist member 20 in the diaper 1. For example, a configuration is also acceptable in which hydrophobic nonwoven fabric (not shown in FIG. 11B) is provided on the skin side of the exterior sheet 25 formed of hydrophilic nonwoven fabric, and the exterior member of the diaper 4 is formed of two sheet members.

Hereinafter, for convenience of description, a portion located on the front side with respect to the longitudinal central position CL of the diaper 4 is defined as the front waist portion 30, and a portion located on the back side with respect to the central position CL is defined as the back waist portion 40 (see FIG. 11A).

The pair of side sheets 18 are each a liquid-permeable nonwoven fabric sheet similar to the top sheet 12. The lateral inner end portions of the side sheets 18 are provided with the leak-proof-wall elastic members 16 such as elastic strings that can stretch and contract in the longitudinal direction. While the diaper 4 is put on, the lateral inner end portions of the side sheets 18 rise toward the wearer's skin side due to the contractive force developed by the leak-proof-wall elastic members 16, forming the leak-proof wall portions 15 in the same manner as the diaper 1.

On the back side of the diaper 4 in the longitudinal direction (back waist portion 40), a pair of fastening tapes 26 that extend outward on two lateral sides are provided. Further, on the non-skin-side surface of the diaper 4 on the front side in the longitudinal direction (front waist portion 30), a target sheet 27 for engaging the fastening tapes 26 when putting on the diaper 4 is provided. On the skin-side surfaces located on the free end sides (outside in the lateral direction) of the fastening tapes 26, hook-and-loop fasteners 26f including a plurality of hook members (not shown) are provided. The target sheet 27 is a member that can be engaged with the hook-and-loop fasteners 26f (hook members) of the fastening tapes 26, and is formed of, for example, nonwoven fabric.

When putting on the diaper 4, for example, the diaper 4 in the unfolded state shown in FIG. 11A is arranged in the wearer's crotch portion, the front waist portion 30 is applied to the wearer's stomach side portion, and the back waist portion 40 is applied to the wearer's back side portion (buttocks). Then, the pair of fastening tapes 26 and 26 are wrapped from the back side to the front side along the wearer's waist, and the hook-and-loop fasteners 26f are engaged with the target sheet 27 on the wearer's stomach side. This makes it possible to form the waist opening BH and the pair of leg openings LH and LH substantially similar to those of the diaper 1, and to fix the position of the diaper 4 to the wearer's body (crotch portion).

It should be noted that the target sheet 27 need not be necessarily provided in the diaper 4. For example, the diaper 4 may be put on by directly engaging the hook-and-loop fasteners 26f with the nonwoven fabric that constitutes the exterior sheet 25, instead of arranging the target sheet 27 on the non-skin-side surface of the exterior sheet 25.

Also in the diaper 4 of the fourth example, the same effect as that of the diaper 1 can be obtained. For example, in the diaper 4, the entire region of the absorbent core 11 overlaps the breathable film (liquid-impermeable sheet 13a) and the hydrophilic nonwoven fabric (exterior sheet 25) when viewed in the thickness direction. In the portion in which the absorbent core 11, the breathable film, and the hydrophilic nonwoven fabric overlap each other, moisture easily moves from the skin side to the non-skin side in the thickness direction, making moisture more likely to be discharged from the inside (the absorbent core 11) to the outside of the diaper 4. In addition, the diaper 4 basically has substantially the same features as those of the diaper 1 described in the first example.

### Fifth Example

The following describes an absorbent article according to a fifth example by way of example of a disposable diaper (hereinafter also referred to as a "diaper 1001").

### Configuration of Diaper 1001

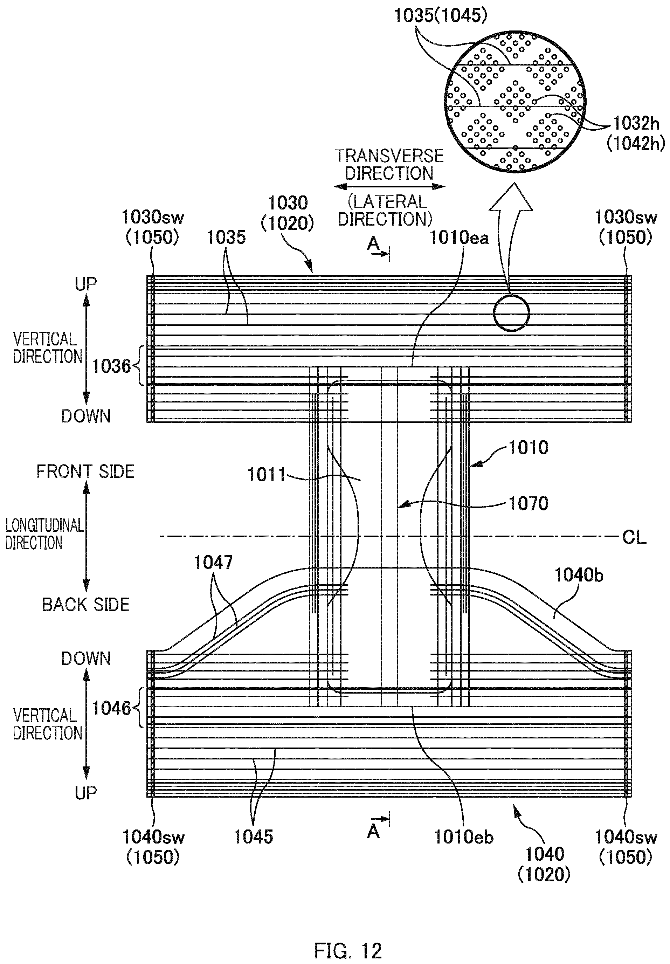

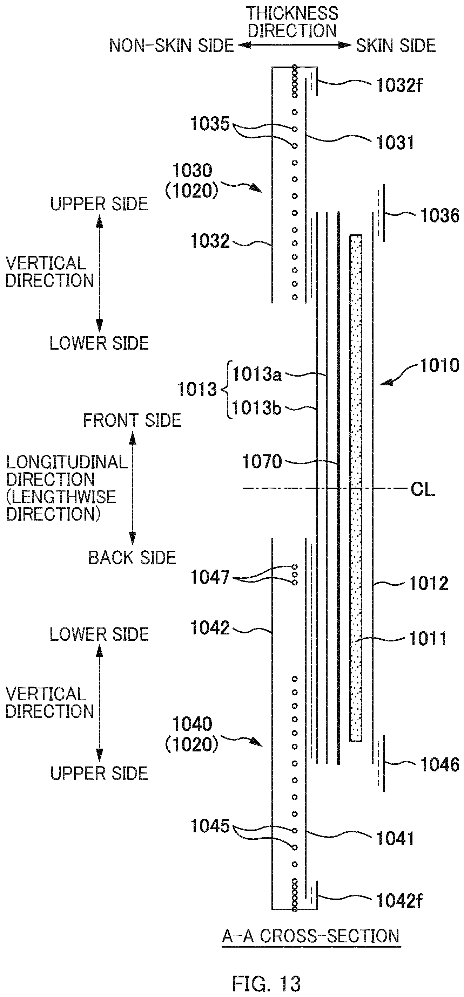

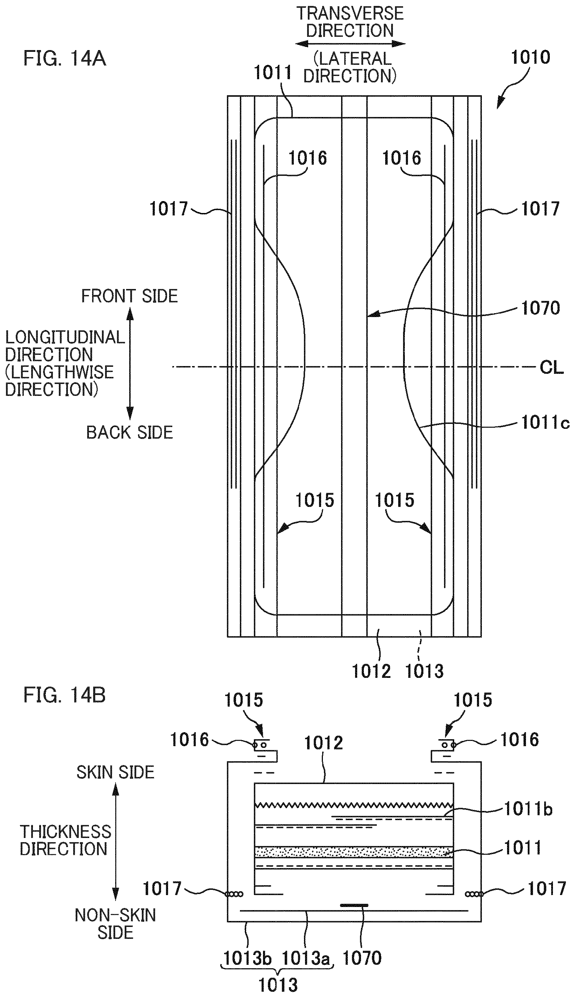

FIG. 12 is a plan view of the diaper 1001 in an unfolded and stretched state. FIG. 13 is a schematic cross-sectional view taken along a line A-A in FIG. 12. FIG. 14A is a plan view of an absorbent main body 1010, and FIG. 14B is a schematic cross-sectional view of the absorbent main body 1010. The diaper 1001 is basically an underpants-shaped disposable diaper having substantially the same configuration as the diaper 1 of the first example except that an "indicator 1070" described below is provided. Therefore, detailed description of constituent elements excluding the indicator 1070 of the diaper 1001 is omitted.

It should be noted that reference signs representing portions constituting the diaper 1001 are represented by numerals obtained by adding 1000 to reference signs representing corresponding portions constituting the diaper 1. For example, in the diaper 1001, the absorbent main body 10 of the diaper 1 is represented by an absorbent main body 1010 (see FIG. 12 and the like). The same applies to the following examples. In addition, in the fifth example, a liquid-impermeable sheet 1013a will also be referred to as a "liquid-impermeable film". That is, the liquid-impermeable film is a sheet member having "leak-proof property" that does not allow liquids to permeate, but having "moisture permeability" or "breathability" that allows water vapor and air to permeate.

### Indicator 1070

An indicator 1070 for detecting the excrement of urine or the like is provided between the absorbent core 1011 and the back sheet 1013 of the diaper 1001 in the thickness direction. The indicator 1070 is an excrement detecting portion that exhibits a predetermined reaction (e.g., a color reaction) by coming into contact with moisture contained in excrement such as urine, and is configured as an indicator including a pH indicator employed in a common diaper, for example. In the present example, as shown in FIGS. 13 and 14B, the indicator 1070 is arranged between the absorbent core 1011 and the liquid-impermeable sheet 1013a (liquid-impermeable film) in the thickness direction. Therefore, moisture such as urine absorbed by the absorbent core 1011 is likely to come into direct contact with the indicator 1070, and the reaction (color reaction) of the indicator 1070 can be easily visually recognized from the outside (non-skin side) of the diaper 1001 through the back sheet 13 .

In the diaper 1001, as shown in FIG. 14A, the indicator 1070 is configured as a band-shaped member extending along the longitudinal direction (the lengthwise direction of the absorbent main body 1010), and the indicator 1070 is arranged in the transverse (lateral) central portion of the absorbent main body 1010 so as to continue from an end portion located on the one side (front side) to an end portion located on the other side (back side) in the longitudinal direction. Accordingly, the presence or absence of excretion can be detected across a wide range in the lengthwise direction of the absorbent main body 1010. However, the configuration of the indicator 1070 is not limited thereto. For example, a plurality of indicators may be arranged spaced apart from each other in the transverse direction, or indicators having different longitudinal lengths and shapes may be arranged.

### Moisture Absorption and Evaporation by Diaper 1001

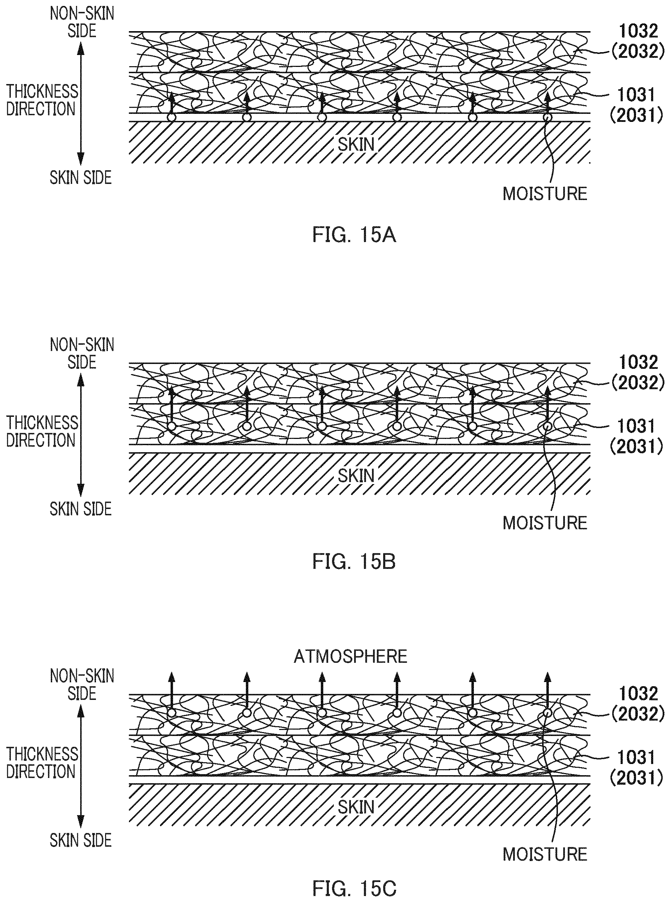

In the diaper 1001, the hydrophilic nonwoven fabric sheet having a high hydrophilicity is provided on the non-skin-side surface of the exterior member (the front waist portion 1030 and the back waist portion 1040), and therefore the diaper 1001 can absorb moisture such as sweat from the wearer's skin and make the absorbed moisture to evaporate to the atmosphere. FIGS. 15A to 15C are explanatory diagrams illustrating the basic principle when moisture is absorbed and evaporated in the diaper 1001. In FIG. 15, the cross-section of the front waist portion 1030 among the members that constitute the diaper 1001 is schematically shown.

First, when the wearer puts on the diaper 1001, the skin-side sheet 1031 (hydrophobic nonwoven fabric) arranged on the skin side of the front waist portion 1030 in the thickness direction comes into contact with the wearer's skin, as shown in FIG. 15A. As described above, the skin-side sheet 1031 of the present example is formed of SMS nonwoven fabric and includes meltblown fibers having a small fiber diameter compared with spunbond fibers. Therefore, the thin meltblown fibers have a portion in which the inter-fiber distance becomes narrow by being densely entangled. According to the above-described configuration, a capillary phenomenon occurs in the skin-side sheet 1031, and moisture such as sweat attached to the wearer's skin is easily absorbed.

Next, as shown in FIG. 15B, the moisture absorbed by the skin-side sheet 1031 is transferred to the non-skin-side sheet 1032 (hydrophilic nonwoven fabric) that is overlaid to be adjacent to the non-skin side of the skin-side sheet 1031. This is because, while the skin-side sheet 1031 is formed of a hydrophobic nonwoven fabric sheet, the non-skin-side sheet 1032 is formed of a hydrophilic nonwoven fabric sheet, a difference in the magnitude of hydrophilicity (hydrophilic gradient) is thus generated between the two sheets, and this makes moisture more likely to move from the low hydrophilic skin-side sheet 1031 side to the highly hydrophilic non-skin-side sheet 1032 side. Therefore, moisture is less likely to be held on the skin-side sheet 1031 side that is in contact with the wearer's skin, and is more likely to be held on the non-skin-side sheet 1032 side that is not in contact with the wearer's skin.

The moisture held in the non-skin-side sheet 1032 is evaporated into the atmosphere from the non-skin-side surface of the non-skin-side sheet 1032. In the diaper 1001, the non-skin-side sheet 1032 is arranged on the outermost (non-skin-side) surface of the absorbent main body 1010. That is, the non-skin-side surface of the non-skin-side sheet 1032 serves as an interface with the atmosphere. Therefore, the moisture contained in the non-skin-side sheet 1032 can be efficiently evaporated to the outside of the diaper 1001 from a wide range of the non-skin-side surface. However, it is not necessary that the hydrophilic nonwoven fabric is arranged on the entire non-skin-side surface of the exterior member. For example, another member different from the non-skin-side sheet 1032 (e.g., a tape member) may be provided on a part of the farthest-non-skin-side surface of the exterior member.

In this manner, in the exterior member (waist member 1020) of the diaper 1001, the moisture absorbed from the wearer's skin by the hydrophobic nonwoven fabric is transferred to the hydrophilic nonwoven fabric located on the non-skin side, and the moisture is released into the atmosphere from the non-skin-side surface of the hydrophilic nonwoven fabric. That is, moisture can be absorbed and efficiently evaporated to the atmosphere. Accordingly, moisture is less likely to come into contact with the wearer's skin, and this makes it possible to suppress the occurrence of skin problems such as rashes caused by the wet skin of the wearer or for the wearer to be less likely to feel discomfort.

Conversely, the diaper 1001 has a configuration in which the hydrophilic nonwoven fabric that constitutes the exterior member is more likely to absorb a large amount of moisture, compared with a conventional diaper. Then, in the case where moisture contained in the hydrophilic nonwoven fabric comes into contact with the indicator 1070, there is a risk that the indicator 1070 reacts with the moisture. That is, there is a risk that the indicator 1070 reacts with moisture excluding excrement such as urine (erroneously detects), worsening the detection accuracy of the excrement.

Therefore, in the diaper 1001 of the present example, in order to suppress the erroneous detection by the indicator 1070 as described above, members is configured in such a manner that the moisture contained in the hydrophilic nonwoven fabric is less likely to come into contact with the indicator 1070. Hereinafter, a method for suppressing erroneous detection by the indicator 1070 in the diaper 1001 will be described.

### Suppression of Erroneous Detection of Indicator 1070

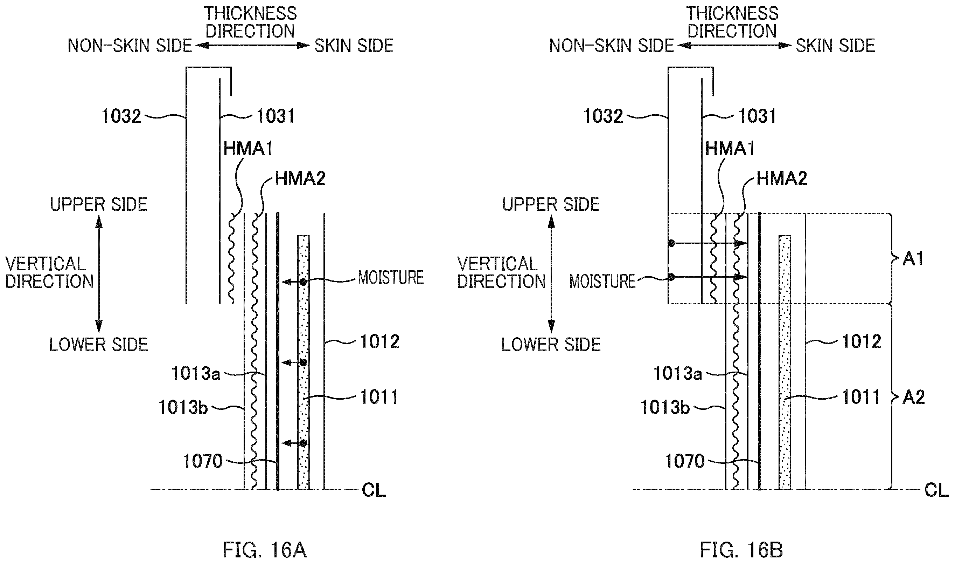

FIGS. 16A and 16B are explanatory diagrams illustrating the relationship between the arrangement of the indicator 1070 and the movement of moisture in the diaper 1001. FIG. 16A and FIG.16B schematically show the cross sections of the overlaid portion of the absorbent main body 1010 and the front waist portion 1030 (exterior member). Here, the movement of moisture in a portion in which the absorbent main body 1010 and the front waist portion 1030 (exterior member) are overlaid on each other will be described, but the same can apply to the back waist portion 1040.

FIG. 16A shows the movement of moisture (hereinafter, also referred to as "excreted fluid") contained in excrement absorbed by the absorbent core 1011. When excrement such as urine is excreted while the diaper 1001 is put on, the excreted fluid is first absorbed by the absorbent core 1011. At least a part of the absorbed excreted fluid moves from the skin side to the non-skin side in the thickness direction while diffusing in the absorbent core 1011, and reaches the indicator 1070 located adjacent to the non-skin side of the absorbent core 1011. Then, in a portion where the indicator 1070 and moisture are in contact with each other, the indicator 1070 causes a color reaction (discoloration). Accordingly, it is possible to detect the excretion of urine or the like.

FIG. 16B shows the movement of moisture such as sweat contained in the non-skin-side sheet 1032 (hydrophilic nonwoven fabric) of the front waist portion 1030 (exterior member). As described above, since the exterior member of the diaper 1001 has a hydrophilic nonwoven fabric, moisture such as sweat is absorbed by the hydrophilic nonwoven fabric, and moisture is more likely to be contained in the exterior member compared with a conventional absorbent article. In the case where moisture contained in the exterior member moves from the non-skin side to the skin side in the thickness direction and comes into contact with the indicator 1070, there is a risk that the indicator causes a color reaction due to the moisture. That is, there is a risk that the indicator 1070 detects moisture excluding the excreted fluid absorbed by the absorbent core 1011, worsening the detection accuracy of excrement.

In contrast, in the diaper 1001 of the present example, as shown in FIG. 16B, the liquid-impermeable sheet 1013a (liquid-impermeable film) having leak-proof properties is arranged between the non-skin-side sheet 1032 (hydrophilic nonwoven fabric) and the indicator 1070. Therefore, in the portion where the liquid-impermeable film is arranged, the movement of moisture in the thickness direction is blocked. That is, even in the case where moisture such as sweat contained in the hydrophilic nonwoven fabric moves from the non-skin side to the skin side, the moisture is less likely to reach the indicator 1070 arranged on the skin side with respect to the liquid-impermeable sheet 1013a (liquid-impermeable film), and a color reaction of the indicator 1070 caused by the moisture such as sweat is less likely to occur.

As described above, in the diaper 1001, the non-skin-side sheet 1032 (hydrophilic nonwoven fabric) is arranged on the non-skin side with respect to the liquid-impermeable sheet 1013a (liquid-impermeable film), and the indicator 1070 is arranged on the skin side with respect to the liquid-impermeable sheet 1013a (liquid-impermeable film). In other words, the hydrophilic nonwoven fabric and the indicator 1070 are arranged on opposite sides in the thickness direction with the liquid-impermeable film having leak-proof properties interposed therebetween. Accordingly, moisture such as sweat contained in the hydrophilic nonwoven fabric is less likely to come into contact with the indicator 1070, and the indicator 1070 can be prevented from erroneously detecting a liquid excluding excreted fluid.

Further, in the diaper 1001, in order to enlarge a range where excrement can be detected, the indicator 1070 is arranged from the one-side end to the other-side end of the absorbent main body 1010 in the lengthwise direction (longitudinal direction). As a result, there is formed a portion where the hydrophilic nonwoven fabric of the waist member 1020 (the front waist portion 1030 and the back waist portion 1040) and the indicator 1070 overlap each other when viewed in the thickness direction (see FIGS. 13 and 16B). At this time, assuming that the hydrophilic nonwoven fabric and the indicator 1070 are arranged on the same side in the thickness direction with respect to the liquid-impermeable sheet 1013a (liquid-impermeable film), there is a high possibility that moisture contained in the hydrophilic nonwoven fabric is likely to come into contact with the indicator 1070 and to cause erroneous detection. In contrast, in the diaper 1001, since the hydrophilic nonwoven fabric and the indicator 1070 are arranged on the opposite sides in the thickness direction with respect to the liquid-impermeable sheet 1013a (liquid-impermeable film), erroneous detection caused by moisture such as sweat can be more likely to be suppressed while securing a wide excrement detection range by the indicator 1070.

In particular, in the exterior member (waist member 1020) of the diaper 1001, the back waist portion 1040 is positioned in a region from the wearer's back to the buttocks, in which the wearer is likely to sweat during usage of the diaper 1001. Therefore, a non-skin-side sheet 1042 (hydrophilic nonwoven fabric) of the back waist portion 1040 is likely to absorb sweat and to contain moisture, on the wearer's back side. The diaper has a portion in which the non-skin-side sheet 1042 (hydrophilic nonwoven fabric) and the indicator 1070 overlap each other when viewed in the thickness direction. Therefore, in the case where the hydrophilic nonwoven fabric and the indicator 1070 are arranged on the same side in the thickness direction with respect to the liquid-impermeable sheet 1013a (liquid-impermeable film), moisture (sweat) contained in the hydrophilic nonwoven fabric is more likely to causes erroneous detection by the indicator 1070. However, in the diaper 1001, since the indicator 1070 and the non-skin-side sheet 1042 (hydrophilic nonwoven fabric) are arranged on the opposite sides with the liquid-impermeable sheet 1013a (liquid-impermeable film) interposed therebetween as described above, the contact of moisture such as sweat on the wearer's back side with the indicator 1070 is suppressed. Therefore, in the back-side in which the wearer is likely to sweat, the erroneous detection of the indicator 1070 can also be effectively suppressed.

Further, in a so-called three-piece type absorbent article such as the diaper 1001, the absorbent main body 1010 is arranged so that only a partial region thereof overlaps the exterior member (the front waist portion 1030 and the back waist portion 1040) when viewed in the thickness direction. That is, there are a portion in which the absorbent main body 1010, the indicator 1070, and the hydrophilic nonwoven fabric of the exterior member (the front waist portion 1030 and the back waist portion 1040) overlap each other when viewed in the thickness direction, and a portion in which the absorbent main body 1010, the indicator 1070, and the hydrophilic nonwoven fabric of the exterior member do not overlap each other when viewed in the thickness direction. In the diaper having such a configuration, comparing the number of overlaid sheet members that are located on the non-skin side with respect to the indicator 1070 between the regions where the indicator 1070 and the non-skin-side sheet 1032 (hydrophilic nonwoven fabric) overlap and do not overlap, it is desirable that the number of the overlaid sheet members in the overlapping region is larger than the number of overlaid sheet members in the non-overlapping region.

For example, in FIG. 16A, the region where the indicator 1070 and the non-skin-side sheet 1032 (hydrophilic nonwoven fabric) overlap each other is defined as region A1, and the region where the indicator 1070 and the non-skin-side sheet 1032 (hydrophilic nonwoven fabric) do not overlap each other is defined as region A2. In this case, in the region A1, four sheet members are overlaid on the non-skin side with respect to the indicator 1070, and in the region A2, two sheet members are overlaid on the non-skin side with respect to the indicator 1070. That is, the number of overlaid sheet members in the region A1 is larger than the number of overlaid sheet members in the region A2. As described above, the larger the number of overlaid sheet members in the region A1, the less likely the moisture such as sweat contained in the non-skin-side hydrophilic nonwoven fabric (non-skin-side sheet 1032) to move toward the skin side. This can make it more likely to suppress erroneous detection by the indicator 1070. On the other hand, the smaller the number of overlaid sheet members in the region A2, the more flexible the diaper 1001 is and the better fit can be obtained during usage of the diaper.

Further, in the exterior member (the front waist portion 1030 and the back waist portion 1040) of the diaper 1001, the skin-side sheet 1031 (hydrophobic nonwoven fabric) is provided on the skin side of the non-skin-side sheet 1032 (hydrophilic nonwoven fabric), as illustrated in FIG. 15. That is, the hydrophobic nonwoven fabric is interposed between the liquid-impermeable sheet 1013a (liquid-impermeable film) and the non-skin-side sheet 1032 (hydrophilic nonwoven fabric). With such a configuration, moisture such as sweat contained in the non-skin-side sheet 1032 (hydrophilic nonwoven fabric) is less likely to move toward the skin-side sheet 1031 having low hydrophilicity (hydrophobic nonwoven fabric). That is, it becomes more likely to suppress the movement of moisture contained in the hydrophilic nonwoven fabric (non-skin side) to the liquid-impermeable film side (skin side). Therefore, this can make more likely to suppress erroneous detection by the indicator 1070.

At this time, it is desirable that the hydrophobic fibers contain long fibers. The long fiber refers to a fiber having an average length (average fiber length) of 100 mm or more, or a fiber that continues from the one end to the other end in the transverse direction or in the longitudinal direction of the exterior member (the front waist portion 1030 or the back waist portion 1040). The use of the hydrophobic nonwoven fabric containing long fibers having a long fiber length makes it easier for moisture to diffuse in the planar direction of the hydrophobic fibers, and this can make it more likely to suppress the movement of moisture in the thickness direction of the hydrophobic fibers. That is, this can make it more likely to prevent moisture from permeating the hydrophobic fibers in the thickness direction and moving to the indicator 1070 side (skin side). Accordingly, erroneous detection by the indicator 1070 can be suppressed.

It should be noted that the average fiber length of the fibers means the length-weighted average fiber length L(l) as measured using the contour fiber length (Cont). The length-weighted average fiber length is measured as the L(l) value by "Kajaani FiberLab Fiber Properties (off-line)" manufactured by Metso Automation USA Inc. It should be noted that this is also a method recommended in JIS P 8226-2 (in accordance with Pulps-Determination of fibre length by automated optical analysis: Unpolarized light method).

Further, it is desirable that, in the region where the hydrophilic nonwoven fabric (non-skin-side sheets 1032 and 1042) of the exterior member (waist portions 1030 and 1040) and the indicator 1070 overlap each other when viewed in the thickness direction, a layer of an adhesive that joins the sheet members is formed. In FIGS. 16A and 16B, between the non-skin-side sheet 1032 (hydrophilic nonwoven fabric) and the indicator 1070, at least, a first adhesive layer HMA1 that joins the exterior member (front waist portion 1030) and the absorbent main body 1010, and a second adhesive layer HMA2 that joins the liquid-impermeable sheet 1013a (liquid-impermeable film) and the exterior sheet 1013b (a sheet member that is adjacent to the non-skin side of the liquid-impermeable film) are located. Both the first adhesive layer HMA1 and the second adhesive layer HMA2 are layers formed by applying an adhesive such as a hot-melt adhesive between two sheet members by spiral coating or Ω-shaped coating. The adhesive layers HMA1 and HMA2 join the two sheet members adjacent to each other in the thickness direction.

In the diaper 1001, such an adhesive layer (the adhesive layers HMA1 and HMA2) is provided between the non-skin-side sheet 1032 (hydrophilic nonwoven fabric) and the indicator 1070 in the thickness direction, and this makes the adhesive layer likely to hinder the movement of moisture in the thickness direction. That is, it is likely to prevent moisture such as sweat contained in the hydrophilic nonwoven fabric from moving toward the skin side in the thickness direction and coming into contact with the indicator 1070. This can make it more likely to suppress erroneous detection by the indicator 1070.

Further, in the diaper 1001, the first adhesive layer HMA1 and the second adhesive layer HMA2 are arranged overlapping each other when viewed in the thickness direction (see FIG. 16A). In a portion where the two adhesive layers overlap each other, the amount of the adhesive per unit volume is increased, and thus the movement of moisture in the thickness direction is more likely to be suppressed in the overlapping portion. Therefore, moisture such as sweat contained in the hydrophilic nonwoven fabric can be further prevented from moving toward the skin side in the thickness direction and coming into contact with the indicator 1070. This can further suppress erroneous detection by the indicator 1070. It should be noted that there is a high possibility that other adhesive layers are formed in addition to the first adhesive layer HMA1 and the second adhesive layer HMA2 at the time of actually manufacturing the diaper 1001. This can make erroneous detection by the indicator 1070 further less likely to occur.

Further, in the exterior member (waist portions 1030 and 1040) of the diaper 1001, waist elastic members 1035 and 1045 and curved elastic members 1047 that develop stretchability in the transverse direction (lateral direction) are provided. It is desirable that these elastic members 1035, 1045, and 1047 are less likely to cause a contractive force to act in a portion where these elastic members overlap the indicator 1070. That is, it is desirable that the stress of the elastic members 1035, 1045, and 1047 in the portion that overlap the indicator 1070 is smaller than the stress of the elastic members 1035, 1045, and 1047 in the portion that does not overlap the indicator 1070. In other words, it is desirable that the stress of the elastic member in the transverse (lateral) central portion is smaller than the stress of the elastic member in two transverse (lateral) side portions of the central portion. In the diaper 1001, as shown in FIG. 12, at least some of these elastic members 1035, 1045, and 1047 are cut at the transverse central portion, and this decreases the stresses of the elastic members 1035, 1045, and 1047 in the region that overlaps the indicator 1070 (that is, the region that overlaps the indicator before cutting).

In the case where the contractive force generated by the elastic members 1035, 1045, and 1047 acts largely in the portion where the elastic members overlap the indicator 1070, a force that presses the waist portions 1030 and 1040 toward the indicator 1070 side (that is, toward the wearer's skin side) is generated during usage of the diaper 1001. In this case, moisture contained in the non-skin-side sheets 1032 and 1042 (hydrophilic nonwoven fabric) provided in the waist portions 1030 and 1040 is more likely to come into contact with the indicator 1070, and thus there is a risk that the erroneous detection of the indicator 1070 occurs. In contrast, in the diaper 1001, the stress of the elastic members 1035, 1045, and 1047 in the portion that overlaps the indicator 1070 is reduced, and the contractive force is less likely to act on the portion. Thereby the non-skin-side sheets 1032 and 1042 (hydrophilic nonwoven fabrics) are prevented from being pressed against the indicator 1070. Accordingly, this can make erroneous detection by the indicator 1070 less likely to occur.

The stress of the elastic member can be measured as follows using, for example, an autograph tensile tester (e.g., AG-1KN1) manufactured by Shimadzu Corporation. First, in a state where the waist member 1020 (1030 and 1040) of the diaper 1001 is stretched to an extent that no wrinkles are formed on the surface thereof, there are measured the lateral dimensions of to-be-measured regions in regions where the elastic members 1035, 1045, and 1047 are arranged (the lateral central portion and two side portions of the absorbent main body 1010). Then a cutter is used to cut the to-be-measured regions to the dimensions to obtain samples. Next, the one-side end of the sample is pinched between a fixed chuck, the other-side end is pinched between a movable chuck. After stretching the sample at a speed of 300 mm/min to a length of about 90% the initial dimension, the sample is inverted, and then a tensile load (N) is obtained when contracting the sample to a length of about 75% the initial dimension. Thus, the tensile load is converted into a stress (N/mm) per unit length (mm), and is used as a stress.

### Modified Example of Indicator 1070

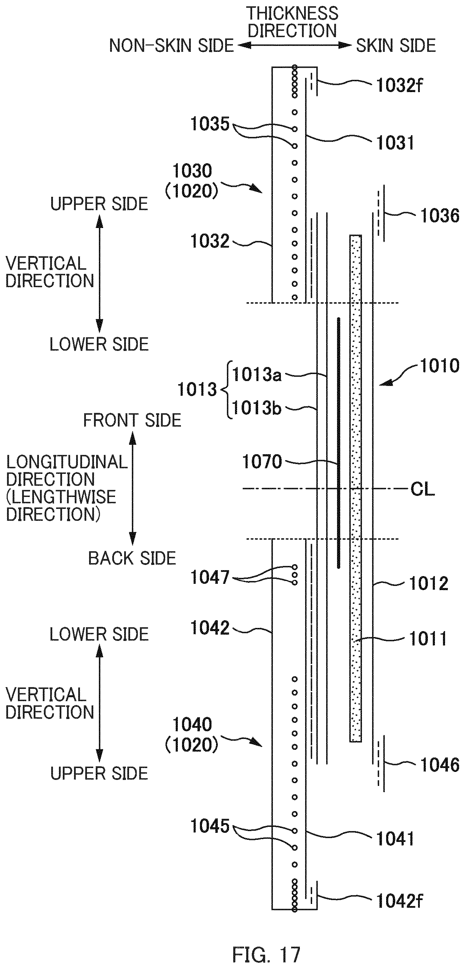

In the above-described example, a configuration has been described in which the indicator 1070 is continuously arranged from the one end side to the other end side of the absorbent main body 1010 in the lengthwise direction (longitudinal direction). However, the arrangement of the indicator 1070 is not limited thereto. FIG. 17 is a schematic cross-sectional view illustrating a modified example of the arrangement of the indicator 1070. FIG. 17 is a diagram corresponding to FIG. 13.

In the modified example of FIG. 17, the length of the indicator 1070 in the lengthwise direction (longitudinal direction) is shorter than in the case of FIG. 13. With the above-described indicator 1070, the cost of the indicator 1070 itself can be suppressed while detecting the presence or absence of excretion in the vicinity of the wearer's crotch portion (that is, in the vicinity of the excretion opening for urine or the like) while the diaper 1001 is put on. Further, in FIG. 17, the non-skin-side sheet 1032 (hydrophilic nonwoven fabric) of the front waist portion 1030 and the indicator 1070 do not overlap each other when viewed in the thickness direction. Therefore, in the front waist portion 1030, there is a lower possibility that moisture such as sweat contained in the non-skin-side sheet 1032 (hydrophilic nonwoven fabric) comes into contact with the indicator 1070. This can make it likely to suppress the erroneous detection of excretion on the front side of the diaper 1001.

### Sixth Example

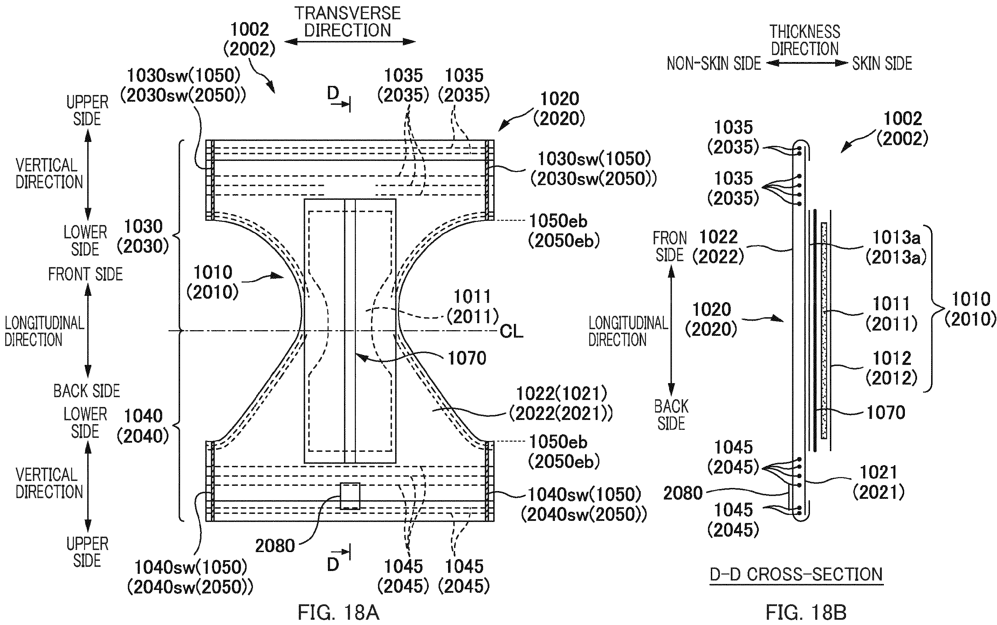

In a sixth example, an underpants-shaped diaper 1002 (hereinafter, also referred to as a "diaper 1002") having a configuration partially different from that of the fifth example will be described. FIG. 18A is a plan view of the diaper 1002 in the unfolded and stretched state. FIG. 18B is a schematic cross-sectional view taken along a line D-D in FIG. 18A. It should be noted that the directions (e.g., the longitudinal direction, the transverse direction, or the like) in FIGS. 18A and 18B is the same as the directions defined in the fifth example. Further, FIGS. 18A and 18B commonly show a diaper 2002 described below.

The diaper 1002 of the sixth example has the liquid-absorbent absorbent main body 1010, and the waist member 1020 that is joined to the non-skin side of the absorbent main body 1010 as an exterior member. Further, as shown in FIGS. 18A and 18B, the waist member 1020 of the diaper 1002 is integrally constituted so as to be continuous in the longitudinal direction (the lengthwise direction of the absorbent main body 1010). That is, the diaper 2 is a so-called two-piece type disposable diaper formed of two parts: the absorbent main body 1010 and an exterior member (waist member 1020). Hereinafter, in the exterior member (waist member 1020) of the diaper 2, a portion located on the front side with respect to the longitudinal central position CL is defined as the front waist portion 1030, and a portion located on the back side with respect to the central position CL is defined as the back waist portion 1040 (see FIG. 18A).

When shaping the diaper 1002 in the unfolded state in FIG. 18A into an underpants shape, the absorbent main body 1010 and the waist member 1020 are folded one time at a fold position, which is the longitudinal central position CL. In this folded state, the front waist portion 1030 and the back waist portion 1040 that face each other are joined and connected to each other at two lateral side portions 1030sw and 1040sw to form the pair of side joining portions 1050 and 1050. Accordingly, similar to the diaper 1 (diaper 1001) in FIG. 1, the diaper 1002 is in an underpants-shaped state having a waist opening BH and a pair of leg openings LH and LH.

In the diaper 1002, the basic configuration and function of the absorbent main body 1010 are substantially the same as those of the absorbent main body 1010 of the diaper 1001 of the fifth example, and therefore description thereof is omitted. However, the back sheet 1013 of the diaper 1002 may include only the liquid-impermeable sheet 1013a (liquid-impermeable film) and need not include the exterior sheet 1013b. This is because in the diaper 1002, the waist member 1020 is provided on the entire non-skin-side surface of the absorbent main body 1010, and the waist member 1020 functions as an exterior sheet of the absorbent main body 1010. Therefore, as the back sheet 1013 of the absorbent main body 1010 of the diaper 1002, a sheet member corresponding to the exterior sheet 1013b of the diaper 1001 need not be provided (see FIG. 18B).

Further, in the diaper 1002, an indicator 1070 similar to that described in the fifth example is provided between the absorbent core 1011 and the liquid-impermeable sheet 1013a (liquid-impermeable film) in the thickness direction. That is, in the transverse central portion of the absorbent main body 1010, a band-shaped indicator 1070 extending along the longitudinal direction (the lengthwise direction of the absorbent main body 1010) is arranged.

The waist member 1020 includes: a skin-side sheet 1021; a non-skin-side sheet 1022 that is overlaid to be adjacent to the non-skin side of the skin-side sheet 1021; and waist elastic members 1035 and 1045 that are arranged between the skin-side sheet 1021 and the non-skin-side sheet 1022 in the thickness direction (see FIG. 18B). The skin-side sheet 1021 is a hydrophobic nonwoven fabric sheet similar to the skin-side sheets 1031 and 1041 of the diaper 1001. The non-skin-side sheet 1022 is a hydrophilic nonwoven fabric sheet similar to the non-skin-side sheets 1032 and 1042 of the diaper 1001. That is, in the diaper 1002, the non-skin-side sheet 1022 is a nonwoven fabric sheet having higher hydrophilicity than the skin-side sheet 1021. Then, similar to the diaper 1001, the waist elastic members 1035 and 1045 are formed of elastic strings or the like, and are attached between the skin-side sheet 1021 and the non-skin-side sheet 1022 in a state of being stretched in the lateral direction.

Also in the diaper 1002 of the sixth example, the same effect as that of the diaper 1001 can be obtained. That is, also in the diaper 1002, the indicator 1070 is arranged on the skin side with respect to the liquid-impermeable sheet 1013a (liquid-impermeable film), and the non-skin-side sheet 1022 (hydrophilic nonwoven fabric) is arranged on the non-skin side with respect to the liquid-impermeable sheet 1013a (liquid-impermeable film). In such a configuration, even in the case where moisture such as sweat absorbed by the non-skin-side sheet 1022 (hydrophilic nonwoven fabric) moves from the non-skin side to the skin side, the moisture is less likely to reach the indicator 1070 arranged on the skin side with respect to the liquid-impermeable film, and a color reaction of the indicator 1070 caused by the moisture such as sweat is less likely to occur. That is, moisture such as sweat contained in the hydrophilic nonwoven fabric is less likely to come into contact with the indicator 1070, and it is possible to prevent the indicator 1070 from erroneously detecting a liquid excluding excreted fluid. In addition, the diaper 1002 basically has substantially the same features as those of the diaper 1001 described in the fifth example.

Further, in the exterior member (waist member 1020) of the diaper 1002, there is a portion where the non-skin-side sheet 1022 (hydrophilic nonwoven fabric) and the indicator 1070 overlap each other when viewed in the thickness direction (see FIG. 18B). In the portion in which the hydrophilic nonwoven fabric and the indicator 1070 overlap each other, there is a risk that moisture contained in the hydrophilic nonwoven fabric comes into contact with the indicator 1070. However, by arranging the hydrophilic nonwoven fabric and the indicator 1070 on the opposite sides with the liquid-impermeable film interposed therebetween as described above, it is possible to suppress erroneous detection by the indicator 1070. Therefore, in the diaper 1002, the indicator 1070 can be arranged in a wide region that overlaps the hydrophilic nonwoven fabric. That is, it is possible to make it more likely to suppress the erroneous detection caused by moisture such as sweat while securing a wide excrement detection range of the indicator 1070.

It should be noted that the diaper 1002 has a portion in which a portion of the exterior member (waist member 1020) located on the back side with respect to the longitudinal central position CL (back waist portion 1040) and the indicator 1070 overlap each other when viewed in the thickness direction. During usage of the diaper 1002, the back waist portion 1040 is positioned on the back side where the wearer is likely to sweat, the non-skin-side sheet 1022 (hydrophilic nonwoven fabric) is likely to absorb sweat and to contain moisture. In contrast, in the diaper 1002, the indicator 1070 is arranged on the opposite side of the non-skin-side sheet 1022 (hydrophilic nonwoven fabric) with the liquid-impermeable film interposed therebetween as described above, and this suppresses the contact of moisture such as sweat with the indicator 1070, on the wearer's back side. Therefore, in the back-side in which the wearer is likely to sweat, the erroneous detection of the indicator 1070 can also be effectively suppressed.

Further, in the diaper 1002, the absorbent main body 1010 is arranged so that the entirety thereof overlaps the skin-side surface of the exterior member (waist member 1020), and the entirety of the indicator 1070 overlaps the non-skin-side sheet 1022 (hydrophilic nonwoven fabric) when viewed in the thickness direction (see FIG. 18B). The liquid-impermeable sheet 1013a (liquid-impermeable film) is provided between the indicator 1070 and the non-skin-side sheet 1022 (hydrophilic nonwoven fabric). Accordingly, the entire region of the indicator 1070 can be made less likely to come into contact with moisture contained in the hydrophilic nonwoven fabric. Thus, in the diaper 1002, by arranging the indicator 1070 in a wide range, erroneous detection can be less likely to occur while increasing the excrement detection range.

### Seventh Example

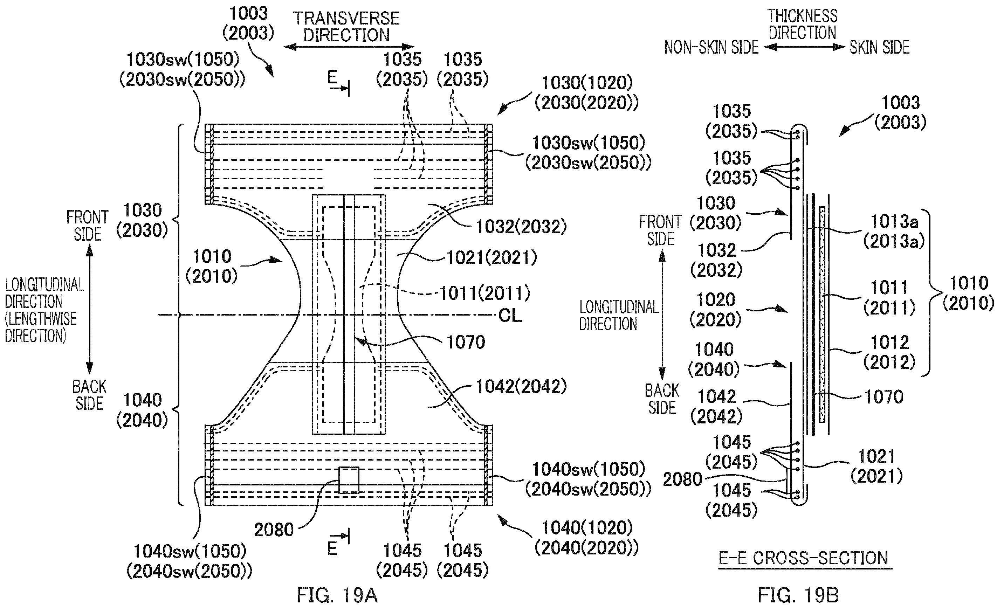

In a seventh example, an underpants-shaped diaper 1003 (hereinafter, also referred to as a "diaper 1003") having a configuration partially different from that of the above examples will be described. FIG. 19A is a plan view of the diaper 1003 in the unfolded and stretched state. FIG. 19B is a schematic cross-sectional view taken along a line E-E in FIG. 19A. It should be noted that the directions (e.g., the longitudinal direction, the transverse direction, or the like) in FIGS. 19A and 19B is the same as the directions defined in the fifth example. Further, FIGS. 19A and 19B commonly show a diaper 2003 described below.

The diaper 1003 of the seventh example has the liquid-absorbent absorbent main body 1010, and the waist member 1020 that is joined to the non-skin side of the absorbent main body 1010 as an exterior member. As shown in FIGS. 19A and 19B, the waist member 1020 of the diaper 1003 is integrally constituted so as to be continuous in the longitudinal direction (the lengthwise direction of the absorbent main body 1010). That is, the skin-side sheet 1021 is configured as an integral sheet member that is continuous from an end portion located on the one side to an end portion located on the other side in the longitudinal direction. On the other hand, the non-skin-side sheet 1032 (front non-skin-side sheet) arranged on the longitudinal front side and the non-skin-side sheet 1042 (back non-skin-side sheet) arranged on the longitudinal back side are configured as different sheet members that are non-continuous in the longitudinal direction. A diaper having such a structure will also be referred to as a simple three-piece type disposable diaper. Further, hereinafter, in the exterior member (waist member 1020) of the diaper 1003, a portion located on the front side with respect to the longitudinal central position CL is defined as the front waist portion 1030, and a portion located on the back side with respect to the central position CL is defined to as the back waist portion 1040 (see FIG. 19A).

When shaping the diaper 1003 in the unfolded state in FIG. 19A into an underpants shape, the absorbent main body 1010 and the waist member 1020 are folded one time at a fold position, which is the longitudinal central position CL. In this folded state, the front waist portion 1030 and the back waist portion 1040 that face each other are joined and connected to each other at two lateral side portions 1030sw and 1040sw to form the pair of side joining portions 1050 and 1050. Accordingly, similar to the diaper 1 (diaper 1001) in FIG. 1, the diaper 1003 is in an underpants-shaped state having a waist opening BH and a pair of leg openings LH and LH.

In the diaper 1003, the basic configuration and function of the absorbent main body 1010 are substantially the same as those of the absorbent main body 1010 of the diaper 1002 of the sixth example, and therefore description thereof is omitted.

In the exterior member (waist member 1020) of the diaper 1003, the front waist portion 1030 includes: the single skin-side sheet 1021 that is located on the skin side in the thickness direction and extends from one end side (front side) to the other end side (back side) in the longitudinal direction; the non-skin-side sheet 1032 that is located on the front side in the longitudinal direction and is overlaid to be adjacent to the non-skin side of the skin-side sheet 1021; and the waist elastic members 1035 that are arranged between the skin-side sheet 1021 and the non-skin-side sheet 1032 in the thickness direction. The back waist portion 1040 includes: the skin-side sheet 1021 that is common to the front waist portion 1030; the non-skin-side sheet 1042 that is located on the back side in the longitudinal direction and is overlaid to be adjacent to the non-skin side of the skin-side sheet 1021; and the waist elastic members 1045 that are arranged between the skin-side sheet 1021 and the non-skin-side sheet 1042 in the thickness direction.

The skin-side sheet 1021 is a hydrophobic nonwoven fabric sheet similar to the skin-side sheets 1031 and 41 of the diaper 1001. The non-skin-side sheets 1032 and 1042 are hydrophilic nonwoven fabric sheets similar to the non-skin-side sheets 1032 and 1042 of the diaper 1001. That is, in the diaper 1003, the non-skin-side sheets 1032 and 1042 are nonwoven fabric sheets having higher hydrophilicity than the skin-side sheet 1021. Then, similar to the diaper 1001, the waist elastic members 1035 and 1045 are formed of elastic strings or the like, and are attached between the skin-side sheet 1021 and the non-skin-side sheets 1032 and 1042 in a state of being stretched in the lateral direction.

Also in the diaper 1003 of the seventh example, the same effect as that of the diaper 1001 and the diaper 1002 can be obtained. That is, also in the diaper 1003, the indicator 1070 is arranged on the skin side with respect to the liquid-impermeable sheet 1013a (liquid-impermeable film), and the non-skin-side sheets 1032 and 1042 (hydrophilic nonwoven fabric) are arranged on the non-skin side with respect to the liquid-impermeable sheet 1013a (liquid-impermeable film). In such a configuration, even in the case where moisture such as sweat absorbed by the non-skin-side sheets 1032 and 1042 (hydrophilic nonwoven fabric) move from the non-skin side to the skin side, the moisture is less likely to reach the indicator 1070 arranged on the skin side with respect to the liquid-impermeable film, and a color reaction of the indicator 1070 caused by the moisture such as sweat is less likely to occur. Therefore, moisture such as sweat contained in the hydrophilic nonwoven fabric is less likely to come into contact with the indicator 1070, and it is possible to prevent the indicator 1070 from erroneously detecting a liquid excluding excreted fluid. In addition, the diaper 1003 basically has substantially the same features as those of the diaper 1002 described in the sixth example.

### Eighth Example

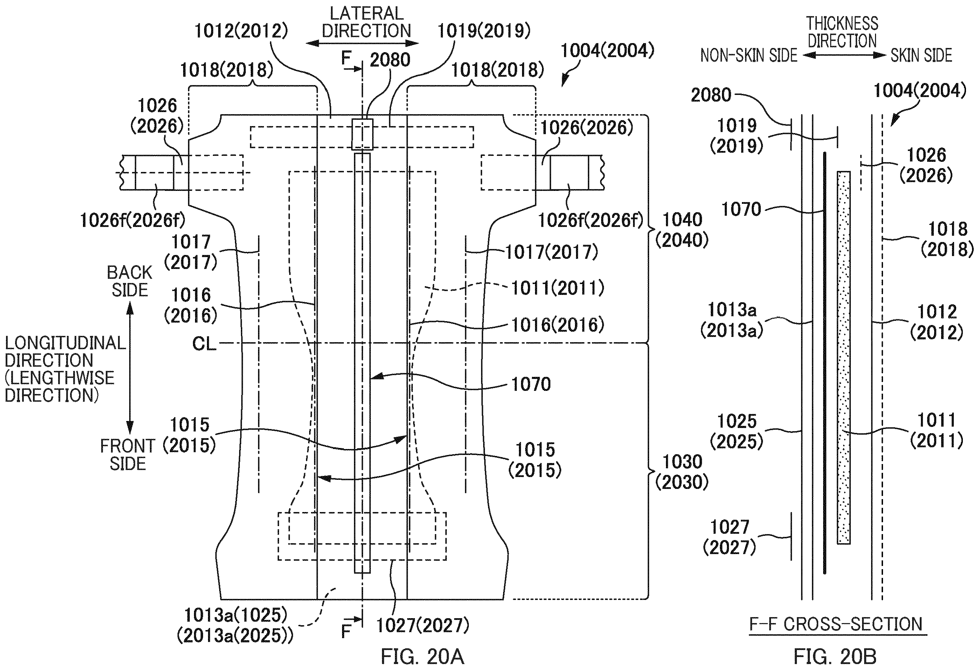

In an eighth example, a "tape-type diaper" different from the "underpants-shaped diaper" described in the fifth to seventh examples (hereinafter, also referred to as "diaper 1004") will be described as an example of the absorbent article. FIG. 20A is a plan view of the diaper 1004 in the unfolded and stretched state. FIG. 20B is a schematic cross-sectional view taken along a line F-F in FIG. 20A. It should be noted that the directions (e.g., the longitudinal direction, the transverse direction, or the like) in FIGS. 20A and 20B is the same as the directions defined in the fifth example. Further, FIGS. 20A and 20B commonly show a diaper 2004 described below.

As shown in FIGS. 20A and 20B, the diaper 1004 includes: the absorbent core 1011 that absorbs excrement; the liquid-permeable top sheet 1012 that is located on the skin side with respect to the absorbent core 1011; the liquid-impermeable sheet 1013a that is located on the non-skin side with respect to the absorbent core 1011; an exterior sheet 1025 that is located on the non-skin side with respect to the liquid-impermeable sheet 1013a; and a pair of side sheets 1018 that are joined to the skin side of the top sheet 1012 in two lateral side portions of the top sheet 1012. Further, the leg elastic members 1017 (e.g., elastic strings) that stretch and contract in the longitudinal direction are arranged on two lateral side portions of the diaper 1004.

The absorbent core 1011, the top sheet 1012, and the liquid-impermeable sheet 1013a of the diaper 1004 are portions corresponding to the absorbent main body 1010 in the diaper 1001, and respectively have the same functions as the absorbent core 1011, the top sheet 1012, and the liquid-impermeable sheet 1013a that constitute the absorbent main body 1010 of the diaper 1001. For example, the liquid-impermeable sheet 1013a is a liquid-impermeable film that allows moisture such as urine absorbed by the absorbent core 1011 to permeate as water vapor.

The exterior sheet 1025 of the diaper 1004 is an exterior member corresponding to the waist member 1020 in the diaper 1001, and is formed of a hydrophilic nonwoven fabric, similar to the non-skin-side sheets 1032 and 1042 that constitute the waist member 1020 of the diaper 1001. It should be noted that the exterior member of the diaper 1004 may have the same configuration as the waist member 1020 in the diaper 1001. For example, a configuration is also acceptable in which hydrophobic nonwoven fabric (not shown in FIG. 20B) is located to be adjacent to the skin side of the exterior sheet 1025 formed of hydrophilic nonwoven fabric, and the exterior member of the diaper 1004 is formed of two sheet members.

Hereinafter, for convenience of description, a portion located on the front side with respect to the longitudinal central position CL of the diaper 1004 is defined as the front waist portion 1030, and a portion located on the back side with respect to the central position CL is defined as the back waist portion 1040 (see FIG. 20A).

The pair of side sheets 1018 are each a liquid-permeable nonwoven fabric sheet similar to that of the top sheet 1012. The lateral inner end portions of the side sheets 1018 are provided with the leak-proof-wall elastic members 1016 such as elastic strings that can stretch and contract in the longitudinal direction. While the diaper 1004 is put on, the lateral inner end portions of the side sheets 1018 rise toward the wearer's skin side due to the contractive force developed by the leak-proof-wall elastic members 1016, forming the leak-proof wall portions 15 in the same manner as the diaper 1001.

On the back side of the diaper 1004 in the longitudinal direction (back waist portion 1040), a pair of fastening tapes 1026 that extend outward on two lateral sides are provided. On the skin-side surfaces located on the free end sides (outside in the lateral direction) of the fastening tapes 1026, hook-and-loop fasteners 1026f including a plurality of hook members (not shown) are provided. Further, on the non-skin-side surface of the diaper 1004 on the front side in the longitudinal direction (front waist portion 1030), a target sheet 1027 for engaging the fastening tapes 1026 when putting on the diaper 1004 is provided. The target sheet 1027 is a member that can be engaged with the hook-and-loop fasteners 1026f (hook members) of the fastening tapes 1026, and is formed of, for example, nonwoven fabric.

Further, on the back side of the diaper 1004 in the longitudinal direction (back waist portion 1040), back elastic members 1019 that stretch and contract along the lateral direction (transverse direction) are provided. The back elastic member 1019 is a band-shaped elastic member formed of a stretchable nonwoven fabric or the like, and is attached to the diaper 1004 in a state of being stretched in the lateral direction. The stretching/contracting force developed by the back elastic member 1019 makes the diaper 1004 more likely to fit around the wearer's waist at the time of putting the diaper 1004 on the wearer's body. It should be noted that similar to the waist elastic members 1035 and 1045 and the curved elastic member 1047 described in the diaper 1001 of the fifth example, in the diaper 1004, the back elastic member 1019 is arranged so as not to exhibit stretchability in the portion that overlaps the indicator 1070. Accordingly, the exterior sheet 1025 (hydrophilic nonwoven fabric) containing moisture is prevented from being pressed against the indicator 1070, and this can make erroneous detection of the indicator 1070 less likely to occur.

When putting on the diaper 1004, for example, the diaper 1004 in the unfolded state shown in FIG. 20A is arranged in the wearer's crotch portion, the front waist portion 1030 is applied to the wearer's stomach side portion, and the back waist portion 1040 is applied to the wearer's back side portion (buttocks). Then, the pair of fastening tapes 1026 and 26 are wrapped from the back side to the front side along the wearer's waist, and the hook-and-loop fasteners 1026f are engaged with the target sheet 1027 on the wearer's stomach side. This makes it possible to form the waist opening BH and the pair of leg openings LH and LH substantially similar to those of the diaper 1001, and to fix the position of the diaper 1004 to the wearer's body (crotch portion).

It should be noted that the target sheet 1027 need not be necessarily provided in the diaper 1004. For example, the diaper 1004 may be put on by directly engaging the hook-and-loop fasteners 1026f with the nonwoven fabric that constitutes the exterior sheet 1025, instead of arranging the target sheet 1027 on the non-skin-side surface of the exterior sheet 1025.

Also in the diaper 1004 of the eighth example, the same effect as that of the diaper 1001 can be obtained. That is, also in the diaper 1004, the indicator 1070 is arranged on the skin side with respect to the liquid-impermeable sheet 1013a (liquid-impermeable film), and the exterior sheet 1025 (hydrophilic nonwoven fabric) is arranged on the non-skin side with respect to the liquid-impermeable sheet 1013a (liquid-impermeable film). In such a configuration, even in the case where moisture such as sweat absorbed by the exterior sheet 1025 (hydrophilic nonwoven fabric) moves from the non-skin side to the skin side, the moisture is less likely to reach the indicator 1070 arranged on the skin side with respect to the liquid-impermeable film, and a color reaction of the indicator 1070 caused by the moisture such as sweat is less likely to occur. Therefore, moisture such as sweat contained in the hydrophilic nonwoven fabric is less likely to come into contact with the indicator 1070, and it is possible to prevent the indicator 1070 from erroneously detecting a liquid excluding excreted fluid. In addition, the diaper 1004 basically has substantially the same features as those of the diaper 1001 described in the fifth example.

### Ninth Example (First Embodiment)

The following describes an absorbent article according to the present invention by way of example of a disposable diaper (hereinafter also referred to as a "diaper 2001").

### Configuration of Diaper 2001

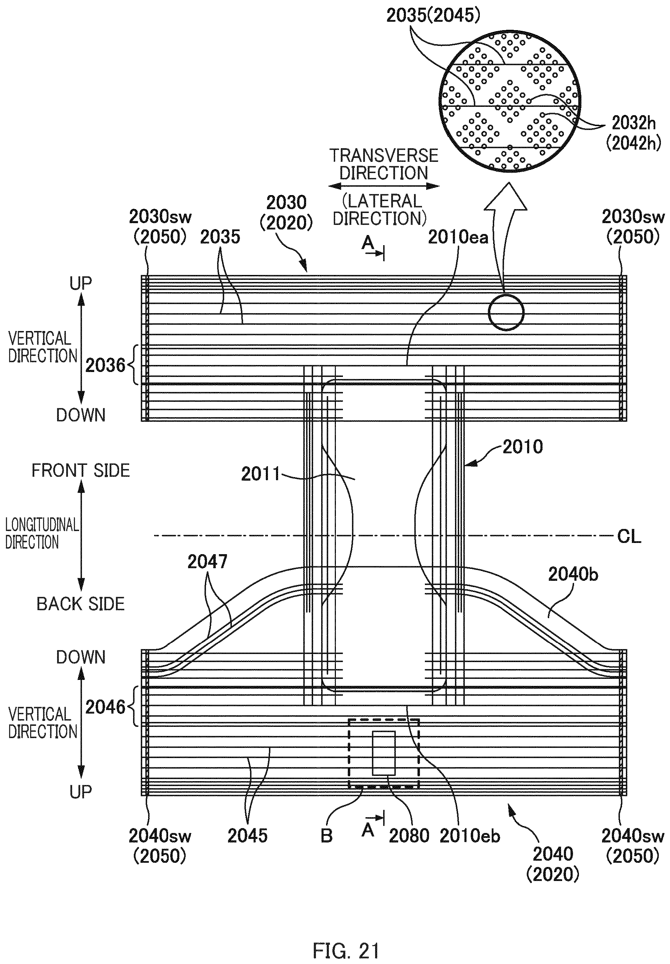

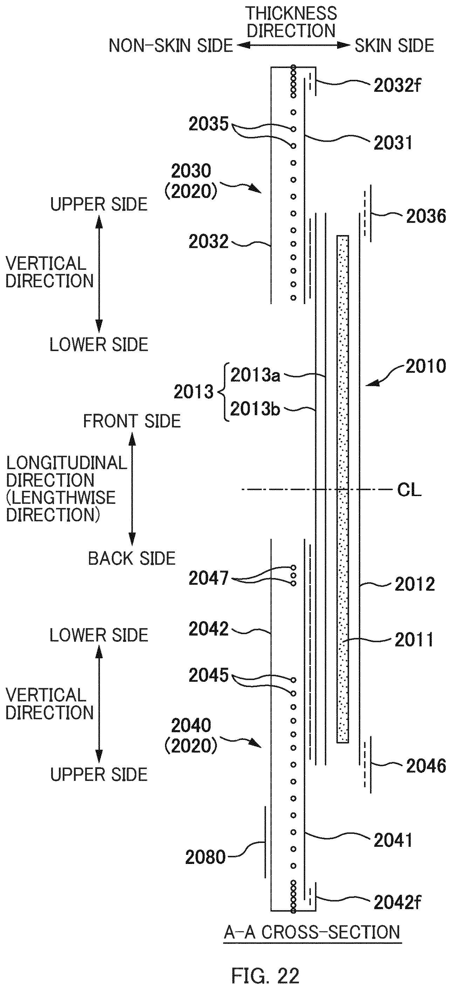

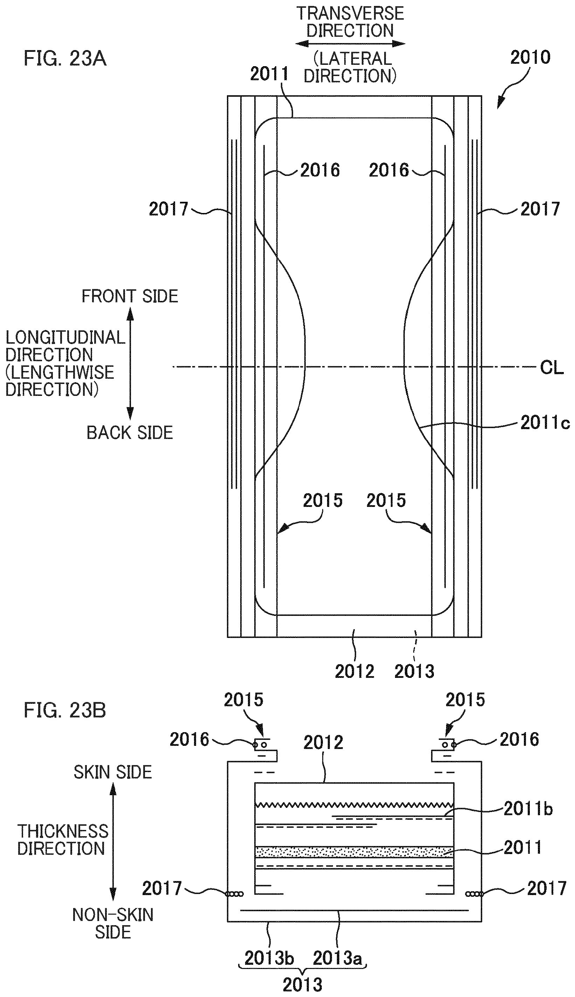

FIG. 21 is a plan view of a diaper 2001 in an unfolded and stretched state. FIG. 22 is a schematic cross-sectional view taken along a line A-A in FIG. 21. FIG. 23A is a plan view of an absorbent main body 2010, and FIG. 23B is a schematic cross-sectional view of the absorbent main body 2010. The diaper 2001 is basically an underpants-shaped disposable diaper having substantially the same configuration as the diaper 1 of the first example except that a "post handling tape 2080 (attachment)" described below is provided. Therefore, detailed description of the configuration excluding the post handling tape 2080 of the diaper 2001 is omitted. Further, in the diaper 2001, as illustrated in FIGS. 15A to 15C, the hydrophilic nonwoven fabric sheet having a high hydrophilicity is provided on the non-skin-side surface of the exterior member (a front waist portion 2030 and a back waist portion 2040), and therefore the diaper 2001 can absorb moisture such as sweat from the wearer's skin and make the absorbed moisture to evaporate to the atmosphere.

As shown in FIGS. 21 and 22, the post handling tape 2080 (attachment) described below is attached to the non-skin-side surface of the back waist portion 2040. Note that, as an attachment attached to the diaper 2001, it is possible to attach an information display seal that displays information such as the front and back of the diaper or the size of the diaper, in addition to the post handling tape 2080. Further, the member to which the attachment is attached is not limited to the back waist portion 2040 of the exterior member, and the attachment may be attached to the front waist portion 2030.

In addition, in the ninth example (first embodiment), a liquid-impermeable sheet 2013a will also be referred to as a "breathable film". That is, the breathable film is a sheet member having "leak-proof property" that does not allow liquids to permeate, but having "moisture permeability" or "breathability" that allows water vapor and air to permeate.

### Post Handling Tape 2080

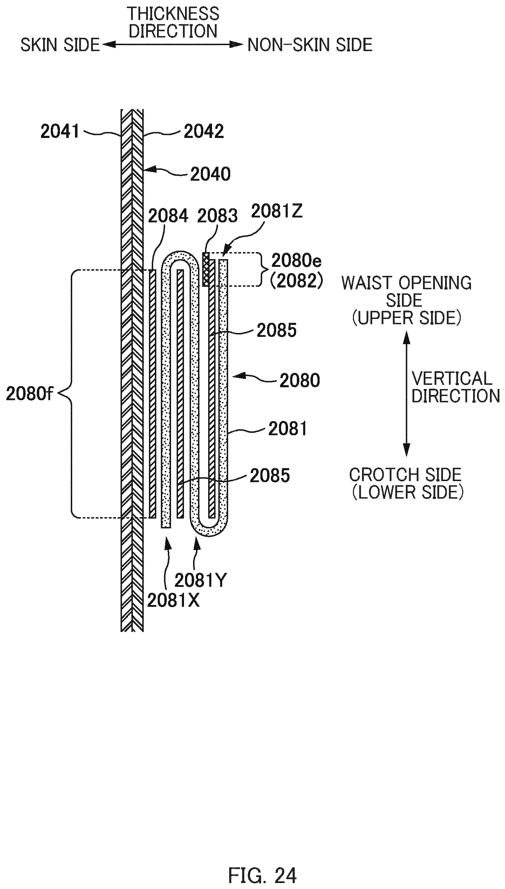

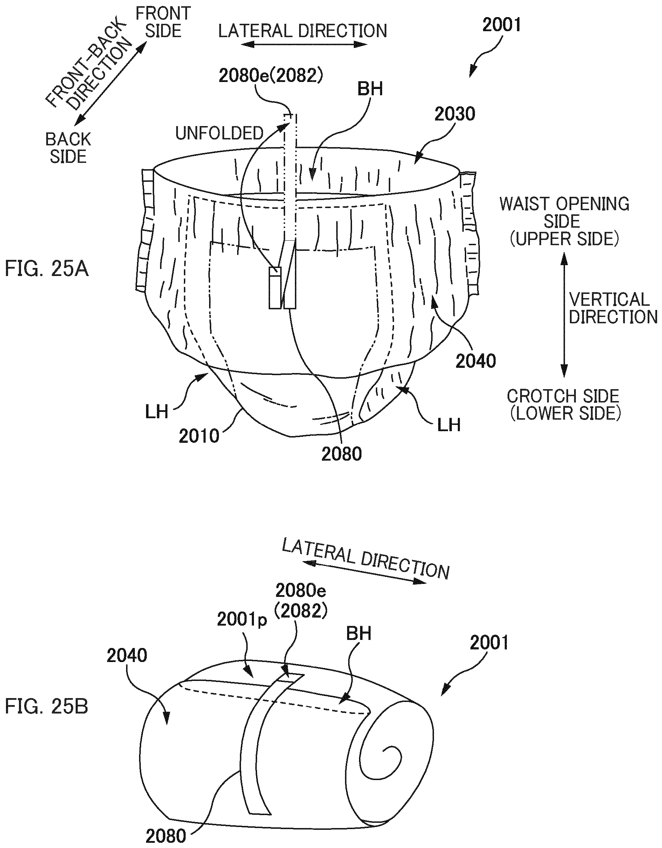

A post handling tape 2080, which is be used when discarding the diaper 2001, is provided on the non-skin-side surface of the back waist portion 2040 of the diaper 2001. FIG. 24 is a schematic cross-sectional view illustrating the post handling tape 2080 provided on the non-skin-side surface of the back waist portion 2040. FIG. 25A is a schematic perspective view of the diaper 2001 in a state where the post handling tape 2080 is unfolded, when viewed from the back side. FIG. 25B is a schematic perspective view of a state in which the diaper 2001 in the state in FIG. 25A has been rolled up for disposal. It should be noted that, for the convenience of description, FIGS. 24 and 25 are simplified views in which elastic members 2045 and the like are omitted.

As shown in FIGS. 21 and 25, the post handling tape 2080 is a long, band-shaped member extending along the vertical direction, and is attached (adhere) to the non-skin-side surface of a non-skin-side sheet 2042 of the back waist portion 2040 with an adhesive or the like. The post handling tape 2080 includes a tape main body 2081, a tape grab portion 2082, a tape grab member 2083, an attaching portion 2084, and a plurality of tape adhesive portions 2085. In a state where the lengthwise direction and the lateral direction of the rectangular post handling tape 2080 are respectively aligned along the vertical (longitudinal) direction and the lateral (transverse) direction of the diaper 2001, the tape grab portion 2082 is provided in the one end portion 2080e of the post handling tape 2080 in the lengthwise direction, and the attaching portion 2084 is located at the other end portion 2080f in the lengthwise direction.

The one end portion 2080e of the post handling tape 2080 is capable of extending toward the waist opening BH side by being unfolded as shown in FIG. 25A. In order to make the diaper 2001 into a compact form when disposing of the diaper 2001, for example, two lateral end portions of the back waist portion 2040 are folded toward the front side, and the diaper 2001 is rolled up from the crotch side toward the waist opening side, making the diaper 2001 into the state shown in FIG. 25B. At this time, the diaper 2001 can be maintained in a compact form by making the post handling tape 2080 adhere to a portion 1p that is located beyond the waist opening BH, with the tape adhesive portion 2085.

The other end portion 2080f of the post handling tape 2080 is fixed to the non-skin-side surface (non-skin-side sheet 2042) of the back waist portion 2040, with the attaching portion 2084 which is formed of an adhesive. It is desirable that, even in the case where the one end portion 2080e is pulled at the time of unfolding the post handling tape 2080 as in FIG. 25A, the post handling tape 2080 is not separated from the back waist portion 2040, that is, it is desirable that the attaching portion 2084 is not peeled off.

For example, as shown in FIG. 24, the post handling tape 2080 in an unused state is in a state of being folded in the lengthwise direction into a Z-shape. By folding the tape main body 2081 is folded into a Z-shape, the tape main body 2081 has three layers, namely a first layer 2081x that is positioned farthest on the skin side, a third layer 2081z that is positioned farthest on the non-skin side, and a second layer 2081y that is positioned between the first layer and the third layer. In a state of being folded in a Z-shape, the tape adhesive portions 2085 having a peelable adhesive strength are provided between the non-skin-side surface of the first layer 2081x and the skin-side surface of the second layer 2081y that are adjacent to each other in the thickness direction, and between the non-skin-side surface of the second layer 2081y and the skin-side surface of the third layer 2081z that are adjacent to each other in the thickness direction. That is, the tape adhesive portion 2085 makes adhesion of the first layer 2081x and the second layer 2081y and adhesion of the second layer 2081y and the third layer 2081z capable of re-adhering after being peeled off.

The unused post handling tape 2080 is temporarily fixed in a Z-shaped folded state by the tape adhesive portion 2085. It should be noted that the tape adhesive portions 2085 are not necessarily provided across the entire region between the non-skin-side surface of the first layer 2081x and the skin-side surface of the second layer 2081y, and also are not necessarily provided across the entire region between the non-skin-side surface of the second layer 2081y and the skin-side surface of the third layer 2081z. It is sufficient that each tape adhesive portion 2085 have an adhesive strength capable of temporarily fixing the unused post handling tape 2080 into the Z-shaped folded state, and any adhesive range is possible. Further, all of the tape adhesive portions 2085 need not be formed of an adhesive. For example, the temporary fixing between the second layer 2081y and the third layer 2081z may be made by compression, pressing, or the like instead of using the adhesive.

Then, when the tape grab portion 2082 is pulled in the lengthwise direction (vertical direction) as indicated by the dashed double-dotted line in FIG. 25A, the state of close contact between the surfaces by the tape adhesive portion 2085 is released, and the post handling tape 2080 is unfolded and extends toward the waist opening BH located on the upper side.

The tape main body 2081 is a member that serves as a base material of the post handling tape 2080, and can be exemplified by a resin material such as polyethylene or polypropylene. For the tape main body 2081 in the present embodiment, a transparent resin material having smooth surfaces is used for its two surfaces. The tape main body 2081 may be, for example, a pulp material such as paper or the like, and the form thereof is not limited to one having two surfaces which is smooth. For example, the tape main body 2081 may be a fiber aggregate of nonwoven fabric, woven fabric, or the like. However, in order to make releasable the close contact state between the surfaces of the post handling tapes 2080 while maintaining the state folded in the Z-shape by the adhesion of the tape adhesive portion 2085, it is more preferable that the post handling tape 2080 has two surfaces which is smooth, and made of a resin material such as polyethylene or polypropylene.

The tape grab portion 2082 is a portion in which a person who performs post handling of the diaper 2001 grabs the post handling tape 2080. The tape grab portion 2082 is provided in the end portion of the post handling tape 2080, and the tape grab member 2083 is provided in the tape grab portion 2082. Specifically, the tape grab member 2083 is non-peelably fixed to the skin-side surface of the tape main body 2081 with an adhesive (not shown), and the adhesive does not adhere to the outer circumferential surface of the tape grab portion 2082. The tape grab member 2083 is formed of a colorless and transparent resin material such as polyethylene or polypropylene. For example, in the tape grab portion 2082, a portion of the one end portion 2080e of the tape main body 2081 where the adhesive is not provided may be used as the tape grab portion 2082, or a portion of the one end portion 2080e of the tape main body 2081 which is folded back toward the skin side and is made by making portions of the tape main body 2081 adhere may be used as the tape grab portion 2082.

It should be noted that, although the post handling tape 2080 itself of the diaper 2001 is substantially non-stretched/contracted in the lengthwise direction, the present invention is not limited thereto. The tape main body 2081 may be formed of an elastic material such as rubber and have stretchability in the lengthwise direction. Further, the post handling tape 2080 need not be folded into a Z shape, and may have a two-folded V shape, or may have a four-folded W shape. Or the post handling tape 2080 may be folded greater times than to make the W shape.

### Operation of Diaper 2001 during Post Handling

Next, the following describes a problem that occurs at the time of disposal after the diaper 2001 is used. When disposing of the diaper 2001 after use, as illustrated in FIGS. 25A and 25B, a post handling operation is performed in which the post handling tape 2080 is unfolded and then an absorbent main body 2010 which has absorbed excrement is fixed with the post handling tape 2080 in a state where the diaper 2001 is rolled up with the absorbent main body 2010 inside. In the post handling operation, in order to unfold the post handling tape 2080 which is in a folded state, an operation of pulling the tape grab portion 2082 (one end portion 2080e) toward the non-skin side is performed, but at this time, there is a risk that the attaching portion 2084 is peeled off. That is, there is a risk that the post handling tape 2080 (attachment) is separated from the non-skin-side sheet 2042 of the back waist portion 2040 when pulling the tape grab portion 2082.

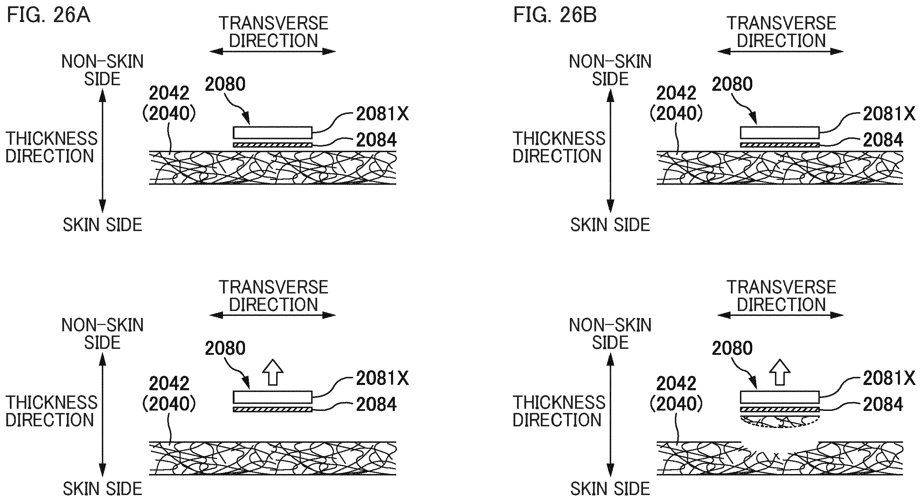

FIGS. 26A and 26B are explanatory diagrams illustrating a factor that causes the post handling tape 2080 to be separated in the post handling operation of the diaper 2001.

FIG. 26A is an explanatory diagram illustrating the influence of decrease in the adhesive force of the attaching portion 2084 as a first factor. As described above, in the post handling tape 2080, the first layer 2081x farthest on the skin side of the tape main body 2081 is attached to the non-skin-side sheet 2042 of the back waist portion 2040 through the attaching portion 2084 formed of an adhesive. That is, in a state where the first layer 2081x and the non-skin-side sheet 2042 face each other, the first layer 2081x and the non-skin-side sheet 2042 are fixed to each other by the adhesive force of the attaching portion 2084. In this state, when the tape grab portion 2082 (one end portion 2080e) of the post handling tape 2080 is pulled toward the non-skin side, a force acts in a direction in which the first layer 2081x (attaching portion 2084) of the tape main body 2081 is pulled apart from the non-skin-side sheet 2042.

In the diaper 2001, a hydrophilic nonwoven fabric subjected to a hydrophilic treatment with a predetermined oil agent is used as the non-skin-side sheet 2042, and the non-skin-side sheet 2042 contains an oil agent. Therefore, there is a case where the attaching portion 2084 is easily peeled off due to the influence of the oil agent. That is, there is a case where the adhesive force of the attaching portion 2084 is decreased compared with the case of attaching to a nonwoven fabric containing no oil agent (hydrophobic nonwoven fabric). In this case, as shown in FIG. 26A, there is a risk that the attaching portion 2084 is peeled off from the non-skin-side surface of the non-skin-side sheet 2042 and the post handling tape 2080 is separated.

Next, FIG. 26B is an explanatory diagram illustrating the influence of the bonding between the fibers of the hydrophilic nonwoven fabric as a second factor. Generally, a nonwoven fabric is formed by tangling of a plurality of fibers. The greater the number of tangled portions (also referred to as "entanglement points") with other fibers per, the stronger the bonding force between the fibers and the less likely the nonwoven fabric is torn. In the post handling operation of the diaper 2001, when the post handling tape 2080 (attachment) attached to the surface of such a nonwoven fabric is pulled toward the non-skin side, a force acts in a direction in which the attaching portion 2084 is pulled apart from the non-skin-side sheet 2042.

Further, there is a case where, when the post handling tape 2080 is pulled, the bonding between the fibers that constitute the nonwoven fabric are broken, and thereby a part of the nonwoven fabric is torn. For example, when a force acts in the direction in which the attaching portion 2084 is pulled apart from the non-skin-side sheet 2042 (hydrophilic nonwoven fabric), in the case where the bonding force between the fibers in the non-skin-side sheet 2042 is weaker than the adhesive force between the non-skin-side sheet 2042 and the attaching portion 2084, the non-skin-side sheet 2042 may be torn in some cases. That is, there is a risk that tangling (entanglement) between the fibers in the vicinity of the surface of the non-skin-side sheet 2042 is released and the vicinity of the surface of the non-skin-side sheet 2042 is torn and separated as shown in FIG. 26B. As a result, there is a risk that the post handling tape 2080 (attaching portion 2084) is separated from the non-skin-side sheet 2042.

In response to such a problem, in the diaper 2001 of the present embodiment, by adjusting the length of fibers that constitute the non-skin-side sheet 2042 (hydrophilic nonwoven fabric) to which the post handling tape 2080 is attached, it suppresses the separation of the post handling tape 2080 during the post handling operation.

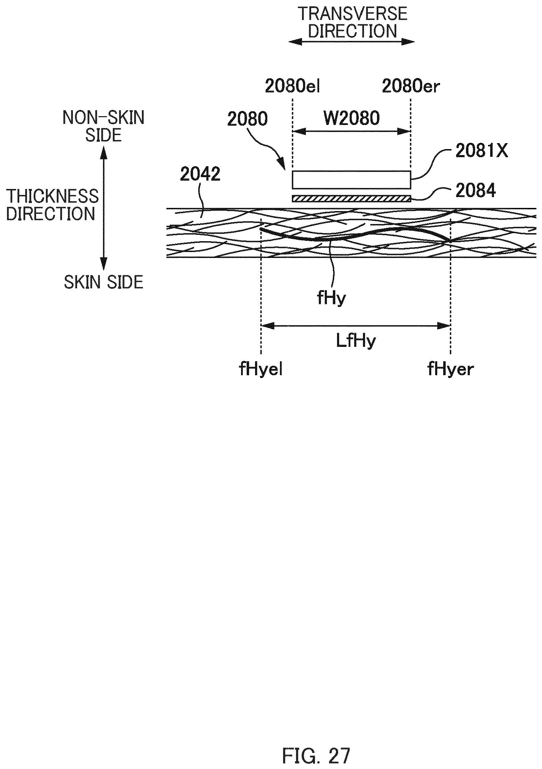

FIG. 27 is an explanatory diagram illustrating the relationship between fibers that constitute the non-skin-side sheet 2042 (hydrophilic nonwoven fabric) of the diaper 2001 and the post handling tape 2080. In the diaper 2001 of the present embodiment, the non-skin-side sheet 2042 (hydrophilic nonwoven fabric) is configured to have fibers having a fiber length longer than the width dimension of the post handling tape 2080 (attachment). Here, the width dimension of the attachment refers to the smaller dimension of the dimensions of the attachment in the longitudinal direction and in the transverse direction. In the diaper 2001, in the case where the attachment is a longitudinally-elongated post handling tape 2080, the dimension of the post handling tape 2080 in the transverse direction becomes the width dimension of the attachment.

In the case of FIG. 27, regarding a certain fiber fHy of the fibers that constitute the non-skin-side sheet 2042 (hydrophilic nonwoven fabric), which is an object to which the attachment is attached, its fiber length LfHy in the natural state is longer than the dimension W2080 of the post handling tape 2080, which is an attachment, in the transverse direction (LfHy > W2080). As the fiber length LfHy of the fiber fHy that constitutes the hydrophilic nonwoven fabric increases, the number of entanglement points with other fibers increases. This makes the fibers more likely to be tangled with each other, increasing the bonding force. Therefore, the hydrophilic nonwoven fabric is less likely to be torn. Further, since the fiber length LfHy is longer than the width W2080 of the post handling tape 2080, tangling between the fibers is likely to be formed in a range larger than the width W2080 of the post handling tape 2080. That is, the entanglement points of the fibers are formed in a region larger than the width W2080, and therefore even in the case where a force acts in the direction in which the post handling tape 2080 is pulled, it is possible to make the hydrophilic nonwoven fabric less likely to be torn in the portion to which the post handling tape 2080 is attached. Therefore, it is possible to suppress the peeling of the post handling tape 2080 due to the weak bonding force between the fibers that constitute the hydrophilic nonwoven fabric, as described in at least FIG. 26B. This enables to make the post handling tape 2080 (attachment) less likely to peel off from the non-skin-side sheet 2042 (hydrophilic nonwoven fabric).

Further, in FIG. 27, the fiber fHy is arranged so as to extend across the post handling tape 2080 (attachment) in a direction (transverse direction) orthogonal to a direction (longitudinal direction) in which the post handling tape 2080 is pulled. In other words, in the transverse direction, the left end fHyel of the fiber fHy is positioned on the left side with respect to the left end 2080el of the post handling tape 2080, and the right end fHyer of the fiber fHy is positioned on the right side with respect to the right end 2080er of the post handling tape 2080. In this case, there is high possibility that the fiber fHy has entanglement points with other fibers in the regions on the outside of the two ends 2080el and 2080er of the post handling tape 2080 in the lateral direction. Therefore, in two regions of the hydrophilic nonwoven fabric located laterally outside the portion which has the width W2080 and to which the post handling tape 2080 is attached, the entanglement between the fibers is less likely to be released and the bonding force becomes stronger. Accordingly, the hydrophilic nonwoven fabric is much less likely to be torn against the force that pulls the post handling tape 2080 (attaching portion 2084), and this enables to make it more likely to suppress the separation of the post handling tape 2080.

It should be noted that the direction in which fibers that constitute the non-skin-side sheet 2042 (hydrophilic nonwoven fabric) are arranged is not limited to the direction along the transverse direction as in FIG. 27, and there is a possibility that a part of fibers are arranged along another direction (e.g., the longitudinal direction). However, as described above, in the case where the fibers are arranged so as to extend across the attachment in a direction (transverse direction) orthogonal to a direction (longitudinal direction) in which the post handling tape 2080 (attachment) is pulled, it is possible to make the hydrophilic nonwoven fabric less likely to be torn. That is, the attachment can be made less likely to be peeled off from the hydrophilic nonwoven fabric. Therefore, it is desirable that the fiber orientation of the hydrophilic nonwoven fabric is a direction (transverse direction) orthogonal to the direction in which the post handling tape 2080 (attachment) is pulled. In other words, it is desirable that the number of fibers per unit volume arranged to extend across the attachment in the transverse direction is larger than the number of fibers per unit volume arranged to extend across the attachment in the longitudinal direction. With such a configuration, it is possible to make the attachment further less likely to peel off.

In the diaper 2001, the post handling tape 2080, which is an attachment attached to the non-skin-side sheet 2042 (hydrophilic nonwoven fabric), is provided on the premise that the post handling tape is pulled in a direction in which the post handling tape is pulled apart from the hydrophilic nonwoven fabric when performing a post handling operation after the diaper 2001 is used. Therefore, if the attaching portion 2084 is peeled off easily, the function of the post handling tape 2080 is lost, making the user difficult to comfortably perform the post handling operation. In contrast, in the present embodiment, as described above, by adjusting the fiber length and orientation of the fibers that constitute the hydrophilic nonwoven fabric and by adjusting the arrangement of the post handling tape 2080, it makes it easier to suppress the separation of the post handling tape 2080 from the non-skin-side sheet 2042 (hydrophilic nonwoven fabric) during the post handling operation of the diaper 2001. Therefore, it is possible to reduce the stress and discomfort of the user associated with the post handling operation of the diaper 2001.

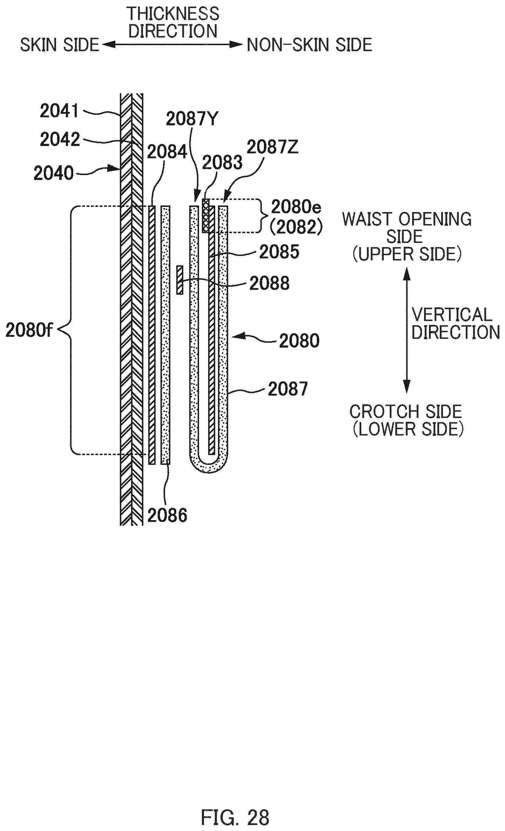

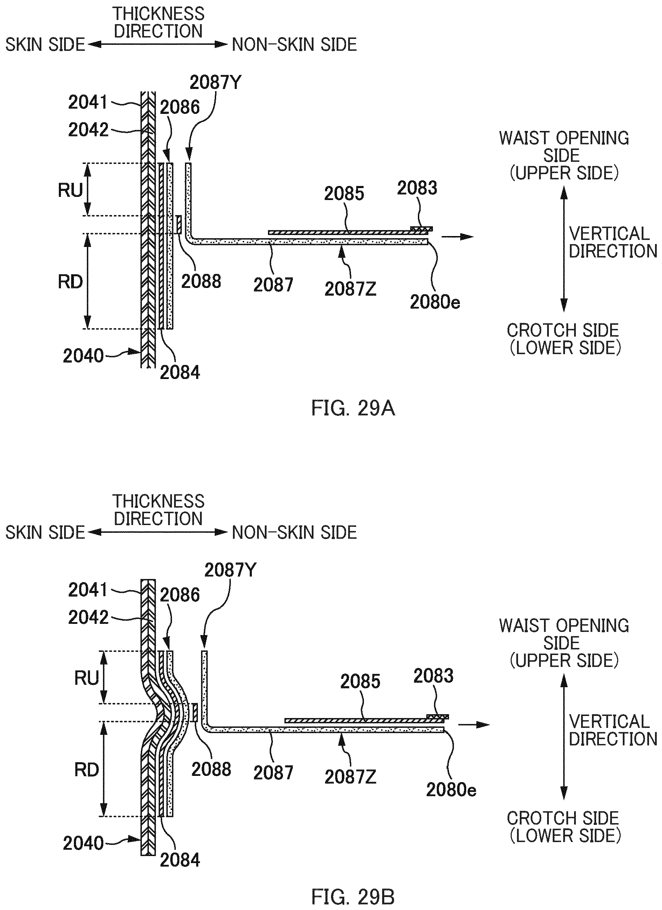

Further, in order to make the post handling tape 2080 (attachment) less likely to separate from the hydrophilic nonwoven fabric, the structure itself of the post handling tape 2080 may be changed. FIG. 28 is a schematic cross-sectional view showing a modified example of the post handling tape 2080. FIGS. 29A and 29B are explanatory diagrams illustrating forces that act on the post handling tape 2080 of the modified example when performing the post handling operation.

The post handling tape 2080 illustrated in FIG. 24 is formed of the tape main body 2081 that is a single tape base member folded in a Z shape, but the post handling tape 2080 of the modified example is formed of two different tape base members, namely a first member 2086 and a second member 2087 provided on the non-skin side, as shown in FIG. 28. However, another base member (e.g., a third member) may be provided. The configuration excluding the members 2086 and 2087 that serve as tape base members and a joining portion 2088 to which these members are joined are basically the same as those of the post handling tape 2080 illustrated in FIG. 24.

Similar to the tape main body 2081 described above, both the first member 2086 and the second member 2087 are band-shaped members formed of a resin material such as polyethylene or polypropylene. The first member 2086 is fixed to the non-skin-side sheet 2042 (hydrophilic nonwoven fabric) of the back waist portion 2040 with the attaching portion 2084. In contrast, the second member 2087 is folded in a V shape and has two layers: a first layer 2087y positioned on the skin side and a second layer 2087z positioned on the non-skin side. The first layer 2087y and the second layer 2087z are made peelably adhere by the tape adhesive portion 2085. On the other hand, the first member 2086 and the second member 2087 are non-peelably joined with the joining portion 2088 formed of an adhesive or the like. It should be noted that the joining portion 2088 is provided between the one end and the other end of the first member 2086 in the lengthwise direction. In other words, the joining portion 2088 is provided spaced apart from the upper end and the lower end of the first member 2086 by a predetermined distance in the vertical direction in FIG. 28. That is, as shown in FIG. 29, in an upper end portion RU and a lower end portion RD in the vertical direction, the first member 2086 and the second member 2087 have portions that are not joined to each other.

When the post handling operation of the diaper 2001 is performed using the post handling tape 2080 of the modified example, first, the tape grab member 2083 (one end portion 2080e in the lengthwise direction) is pulled toward the non-skin side to peel off the tape adhesive portion 2085, and the post handling tape 2080 is unfolded in the lengthwise direction. At this time, the second member 2087 is in a state of rising on the first member 2086 toward the non-skin side from the joining portion 2088 as shown in FIG. 29A. In this state, when the second member 2087 (tape grab member 2083) is further pulled toward the non-skin side, the portion positioned between two end portions of the first member 2086 in the lengthwise direction (the vertical direction in FIG. 29A) (a portion in which the joining portion 2088 is provided) are pulled toward the non-skin side. Therefore, the tensile force applied to the first member 2086 (attaching portion 2084) acts dispersedly on two lengthwise sides with the joining portion 2088 being centered.

The post handling tape 2080 illustrated in FIG. 24 has a configuration in which a single tape main body 2081 is folded in a Z shape. Therefore, when the tape grab member 2083 (one end portion 2080e) is pulled during the post handling operation, a tensile force to the attaching portion 2084 acts in a concentrated manner on the vertical upper end portion of the first layer 2081x of the tape main body 2081 (in FIG. 24, the boundary portion between the first layer 2081x and the second layer 2081y). That is, the tensile force is likely to be concentrated at a single lengthwise position (the vertical upper end portion) of the attaching portion 2084. In contrast, in the post handling tape 2080 of the modified example, the tensile force to the attaching portion 2084 can be dispersed in the lengthwise direction (vertical direction), and therefore it is possible to make the attaching portion 2084 further less likely to peel off compared with the case where a large force acts on a single place of the attaching portion 2084.

When the second member 2087 (tape grab member 2083) is further pulled toward the non-skin side from the state in FIG. 29A, a portion of the first member 2086 that is made adhere to the second member 2087 by the joining portion 2088 is pulled toward the non-skin side as shown in FIG. 29B. On the other hand, in the first member 2086, the end portion RU located on the lengthwise one side (vertical upper side) with respect to the joining portion 2088 and the end portion RD located on the lengthwise other side (vertical lower side) with respect to the joining portion 2088 are attached to the non-skin-side sheet 2042 (hydrophilic nonwoven fabric) by the attaching portion 2084. The first member can resist the tensile force in the portions, namely the end portion RU and the end portion RD.

That is, in FIG. 29B, in the first member 2086, two lengthwise side portions RU and RD of the portion where a tensile force is applied by the second member 2087 (a portion of the joining portion 2088) are each provided with adhesive portions (attaching portions 2084) adhering to the non-skin-side sheet 2042 (hydrophilic nonwoven fabric). Therefore, the two attaching portions 2084 on two sides of the first member 2086 can resist the tensile force acting on the first member 2086, and this makes the first member 2086 less likely to peel off. Accordingly, the post handling tape 2080 is less likely to be separated from the non-skin-side sheet 2042 (hydrophilic nonwoven fabric), and the post handling operation of the diaper 2001 can be more easily performed.

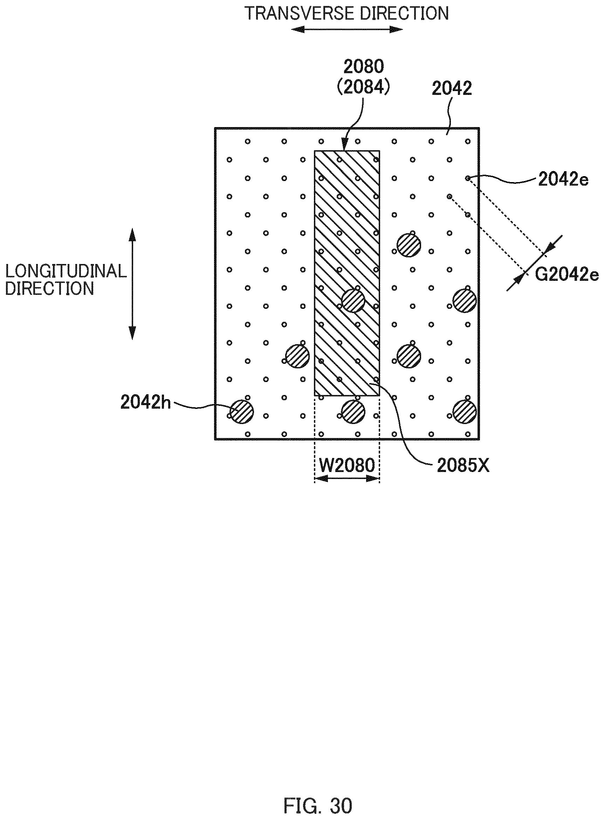

FIG. 30 is an enlarged plan view of a region B in FIG. 21, and is an explanatory diagram illustrating the state of the surface of the non-skin-side sheet 2042 in a portion to which the post handling tape 2080 is attached. As shown in FIG. 30, on the surface of the non-skin-side sheet 2042 (hydrophilic nonwoven fabric), a plurality of compressed portions 2042e where the non-skin-side sheet 2042 is compressed in the thickness direction are provided. The compressed portion 2042e is formed by performing so-called embossing when manufacturing the hydrophilic nonwoven fabric, and providing the compressed portions 2042e makes it possible to increase the strength of the hydrophilic nonwoven fabric. In FIG. 30, each compressed portion 2042e has a circular shape with a diameter of, for example, approximately 0.5 mm, and are arranged side by side in a manner such that a space G2042e between adjacent compressed portions 2042e and 2042e has a grid shape of approximately 2 mm. Accordingly, the non-skin-side sheet 2042 is less likely to be torn, and it can make the separation of the post handling tape 2080 (attachment) as illustrated in FIG. 26B less likely to occur.

It should be noted that it is desirable that the minimum value G2042e of the space between the plurality of compressed portions 2042e and 2042e formed on the non-skin-side sheet 2042 (hydrophilic nonwoven fabric) is smaller than the fiber length LfHy of the fibers fHy that constitute the hydrophilic nonwoven fabric (G2042e < LfHy). If the space G2042e is shorter than the fiber length LfHy, there is a high possibility that the compressed portion 2042e is formed at a position that overlaps the fiber fHy. That is, there is a high possibility that the fiber fHy itself is compressed when the compressed portion 2042e are formed. In the portion where the fiber fHy is compressed, the fiber fHy and other fibers are more likely to be pressed against each other. Therefore, compared with the case where the fibers are only intertwined and entangled with each other, the bonding force between the fibers becomes much stronger, and the hydrophilic nonwoven fabric is less likely to be torn. This enables to make the attachment further less likely to peel off.

Further, it is desirable that, at least one compressed portion 2042e is arranged in a portion in which the post handling tape 2080 (attachment) and the non-skin-side sheet 2042 (hydrophilic nonwoven fabric) overlap each other when viewed in the thickness direction of the non-skin-side sheet 2042 (hydrophilic nonwoven fabric). As described above, in the portion where the compressed portion 2042e is arranged, the strength of the hydrophilic nonwoven fabric is increased, and the nonwoven fabric is less likely to be torn. Therefore, the attachment is attached to such a portion which is less likely to be torn, enabling to make it more likely to suppress the peeling of the attachment. In the present embodiment, as shown in FIG. 30, since the plurality of compressed portions 2042e are arranged in the region (attaching portion 2084) to which the post handling tape 2080 is attached, the post handling tape 2080 can be made less likely to be separated.

At this time, it is more desirable that the width W2080 of the post handling tape 2080 (which is also the width of the attaching portion 2084) is larger than a space G2042e between the compressed portions 2042e (W2080 > G2042e). If the width W2080 of the post handling tape 2080 is larger than the space G2042e between the compressed portions 2042e, there is a high possibility that at least one compressed portion 2042e is arranged in the post handling tape 2080 in the transverse direction (lateral direction), and two or more compressed portions 2042e are further arranged. Therefore, as described above, in the portion to which the post handling tape 2080 (attachment) is attached, the bonding force between the fibers becomes stronger, and the hydrophilic nonwoven fabric is less likely to be torn. This can make the post handling tape 2080 further less likely to be separated.

Further, it is desirable that at least one hole portion 2042h is arranged in a portion where the post handling tape 2080 (attachment) and the non-skin-side sheet 2042 (hydrophilic nonwoven fabric) overlap each other when viewed in the thickness direction of the non-skin-side sheet 2042 (hydrophilic nonwoven fabric). In a portion where the hole portion 2042h is arranged, the adhesive that forms the attaching portion 2084 is attached to the skin-side sheet 41 (hydrophobic nonwoven fabric) arranged on the skin side of the non-skin-side sheet 2042 (hydrophilic nonwoven fabric). That is, in the portion of the hole portion 2042h, the hydrophobic nonwoven fabric containing no oil agent and the post handling tape 2080 (attachment) are made directly adhere to each other, and this makes it possible to enhance the adhesive force compared with the case of adhering to the hydrophilic nonwoven fabric containing the oil agent. Therefore, compared with the case where the hole portion 2042h and the attaching portion 2084 do not overlap each other, it can make it more likely to suppress the peeling of the post handling tape 2080 (attaching portion 2084) from the back waist portion 2040.

### Tenth Example (Second Embodiment)

In a tenth example (second embodiment), an underpants-shaped diaper 2002 (hereinafter, also referred to as a "diaper 2002") having a configuration partially different from that of the ninth example (first embodiment) will be described. The diaper 2002 has substantially the same configuration as the diaper 1002 illustrated in FIGS. 18A and 18B. However, the indicator 1070 need not necessarily be provided in the diaper 2002, and the post handling tape 2080 is provided therein.

The post handling tape 2080 (attachment) used when performing the post handling operation of the diaper 2002 is attached to a non-skin-side sheet 2022 (hydrophilic nonwoven fabric) of the back waist portion 2040 of the diaper 2002. The function and configuration of the post handling tape 2080 are substantially the same as those of the post handling tape 2080 described in the ninth example (first embodiment), and therefore description thereof will be omitted (see FIGS. 24 and 28 and the like).

Also in the diaper 2002 of the tenth example (second embodiment), the same effect as that of the diaper 2001 can be obtained. That is, in the diaper 2002, the hydrophilic nonwoven fabric that constitutes the non-skin-side sheet 2022 of the back waist portion 2040 has fibers having a fiber length longer than the dimension of the post handling tape 2080 (attachment), the dimension of the post handling tape 2080 being smaller one of the dimension of the post handling tape 2080 in the longitudinal direction and the dimension of the post handling tape 2080 in the transverse direction. In such a configuration, a plurality of fibers that constitute the hydrophilic nonwoven fabric are likely to be tangled with each other, and it increases the bonding force, making the hydrophilic nonwoven fabric less likely to be torn. Further, the fiber length is longer than the width of the post handling tape 2080, the fibers are likely to be entangled with each other outside the region where the post handling tape 2080 is attached, and this makes the hydrophilic nonwoven fabric less likely to be torn against the tensile force even in the case where the post handling tape 2080 is pulled. Therefore, it can make the post handling tape 2080 (attachment) less likely to peel off from the non-skin-side sheet 2022 (hydrophilic nonwoven fabric). In addition, the diaper 2002 basically has substantially the same features as those of the diaper 2001 described in the ninth example (first embodiment).

### Eleventh Example (Third Embodiment)

In an eleventh example (third embodiment), an underpants-shaped diaper 2003 (hereinafter, also referred to as a "diaper 2003") having a configuration partially different from that of the above-described embodiments will be described. The diaper 2003 has substantially the same configuration as the diaper 1003 illustrated in FIGS. 19A and 19B. However, the indicator 1070 need not necessarily be provided in the diaper 2003, and the post handling tape 2080 is provided therein.

The post handling tape 2080 (attachment) used when performing the post handling operation of the diaper 2003 is attached to a non-skin-side sheet 2042 (hydrophilic nonwoven fabric) of the back waist portion 2040 of the diaper 2003. The function and configuration of the post handling tape 2080 are substantially the same as those of the post handling tape 2080 described in the ninth example (first embodiment), and therefore description thereof will be omitted (see FIGS. 24 and 28 and the like).

Also in the diaper 2003 of the eleventh example (third embodiment), the same effect as that of the diaper 2001 can be obtained. That is, in the diaper 2003, the hydrophilic nonwoven fabric that constitutes the non-skin-side sheet 2042 of the back waist portion 2040 has fibers having a fiber length longer than the dimension of the post handling tape 2080 (attachment), the dimension of the post handling tape 2080 being smaller one of the dimension of the post handling tape 2080 in the longitudinal direction and the dimension of the post handling tape 2080 in the transverse direction. In such a configuration, a plurality of fibers that constitute the hydrophilic nonwoven fabric are likely to be tangled with each other, and it increases the bonding force, making the hydrophilic nonwoven fabric less likely to be torn. Further, the fiber length is longer than the width of the post handling tape 2080, the fibers are likely to be entangled with each other outside the region where the post handling tape 2080 is attached, and this makes the hydrophilic nonwoven fabric less likely to be torn against the tensile force even in the case where the post handling tape 2080 is pulled. Therefore, it can make the post handling tape 2080 (attachment) less likely to peel off from the non-skin-side sheet 2042 (hydrophilic nonwoven fabric). In addition, the diaper 2003 basically has substantially the same features as those of the diaper 2001 described in the ninth example (first embodiment).

### Twelfth Example (Fourth Embodiment)

In a twelfth example (fourth embodiment), a "tape-type diaper" different from the "underpants-shaped diaper" described in the ninth to eleventh examples (first to third embodiments) (hereinafter, also referred to as "diaper 2004") will be described as an example of the absorbent article. The diaper 2004 has substantially the same configuration as the diaper 1004 illustrated in FIGS. 20A and 20B. However, the indicator 1070 need not necessarily be provided in the diaper 2004, and the post handling tape 2080 is provided therein.

The post handling tape 2080 (attachment) used when performing the post handling operation of the diaper 2004 is attached to the exterior sheet 2025 (hydrophilic nonwoven fabric) of the back waist portion 2040 of the diaper 2004.

The function and configuration of the post handling tape 2080 are substantially the same as those of the post handling tape 2080 described in the ninth example (first embodiment), and therefore description thereof will be omitted (see FIGS. 24 and 28 and the like).

Also in the diaper 2004 of the twelfth example (fourth embodiment), the same effect as that of the diaper 2001 can be obtained. That is, in the diaper 2004, the hydrophilic nonwoven fabric that constitutes the exterior sheet 2025 of the back waist portion 2040 has fibers having a fiber length longer than the dimension of the post handling tape 2080 (attachment), the dimension of the post handling tape 2080 being smaller one of the dimension of the post handling tape 2080 in the longitudinal direction and the dimension of the post handling tape 2080 in the transverse direction. In such a configuration, a plurality of fibers that constitute the hydrophilic nonwoven fabric are likely to be tangled with each other, and it increases the bonding force, making the hydrophilic nonwoven fabric less likely to be torn. Further, the fiber length is longer than the width of the post handling tape 2080, the fibers are likely to be entangled with each other outside the region where the post handling tape 2080 is attached, and this makes the hydrophilic nonwoven fabric less likely to be torn against the tensile force even in the case where the post handling tape 2080 is pulled. Therefore, it can make the post handling tape 2080 (attachment) less likely to peel off from the exterior sheet 2025 (hydrophilic nonwoven fabric). In addition, the diaper 2004 basically has substantially the same features as those of the diaper 2001 described in the ninth example (first embodiment).

### Other Embodiments

Although the above embodiments of the present invention have been described, but the above-described embodiments are intended to facilitate the understanding of the present invention and are not intended to limit the interpretation of the present invention. In addition, the present invention can be modified or improved within the scope of the gist of the present invention, and it is needless to say that equivalents thereof are included in the present invention.

### REFERENCE SIGNS LIST

1: diaper (absorbent article, underpants-shaped diaper) (first example),
2: diaper (absorbent article, underpants-shaped diaper) (second example),
3: diaper (absorbent article, underpants-shaped diaper) (third example),
4: diaper (absorbent article, tape-type diaper) (fourth example),
10: absorbent main body,
11: absorbent core, 11b: core-wrapping sheet, 11c: narrow portion,
11s: slit portion,
12: top sheet,
13: back sheet, 13a: liquid-impermeable sheet, 13b: exterior sheet,
15: leak-proof wall portion, 16: leak-proof-wall elastic member,
17: leg elastic member,
18: side sheet,
20: waist member,
21: skin-side sheet (hydrophobic nonwoven fabric),
22: non-skin-side sheet (hydrophilic nonwoven fabric),
25: exterior sheet (hydrophilic nonwoven fabric),
26: fastening tape, 26f: hook-and-loop fastener, 27: target sheet,
30: front waist portion, 30sw: side portion,
31: skin-side sheet (hydrophobic nonwoven fabric),
32: non-skin-side sheet (hydrophilic nonwoven fabric), 32f: folded-back portion, 32h: hole portion,
35: waist elastic member,
36: skin surface sheet,
40: back waist portion, 40b: buttocks cover, 40sw: side portion,
41: skin-side sheet (hydrophobic nonwoven fabric),
42: non-skin-side sheet (hydrophilic nonwoven fabric), 42f: folded-back portion, 42h: hole portion,
45: waist elastic member,
46: skin surface sheet, 47: curved elastic member,
50: side joining portion,
60: welding portion,
60s: welding portion pair,
1001: diaper (absorbent article, underpants-shaped diaper) (fifth example),
1002: diaper (absorbent article, underpants-shaped diaper) (sixth example),
1003: diaper (absorbent article, underpants-shaped diaper) (seventh example),
1004: diaper (absorbent article, tape-type diaper) (eighth example),
1010: absorbent main body,
1011: absorbent core, 1011b: core-wrapping sheet, 1011c: narrow portion,
1012: top sheet,
1013: back sheet,
1013a: liquid-impermeable sheet (liquid-impermeable film),
1013b: exterior sheet,
1015: leak-proof wall portion, 1016: leak-proof-wall elastic member, 1017: leg elastic member,
1018: side sheet, 1019: back elastic member,
1020: waist member,
1021: skin-side sheet (hydrophobic nonwoven fabric),
1022: non-skin-side sheet (hydrophilic nonwoven fabric),
1025: exterior sheet (hydrophilic nonwoven fabric),
1026: fastening tape, 1026f: hook-and-loop fastener,
1027: target sheet,
1030: front waist portion, 1030sw: side portion,
1031: skin-side sheet (hydrophobic nonwoven fabric),
1032: non-skin-side sheet (hydrophilic nonwoven fabric), 1032f: folded-back portion,
1032h: hole portion,
1035: waist elastic member,
1036: skin surface sheet,
1040: back waist portion, 1040b: buttocks cover, 1040sw: side portion,
1041: skin-side sheet (hydrophobic nonwoven fabric),
1042: non-skin-side sheet (hydrophilic nonwoven fabric), 1042f: folded-back portion,
1042h: hole portion,
1045: waist elastic member,
1046: skin surface sheet, 1047: curved elastic member,
1050: side joining portion,
1060: welding portion,
1060s: welding portion pair,
1070: indicator,
2001: diaper (absorbent article, underpants-shaped diaper) (ninth example, first embodiment),
2002: diaper (absorbent article, underpants-shaped diaper) (tenth example, second embodiment),
2003: diaper (absorbent article, underpants-shaped diaper) (eleventh example, third embodiment),
2004: diaper (absorbent article, tape-type diaper) (twelfth example, fourth embodiment),
2010: absorbent main body,
2011: absorbent core, 2011b: core-wrapping sheet, 2011c: narrow portion,
2012: top sheet,
2013: back sheet,
2013a: liquid-impermeable sheet (breathable film), 2013b: exterior sheet,
2015: leak-proof wall portion, 2016: leak-proof-wall elastic member, 2017: leg elastic member,
2018: side sheet, 2019: back elastic member,
2020: waist member,
2021: skin-side sheet (hydrophobic nonwoven fabric),
2022: non-skin-side sheet (hydrophilic nonwoven fabric),
2025: exterior sheet (hydrophilic nonwoven fabric),
2026: fastening tape, 2026f: hook-and-loop fastener,
2027: target sheet,
2030: front waist portion, 2030sw: side portion,
2031: skin-side sheet (hydrophobic nonwoven fabric),
2032: non-skin-side sheet (hydrophilic nonwoven fabric), 2032f: folded-back portion,
2032h: hole portion,
2035: waist elastic member,
2036: skin surface sheet,
2040: back waist portion, 2040b: buttocks cover, 2040sw: side portion,
2041: skin-side sheet (hydrophobic nonwoven fabric),
2042: non-skin-side sheet (hydrophilic nonwoven fabric), 2042f: folded-back portion,
2042e: compressed portion, 2042h: hole portion,
2045: waist elastic member,
2046: skin surface sheet, 2047: curved elastic member,
2050: side joining portion,
2060: welding portion,
2060s: welding portion pair,
2080: post handling tape (attachment),
2080e: one end portion, 2080f: other end portion,
2081: tape main body, 2081x: first layer, 2081y: second layer, 2081z: third layer,
2082: tape grab portion, 2083: tape grab member, 2084: attaching portion
2085: tape adhesive portion, 2086: first member, 2087: second member,
2087y: first layer, 2087z: second layer,
2088: joining portion,
BH: waist opening, LH: leg opening,
CL: central position (longitudinal direction, lengthwise direction)

## Claims

1. An absorbent article (2001) comprising:
a liquid-absorbent absorbent main body (2010);
an exterior member (2030, 2040) that is provided on a non-skin side with respect to the absorbent main body (2010),
the exterior member (2030, 2040) having a hydrophilic nonwoven fabric (2032, 2042) on a non-skin side,
the hydrophilic nonwoven fabric (2032, 2042) containing a hydrophilic oil agent,
the absorbent article (2001) has an attachment (2080) that is attached with an adhesive to a non-skin-side surface of the hydrophilic nonwoven fabric (2032, 2042), wherein the attachment (2080) is a post handling attachment,
the hydrophilic nonwoven fabric (2032, 2042) having a fiber with a fiber length longer than a dimension of the attachment (2080),
the dimension of the attachment (2080) being a smaller one of a dimension of the attachment (2080) in a longitudinal direction and a dimension of the attachment (2080) in a transverse direction that is orthogonal to the longitudinal direction.

2. The absorbent article (2001) according to claim 1, wherein
the longitudinal direction is a direction in which the attachment (2080) is pulled from the hydrophilic nonwoven fabric (2032, 2042), and
the fiber is arranged so as to extend across the attachment (2080) in the transverse direction.

3. The absorbent article (2001) according to claim 2, wherein
the hydrophilic nonwoven fabric (2032, 2042) has a plurality of the fibers, and
a number of fibers arranged so as to extend across the attachment (2080) in the transverse direction is larger than a number of fibers arranged so as to extend across the attachment (2080) in the longitudinal direction.

4. The absorbent article (2001) according to any one of claims 1 to 3, wherein
the hydrophilic nonwoven fabric (2032, 2042) has a plurality of compressed portions (2042e) where the hydrophilic nonwoven fabric (2032, 2042) is compressed in a thickness direction.

5. The absorbent article (2001) according to claim 4, wherein
the fiber length is larger than a minimum value of a space between two adjacent compressed portions (2042e).

6. The absorbent article (2001) according to claim 4 or 5, wherein
at least one compressed portion (2042e) is arranged in a region where the attachment (2080) and the hydrophilic nonwoven fabric (2032, 2042) overlap each other when viewed in a thickness direction of the exterior member (2030, 2040).

7. The absorbent article (2001) according to any one of claims 4 to 6, wherein
of the dimension of the attachment (2080) in the longitudinal direction and the dimension of the attachment (2080) in the transverse direction,
the smaller dimension is larger than a minimum value of a space between two adjacent compressed portions (2042e).

8. The absorbent article (2001) according to any one of claims 1 to 7, wherein
the exterior member (2030, 2040) has a hydrophobic nonwoven fabric (2041) on a skin side with respect to the hydrophilic nonwoven fabric (2032, 2042),
the hydrophobic nonwoven fabric (2041) having lower hydrophilicity than the hydrophilic nonwoven fabric (2032, 2042).

9. The absorbent article (2001) according to claim 8, wherein
the hydrophilic nonwoven fabric (2032, 2042) has a hole portion (2042h) that penetrates the hydrophilic nonwoven fabric (2032, 2042) in a thickness direction, and
at least one hole portion (2042h) is arranged in a region where the attachment (2080) and the hydrophilic nonwoven fabric (2032, 2042) overlap each other when viewed in a thickness direction of the exterior member (2030, 2040).

10. The absorbent article (2001) according to any one of claims 1 to 9, wherein
the attachment (2080) is a post handling tape for maintaining the absorbent article (2001) in a compact form when disposing of the absorbent article (2001) after use.

11. The absorbent article (2001) according to claim 10, wherein
the post handling tape (2080) includes
a first member (2086) that is attached to the hydrophilic nonwoven fabric (2032, 2042), and
a second member (2087) that is joined to a non-skin side of the first member (2086) by a joining portion (2088),
the joining portion (2088) being provided between a one-side end and a other-side end of the first member (2086) in the lengthwise direction.

12. The absorbent article (2001) according to claim 11, wherein
in the first member (2086),
a portion located on a lengthwise one side with respect to the joining portion (2088)_and a portion located on a lengthwise other side with respect to the joining portion (2088) are each attached to the hydrophilic nonwoven fabric (2032, 2042).

## Patentansprüche

1. Absorbierender Artikel (2001), der Folgendes umfasst:
einen flüssigkeitsabsorbierenden Hauptkörper (2010);
ein äußeres Element (2030, 2040), das auf einer Nicht-Hautseite in Bezug auf den absorbierenden Hauptkörper (2010) bereitgestellt ist,
wobei das äußere Element (2030, 2040) einen hydrophilen Vliesstoff (2032, 2042) auf einer Nicht-Hautseite aufweist,
wobei der hydrophile Vliesstoff (2032, 2042) ein hydrophiles Ölmittel enthält,
wobei der absorbierende Artikel (2001) eine Befestigung (2080) aufweist, die mit einem Haftmittel an einer Nicht-Hautseitenoberfläche des hydrophilen Vliesstoffs (2032, 2042) befestigt ist, wobei die Befestigung (2080) eine Befestigung zur nachfolgenden Handhabung ist,
der hydrophile Vliesstoff (2032, 2042) eine Faser mit einer Faserlänge aufweist, die länger ist als eine Abmessung der Befestigung (2080),
wobei die Abmessung der Befestigung (2080) eine kleinere einer Abmessung der Befestigung (2080) in einer Längsrichtung und einer Abmessung der Befestigung (2080) in einer Querrichtung, die rechtwinklig zu der Längsrichtung vorliegt, ist.

2. Absorbierender Artikel (2001) nach Anspruch 1, wobei
die Längsrichtung eine Richtung ist, in der die Befestigung (2080) von dem hydrophilen Vliesstoff (2032, 2042) gezogen wird, und
die Faser derart angeordnet ist, dass sie sich über die Befestigung (2080) in der Querrichtung erstreckt.

3. Absorbierender Artikel (2001) nach Anspruch 2, wobei
der hydrophile Vliesstoff (2032, 2042) eine Vielzahl der Fasern aufweist und
etliche Fasern, die derart angeordnet sind, dass sie sich über die Befestigung (2080) in der Querrichtung erstrecken, größer sind als etliche Fasern, die derart angeordnet sind, dass sie sich über die Befestigung (2080) in der Längsrichtung erstrecken.

4. Absorbierender Artikel (2001) nach einem der Ansprüche 1 bis 3, wobei
der hydrophile Vliesstoff (2032, 2042) eine Vielzahl von komprimierten Teilen (2042e) ausweist, wo der hydrophile Vliesstoff (2032, 2042) in einer Dickenrichtung komprimiert ist.

5. Absorbierender Artikel (2001) nach Anspruch 4, wobei
die Faserlänge größer ist als ein minimaler Wert eines Raums zwischen zwei benachbarten komprimierten Teilen (2042e).

6. Absorbierender Artikel (2001) nach Anspruch 4 oder 5, wobei
mindestens ein komprimierter Teil (2042e) in einem Bereich angeordnet ist, wo die Befestigung (2080) und der hydrophile Vliesstoff (2032, 2042), bei Betrachtung in einer Dickenrichtung des äußeren Elements (2030, 2040), miteinander überlappen.

7. Absorbierender Artikel (2001) nach einem der Ansprüche 4 bis 6, wobei
von der Abmessung der Befestigung (2080) in der Längsrichtung und der Abmessung der Befestigung (2080) in der Querrichtung
die kleinere Abmessung größer ist als ein minimaler Wert eines Raums zwischen zwei benachbarten komprimierten Teilen (2042e).

8. Absorbierender Artikel (2001) nach einem der Ansprüche 1 bis 7, wobei
das äußere Element (2030, 2040) einen hydrophoben Vliesstoff (2041) auf einer Hautseite in Bezug auf den hydrophilen Vliesstoff (2032, 2042) aufweist,
wobei der hydrophobe Vliesstoff (2041) eine geringere Hydrophilie aufweist als der hydrophile Vliesstoff (2032, 2042).

9. Absorbierender Artikel (2001) nach Anspruch 8, wobei
der hydrophile Vliesstoff (2032, 2042) einen Lochteil (2042h) aufweist, der den hydrophilen Vliesstoff (2032, 2042) in einer Dickenrichtung durchdringt, und
mindestens ein Lochteil (2042h) in einem Bereich angeordnet ist, wo die Befestigung (2080) und der hydrophile Vliesstoff (2032, 2042), bei Betrachtung in einer Dickenrichtung des äußeren Elements (2030, 2040), miteinander überlappen.

10. Absorbierender Artikel (2001) nach einem der Ansprüche 1 bis 9, wobei
die Befestigung (2080) ein Band zur nachfolgenden Handhabung ist, um den absorbierenden Artikel (2001) in einer kompakten Form beizubehalten, wenn der absorbierende Artikel (2001) nach Verwendung entsorgt wird.

11. Absorbierender Artikel (2001) nach Anspruch 10, wobei
das Band zur nachfolgenden Handhabung (2080) Folgendes einschließt:
ein erstes Element (2086), das an dem hydrophilen Vliesstoff (2032, 2042) befestigt ist, und
ein zweites Element (2087), das mit einer Nicht-Hautseite des ersten Elements (2086) durch einen Verbindungsteil (2088) verbunden ist,
wobei der Verbindungsteil (2088) zwischen einem Ende einer Seite und einem Ende einer anderen Seite des ersten Elements (2086) in der Längsrichtung bereitgestellt ist.

12. Absorbierender Artikel (2001) nach Anspruch 11, wobei
in dem ersten Element (2086)
ein Teil, der sich auf einer längs gerichteten einen Seite in Bezug auf den Verbindungsteil (2088) befindet, und ein Teil, der sich auf einer längs gerichteten anderen Seite in Bezug auf den Verbindungsteil (2088) befindet, jeweils an dem hydrophilen Vliesstoff (2032, 2042) befestigt sind.

## Revendications

1. Article absorbant (2001) comportant :
un corps principal absorbant qui absorbe les liquides (2010) ;
un élément extérieur (2030, 2040) qui est mis en œuvre sur un côté non orienté vers la peau par rapport au corps principal absorbant (2010),
l'élément extérieur (2030, 2040) ayant un tissu non tissé hydrophile (2032, 2042) sur un côté non orienté vers la peau,
le tissu non-tissé hydrophile (2032, 2042) contenant un agent huileux hydrophile,
l'article absorbant (2001) a une fixation (2080) qui est fixée au moyen d'un adhésif sur une surface côté non orienté vers la peau du tissu non tissé hydrophile (2032, 2042), dans lequel la fixation (2080) est une fixation de post-manipulation,
le tissu non tissé hydrophile (2032, 2042) ayant une fibre dont la longueur de fibre est supérieure à une dimension de la fixation (2080),
la dimension de la fixation (2080) étant une dimension plus petite parmi une dimension de la fixation (2080) dans une direction longitudinale et une dimension de la fixation (2080) dans une direction transversale qui est orthogonale par rapport à la direction longitudinale.

2. Article absorbant (2001) selon la revendication 1, dans lequel
la direction longitudinale est une direction dans laquelle la fixation (2080) est tirée en provenance du tissu non tissé hydrophile (2032, 2042), et
la fibre est agencée de manière à s'étendre en travers de la fixation (2080) dans la direction transversale.

3. Article absorbant (2001) selon la revendication 2, dans lequel
le tissu non tissé hydrophile (2032, 2042) a une pluralité de fibres, et
un certain nombre de fibres agencées de manière à s'étendre en travers de la fixation (2080) dans la direction transversale est supérieur à un certain nombre de fibres agencées de manière à s'étendre en travers de la fixation (2080) dans la direction longitudinale.

4. Article absorbant (2001) selon l'une quelconque des revendications 1 à 3, dans lequel
le tissu non tissé hydrophile (2032, 2042) a une pluralité de parties comprimées (2042e) dans lesquelles le tissu non tissé hydrophile (2032, 2042) est comprimé dans une direction allant dans le sens de l'épaisseur.

5. Article absorbant (2001) selon la revendication 4, dans lequel
la longueur de fibre est supérieure à une valeur minimale d'un espace entre deux parties comprimées adjacentes (2042e).

6. Article absorbant (2001) selon la revendication 4 ou la revendication 5, dans lequel
au moins une partie comprimée (2042e) est agencée dans une région dans laquelle la fixation (2080) et le tissu non tissé hydrophile (2032, 2042) se chevauchent quand ils sont vus dans une direction allant dans le sens de l'épaisseur de l'élément extérieur (2030, 2040).

7. Article absorbant (2001) selon l'une quelconque des revendications 4 à 6, dans lequel
en ce qui concerne la dimension de la fixation (2080) dans la direction longitudinale et la dimension de la fixation (2080) dans la direction transversale,
la dimension la plus petite est supérieure à une valeur minimale d'un espace entre deux parties comprimées adjacentes (2042e).

8. Article absorbant (2001) selon l'une quelconque des revendications 1 à 7, dans lequel
l'élément extérieur (2030, 2040) a un tissu non tissé hydrophobe (2041) sur un côté orienté vers la peau par rapport au tissu non tissé hydrophile (2032, 2042),
le tissu non tissé hydrophobe (2041) ayant une hydrophilie inférieure par rapport au tissu non tissé hydrophile (2032, 2042).

9. Article absorbant (2001) selon la revendication 8, dans lequel
le tissu non tissé hydrophile (2032, 2042) a une partie formant trou (2042h) qui pénètre dans le tissu non tissé hydrophile (2032, 2042) dans une direction allant dans le sens de l'épaisseur, et
au moins une partie formant trou (2042h) est agencée dans une région dans laquelle la fixation (2080) et le tissu non tissé hydrophile (2032, 2042) se chevauchent quand ils sont vus dans une direction allant dans le sens de l'épaisseur de l'élément extérieur (2030, 2040).

10. Article absorbant (2001) selon l'une quelconque des revendications 1 à 9, dans lequel
la fixation (2080) est une bande de post-manipulation servant à maintenir l'article absorbant (2001) dans une forme compacte lors de la mise au rebut de l'article absorbant (2001) après utilisation.

11. Article absorbant (2001) selon la revendication 10, dans lequel
la bande de post-manipulation (2080) comprend
un premier élément (2086) qui est fixé sur le tissu non tissé hydrophile (2032, 2042), et
un deuxième élément (2087) qui est assemblé au niveau d'un côté non orienté vers la peau du premier élément (2086) par une partie d'assemblage (2088),
la partie d'assemblage (2088) étant mise en œuvre entre une extrémité d'un côté et une extrémité d'une autre extrémité du premier élément (2086) dans la direction allant dans le sens de la longueur.

12. Article absorbant (2001) selon la revendication 11, dans lequel
dans le premier élément (2086),
une partie située sur un côté dans le sens de la longueur par rapport à la partie d'assemblage (2088) et une partie située sur un autre côté dans le sens de la longueur par rapport à la partie d'assemblage (2088) sont chacune fixées sur le tissu non tissé hydrophile (2032, 2042).
